# EUROPEAN PATENT SPECIFICATION

(11) **EP 1 957 678 B1**
(45) Date of publication and mention of the grant of the patent: **13.06.2012**
(21) Application number: 06850200.4
(22) Date of filing: 28.11.2006
(51) Int. Cl.: C12Q 1/70

(54) **COMPOSITIONS FOR USE IN IDENTIFICATION OF ADVENTITIOUS CONTAMINANT VIRUSES**
ZUSAMMENSETZUNG ZUR VERWENDUNG BEI DER IDENTIFIKATION ADVENTIVER KONTAMINATIONSVIREN
COMPOSITIONS DESTINEES A ETRE UTILISEES POUR L'IDENTIFICATION DE VIRUS CONTAMINANTS ADVENTICES

(30) Priority: 28.11.2005 US 740617 P
(43) Date of publication of application: 20.08.2008
(73) Proprietor: Ibis Biosciences, Inc., Carlsbad, CA 92008 (US)
(72) Inventor: SAMPATH, Rangarajan, San Diego, CA 92129 (US); LI, Feng, San Diego, CA 92121 (US); MELTON, Rachael, Vista, CA 92081 (US); HALL, Thomas A., Oceanside, CA 92056 (US); ECKER, David J., Encinitas, CA 92024 (US)
(74) Representative: Hallybone, Huw George
(86) International application number: PCT/US2006/061307
(87) International publication number: WO 2007/100397

(56) References cited:
- EP-A2- 1 310 571
- WO-A-95/11997
- WO-A-02/099130
- WO-A-03/100035
- WO-A-2005/075686
- WO-A-2006/094238
- JURINKE C ET AL: "Application of nested PCR and mass spectrometry for DNA-based virus detection: HBV-DNA detected in the majority of isolated anti-HBc positive sera" GENETIC ANALYSIS: BIOMOLECULAR ENGINEERING, ELSEVIER SCIENCE PUBLISHING, US, vol. 14, no. 3, January 1998 (1998-01), pages 97-102, XP004114932 ISSN: 1050-3862
- SAMPATH RANGARAJAN ET AL: "Rapid identification of emerging pathogens: coronavirus." EMERGING INFECTIOUS DISEASES MAR 2005, vol. 11, no. 3, March 2005 (2005-03), pages 373-379, XP002454984 ISSN: 1080-6040
- KATANO H ET AL: "IDENTIFICATION OF ADENO-ASSOCIATED VIRUS CONTAMINATION IN CELL AND VIRUS STOCKS BY PCR" BIOTECHNIQUES, INFORMA LIFE SCIENCES PUBLISHING, WESTBOROUGH, MA, US, vol. 36, no. 4, April 2004 (2004-04), pages 676-680, XP001207105 ISSN: 0736-6205

### SEQUENCE LISTING

<110> Isis Pharmaceuticals, Inc.
   Sampath, Rangarajan
   Li, Feng
   Melton, Rachael
   Hall, Thomas A.
   Ecker, David J.
<120> COMPOSITIONS FOR USE IN IDENTIFICATION OF ADVENTITIOUS CONTAMINANT VIRUSES
<130> DIBIS-0081WO
<150> 60/740,617
   <151> 2005-11-28
<160> 338
<170> FastSEQ for Windows Version 4.0
<210> 1
   <211> 24
   <212> DNA
   <213> Artificial Sequence
<220>
   <223> Primer
<400> 1
   tagtgcaatc catctagcag ctgt 24
<210> 2
   <211> 23
   <212> DNA
   <213> Artificial Sequence
<220>
   <223> Primer
<400> 2
   tggacattca tctctatcag tgc 23
<210> 3
   <211> 24
   <212> DNA
   <213> Artificial Sequence
<220>
   <223> Primer
<400> 3
   tagatccaca agctttaggg tctg 24
<210> 4
   <211> 23
   <212> DNA
   <213> Artificial Sequence
<220>
   <223> Primer
<400> 4
   tgctgaattc gtaacattga tga 23
<210> 5
   <211> 25
   <212> DNA
   <213> Artificial Sequence
<220>
   <223> Primer
<400> 5
   tagggagact ttttgctaaa atgac 25
<210> 6
   <211> 22
   <212> DNA
   <213> Artificial Sequence
<220>
   <223> Primer
<400> 6
   tgtttgcaca gaggctaaat ga 22
<210> 7
   <211> 23
   <212> DNA
   <213> Artificial Sequence
<220>
   <223> Primer
<400> 7
   tgccttaatt ggagatatga gac 23
<210> 8
   <211> 20
   <212> DNA
   <213> Artificial Sequence
<220>
   <223> Primer
<400> 8
   tgtgtcatct gcgagtgtgg 20
<210> 9
   <211> 17
   <212> DNA
   <213> Artificial Sequence
<220>
   <223> Primer
<400> 9
   tttggacacc caatggt 17
<210> 10 '
   <211> 21
   <212> DNA
   <213> Artificial Sequence
<220>
   <223> Primer
<400> 10
   tagaatgttt gctatgcaac c 21
<210> 11
   <211> 18
   <212> DNA
   <213> Artificial Sequence
<220>
   <223> Primer
<400> 11
   tatgtttgct atgcaacc 18
<210> 12
   <211> 19
   <212> DNA
   <213> Artificial Sequence
<220>
   <223> Primer
<400> 12
   taggaccgtg gataaacac 19
<210> 13
   <211> 24
   <212> DNA
   <213> Artificial Sequence
<220>
   <223> Primer
<400> 13
   tgttaatgtc tatcttcctg actc 24
<210> 14
   <211> 18
   <212> DNA
   <213> Artificial Sequence
<220>
   <223> Primer
<400> 14
   tgtctatctt cctgactc 18
<210> 15
   <211> 25
   <212> DNA
   <213> Artificial Sequence
<220>
   <223> Primer
<400> 15
   taagtaagtt caaccaagcc tttag 25
<210> 16
   <211> 15
   <212> DNA
   <213> Artificial Sequence
<220>
   <223> Primer
<400> 16
   taaccaagcc tttag 15
<210> 17
   <211> 26
   <212> DNA
   <213> Artificial Sequence
<220>
   <223> Primer
<400> 17
   tcaagagatc ttcagtttat gagtaa 26
<210> 18
   <211> 20
   <212> DNA
   <213> Artificial Sequence
<220>
   <223> Primer
<400> 18
   tcaagagatc ttcagtttat 20
<210> 19
   <211> 19
   <212> DNA
   <213> Artificial Sequence
<220>
   <223> Primer
<400> 19
   tgaacatacc aatgcagtt 19
<210> 20
   <211> 21
   <212> DNA
   <213> Artificial Sequence
<220>
   <223> Primer
<400> 20
   tgacgtagcc aaggaggcgt t 21
<210> 21
   <211> 25
   <212> DNA
   <213> Artificial Sequence
<220>
   <223> Primer
<400> 21
   tcgcagccat cttggcaaca tcctc 25
<210> 22
   <211> 25
   <212> DNA
   <213> Artificial Sequence
<220>
   <223> Primer
<400> 22
   tccaggtact tcacccccaa acctg 25
<210> 23
   <211> 25
   <212> DNA
   <213> Artificial Sequence
<220>
   <223> Primer
<400> 23
   tcaaccacat aagaggtggg tgttc 25
<210> 24
   <211> 25
   <212> DNA
   <213> Artificial Sequence
<220>
   <223> Primer
<400> 24
   tggctgaact tttgaaagtg agcgg 25
<210> 25
   <211> 26
   <212> DNA
   <213> Artificial Sequence
<220>
   <223> Primer
<400> 25
   tgggatgatc tactgagact gtgtga 26
<210> 26
   <211> 22
   <212> DNA
   <213> Artificial Sequence
<220>
   <223> Primer
<400> 26
   tggtactcct caactgctgt cc 22
<210> 27
   <211> 21
   <212> DNA
   <213> Artificial Sequence
<220>
   <223> Primer
<400> 27
   tagcaggaag cactatgggc g 21
<210> 28
   <211> 22
   <212> DNA
   <213> Artificial Sequence
<220>
   <223> Primer
<400> 28
   tgagcagcag gaagcactat gg 22
<210> 29
   <211> 32
   <212> DNA
   <213> Artificial Sequence
<220>
   <223> Primer
<400> 29
   tgtgaatatc aagcaggaca taacaaggta gg 32
<210> 30
   <211> 29
   <212> DNA
   <213> Artificial Sequence
<220>
   <223> Primer
<400> 30
   tataatccac ctatcccagt aggagaaat 29
<210> 31
   <211> 26
   <212> DNA
   <213> Artificial Sequence
<220>
   <223> Primer
<400> 31
   tcgaaaaacc tccaggcaag agtcac 26
<210> 32
   <211> 26
   <212> DNA
   <213> Artificial Sequence
<220>
   <223> Primer
<400> 32
   tcaggcaaga gtcactgcta tcgaga 26
<210> 33
   <211> 26
   <212> DNA
   <213> Artificial Sequence
<220>
   <223> Primer
<400> 33
   tgccagggag tagtagaagc aatgaa 26
<210> 34
   <211> 25
   <212> DNA
   <213> Artificial Sequence
<220>
   <223> Primer
<400> 34
   tgccaatcac tcatacaacc cccaa 25
<210> 35
   <211> 25
   <212> DNA
   <213> Artificial Sequence
<220>
   <223> Primer
<400> 35
   tcagagcatc agatcacctg ggacc 25
<210> 36
   <211> 23
   <212> DNA
   <213> Artificial Sequence
<220>
   <223> Primer
<400> 36
   ggaggctccg ttgtctgcat gta 23
<210> 37
   <211> 26
   <212> DNA
   <213> Artificial Sequence
<220>
   <223> Primer
<400> 37
   tactctcaca cggcctcata cagtac 26
<210> 38
   <211> 22
   <212> DNA
   <213> Artificial Sequence
<220>
   <223> Primer
<400> 38
   tcttttccag accccggact cc 22
<210> 39
   <211> 26
   <212> DNA
   <213> Artificial Sequence
<220>
   <223> Primer
<400> 39
   tcgttatcgg ctcagctcta cagttc 26
<210> 40
   <211> 21
   <212> DNA
   <213> Artificial Sequence
<220>
   <223> Primer
<400> 40
   tagaggcgga tgacaatggc g 21
<210> 41
   <211> 23
   <212> DNA
   <213> Artificial Sequence
<220>
   <223> Primer
<400> 41
   tcaccaaggt gcctctaaaa cga 23
<210> 42
   <211> 22
   <212> DNA
   <213> Artificial Sequence
<220>
   <223> Primer
<400> 42
   tggaccatca ttggaaggga cg 22
<210> 43
   <211> 25
   <212> DNA
   <213> Artificial Sequence
<220>
   <223> Primer
<400> 43
   ttatgtcctt ggggtcacct actgg 25
<210> 44
   <211> 25
   <212> DNA
   <213> Artificial Sequence
<220>
   <223> Primer
<400> 44
   tccatacttc gccttcacca ttccc 25
<210> 45
   <211> 24
   <212> DNA
   <213> Artificial Sequence
<220>
   <223> Primer
<400> 45
   tttcgttgtg gcaaggtagg acac 24
<210> 46
   <211> 25
   <212> DNA
   <213> Artificial Sequence
<220>
   <223> Primer
<400> 46
   tcaccacgca atgcttccct atctt 25
<210> 47
   <211> 24
   <212> DNA
   <213> Artificial Sequence
<220>
   <223> Primer
<400> 47
   ttggtccatg actccgacct tgaa 24
<210> 48
   <211> 25
   <212> DNA
   <213> Artificial Sequence
<220>
   <223> Primer
<400> 48
   tccgatgcac attcacggtt ggtat 25
<210> 49
   <211> 26
   <212> DNA
   <213> Artificial Sequence
<220>
   <223> Primer
<400> 49
   tctagccatg gcgttagtat gagtgt 26
<210> 50
   <211> 24
   <212> DNA
   <213> Artificial Sequence
<220>
   <223> Primer
<400> 50
   ttcacgcaga aagcgtctag ccat 24
<210> 51
   <211> 23
   <212> DNA
   <213> Artificial Sequence
<220>
   <223> Primer
<400> 51
   ttcacgcaga aagcgtctag cca 23
<210> 52
   <211> 24
   <212> DNA
   <213> Artificial Sequence
<220>
   <223> Primer
<400> 52
   agcgtctagc catggcgtta gtat 24
<210> 53
   <211> 22
   <212> DNA
   <213> Artificial Sequence
<220>
   <223> Primer
<400> 53
   tcgcaagact gctagccgag ta 22
<210> 54
   <211> 28
   <212> DNA
   <213> Artificial Sequence
<220>
   <223> Primer
<400> 54
   taggtcgcgt aatttgggta aggtcatc 28
<210> 55
   <211> 26
   <212> DNA
   <213> Artificial Sequence
<220>
   <223> Primer
<400> 55
   tccttcacgg aggctatgac taggta 26
<210> 56
   <211> 26
   <212> DNA
   <213> Artificial Sequence
<220>
   <223> Primer
<400> 56
   tcagtgaata tgactagcca ggtgct 26
<210> 57
   <211> 25
   <212> DNA
   <213> Artificial Sequence
<220>
   <223> Primer
<400> 57
   tgctcctctc aaaaccatgg gacac 25
<210> 58
   <211> 30
   <212> DNA
   <213> Artificial Sequence
<220>
   <223> Primer
<400> 58
   tcatctacaa catgatggga aagagagaga 30
<210> 59
   <211> 30
   <212> DNA
   <213> Artificial Sequence
<220>
   <223> Primer
<400> 59
   tggatagagg agaatgaatg gatggaagac 30
<210> 60
   <211> 28
   <212> DNA
   <213> Artificial Sequence
<220>
   <223> Primer
<400> 60
   tgtcacactt gcatttacaa catgatgg 28
<210> 61
   <211> 26
   <212> DNA
   <213> Artificial Sequence
<220>
   <223> Primer
<400> 61
   tcaagatggg gaatgagatt gccctt 26
<210> 62
   <211> 28
   <212> DNA
   <213> Artificial Sequence
<220>
   <223> Primer
<400> 62
   tgcctagtgt gaagatgggg aatgagat 28
<210> 63
   <211> 25
   <212> DNA
   <213> Artificial Sequence
<220>
   <223> Primer
<400> 63
   tccgggctgt caatatgcta aaacg 25
<210> 64
   <211> 24
   <212> DNA
   <213> Artificial Sequence
<220>
   <223> Primer
<400> 64
   tccgtcgcaa gttggtgatg agta 24
<210> 65
   <211> 26
   <212> DNA
   <213> Artificial Sequence
<220>
   <223> Primer
<400> 65
   tgattgaccc ttttcagttg ggcctt 26
<210> 66
   <211> 23
   <212> DNA
   <213> Artificial Sequence
<220>
   <223> Primer
<400> 66
   tcaacctcca atcactcacc aac 23
<210> 67
   <211> 25
   <212> DNA
   <213> Artificial Sequence
<220>
   <223> Primer
<400> 67
   tccccaatct ccaatcactc accaa 25
<210> 68
   <211> 25
   <212> DNA
   <213> Artificial Sequence
<220>
   <223> Primer
<400> 68
   tcgcagtccc caatctccaa tcact 25
<210> 69
   <211> 25
   <212> DNA
   <213> Artificial Sequence
<220>
   <223> Primer
<400> 69
   tggctgctag gctgtgctgc caact 25
<210> 70
   <211> 28
   <212> DNA
   <213> Artificial Sequence
<220>
   <223> Primer
<400> 70
   tactaggagg ctgtaggcat aaattggt 28
<210> 71
   <211> 21
   <212> DNA
   <213> Artificial Sequence
<220>
   <223> Primer
<400> 71
   tacccctgct cgtgttacag g 21
<210> 72
   <211> 23
   <212> DNA
   <213> Artificial Sequence
<220>
   <223> Primer
<400> 72
   taggacccct gctcgtgtta cag 23
<210> 73
   <211> 19
   <212> DNA
   <213> Artificial Sequence
<220>
   <223> Primer
<400> 73
   tggatgtgtc tgcggcgtt 19
<210> 74
   <211> 21
   <212> DNA
   <213> Artificial Sequence
<220>
   <223> Primer
<400> 74
   tatcgctgga tgtgtctgcg g 21
<210> 75
   <211> 24
   <212> DNA
   <213> Artificial Sequence
<220>
   <223> Primer
<400> 75
   tgcagtcccc aatctccaat cact 24
<210> 76
   <211> 24
   <212> DNA
   <213> Artificial Sequence
<220>
   <223> Primer
<400> 76
   tcacctggga ccccatcgat ggac 24
<210> 77
   <211> 25
   <212> DNA
   <213> Artificial Sequence
<220>
   <223> Primer
<400> 77
   tcagagcatc agatcacctg ggacc 25
<210> 78
   <211> 29
   <212> DNA
   <213> Artificial Sequence
<220>
   <223> Primer
<400> 78
   tacccagtct acgtgtttgg cgactgtgt 29
<210> 79
   <211> 21
   <212> DNA
   <213> Artificial Sequence
<220>
   <223> Primer
<400> 79
   tggtgtggtg agagtttggg a 21
<210> 80
   <211> 21
   <212> DNA
   <213> Artificial Sequence
<220>
   <223> Primer
<220>
   <221> misc_feature
   <222> 4
   <223> n = A,T,C or G
<400> 80
   tggngtggtg agagtttggg a 21
<210> 81
   <211> 23
   <212> DNA
   <213> Artificial Sequence
<220>
   <223> Primer
<400> 81
   tgtctttcag gagatggatg gcc 23
<210> 82
   <211> 23
   <212> DNA
   <213> Artificial Sequence
<220>
   <223> Primer
<220>
   <221> misc_feature
   <222> 12, 15
   <223> n = A,T,C or G
<400> 82
   tgtctttcag gnganggatg gcc 23
<210> 83
   <211> 21
   <212> DNA
   <213> Artificial Sequence
<220>
   <223> Primer
<400> 83
   tggtgttgtg agggtctggg a 21
<210> 84
   <211> 23
   <212> DNA
   <213> Artificial Sequence
<220>
   <223> Primer
<400> 84
   tgtctctctg gagatgggtg gcc 23
<210> 85
   <211> 23
   <212> DNA
   <213> Artificial Sequence
<220>
   <223> Primer
<220>
   <221> misc_feature
   <222> 9, 12, 18
   <223> n = I
<400> 85
   tgtctctcng gngatggntg gcc 23
<210> 86
   <211> 23
   <212> DNA
   <213> Artificial Sequence
<220>
   <223> Primer
<220>
   <221> misc_feature
   <222> 9, 12, 18
   <223> n = I
<220>
   <221> misc_feature
   <222> 19
   <223> n = propynylated T
<220>
   <221> misc_feature
   <222> 22, 23
   <223> n = propynylated C
<400> 86
   tgtctctcng gngatggnng gnn 23
<210> 87
   <211> 30
   <212> DNA
   <213> Artificial Sequence
<220>
   <223> Primer
<400> 87
   tagtctcaaa gagaaagaga tcaaacaaga 30
<210> 88
   <211> 32
   <212> DNA
   <213> Artificial Sequence
<220>
   <223> Primer
<400> 88
   tgtacaatct atgtaggaga tccttactgt cc 32
<210> 89
   <211> 27
   <212> DNA
   <213> Artificial Sequence
<220>
   <223> Primer
<400> 89
   tatcagtgca atccatctag cagctgt 27
<210> 90
   <211> 24
   <212> DNA
   <213> Artificial Sequence
<220>
   <223> Primer
<400> 90
   tagtgcaatc catctagcag ctgt 24
<210> 91
   <211> 26
   <212> DNA
   <213> Artificial Sequence
<220>
   <223> Primer
<400> 91
   tgagaccatc ataagtagca agatgt 26
<210> 92
   <211> 26
   <212> DNA
   <213> Artificial Sequence
<220>
   <223> Primer
<400> 92
   tatagggtca tgaatcaaga acccgg 26
<210> 93
   <211> 26
   <212> DNA
   <213> Artificial Sequence
<220>
   <223> Primer
<220>
   <221> misc_feature
   <222> 24
   <223> n = I
<400> 93
   tatagggtca tgaatcaaga accngg 26
<210> 94
   <211> 25
   <212> DNA
   <213> Artificial Sequence
<220>
   <223> Primer
<400> 94
   ttggatcagc cactgatgaa agatc 25
<210> 95
   <211> 24
   <212> DNA
   <213> Artificial Sequence
<220>
   <223> Primer
<400> 95
   tgatgagata tcgtggatgg aagc 24
<210> 96
   <211> 24
   <212> DNA
   <213> Artificial Sequence
<220>
   <223> Primer
<400> 96
   tgatgaaata tcgtggatgg aagc 24
<210> 97
   <211> 26
   <212> DNA
   <213> Artificial Sequence
<220>
   <223> Primer
<400> 97
   tgtgcatctt actcactaaa ggagaa 26
<210> 98
   <211> 27
   <212> DNA
   <213> Artificial Sequence
<220>
   <223> Primer
<400> 98
   tcagttgatg ggcctacctc atttctt 27
<210> 99
   <211> 24
   <212> DNA
   <213> Artificial Sequence
<220>
   <223> Primer
<400> 99
   tgtaggtgat cccttcaatc ctcc 24
<210> 100
   <211> 25
   <212> DNA
   <213> Artificial Sequence
<220>
   <223> Primer
<220>
   <221> misc_feature
   <222> 15, 22
   <223> n = propynylated T
<220>
   <221> misc_feature
   <222> 19
   <223> n = propynylated C
<400> 100
   tatgtcaaaa gctgnggana angat 25
<210> 101
   <211> 28
   <212> DNA
   <213> Artificial Sequence
<220>
   <223> Primer
<400> 101
   tgtggacaat gatctccatt gctgcaat 28
<210> 102
   <211> 25
   <212> DNA
   <213> Artificial Sequence
<220>
   <223> Primer
<400> 102
   ttgcgtcaat ggcttatatc aaagg 25
<210> 103
   <211> 27
   <212> DNA
   <213> Artificial Sequence
<220>
   <223> Primer
<400> 103
   tacagatttc agcaagttca atcaagc 27
<210> 104
   <211> 26
   <212> DNA
   <213> Artificial Sequence
<220>
   <223> Primer
<400> 104
   ttcaatcaag catttcggta tgaaac 26
<210> 105
   <211> 26
   <212> DNA
   <213> Artificial Sequence
<220>
   <223> Primer
<400> 105
   tcacgagatc tgcagtttat gagtaa 26
<210> 106
   <211> 32
   <212> DNA
   <213> Artificial Sequence
<220>
   <223> Primer
<400> 106
   tatatatcac gagatctgca gtttatgagt aa 32
<210> 107
   <211> 27
   <212> DNA
   <213> Artificial Sequence
<220>
   <223> Primer
<400> 107
   tatatcacgt gatctgcagt ttatgag 27
<210> 108
   <211> 28
   <212> DNA
   <213> Artificial Sequence
<220>
   <223> Primer
<400> 108
   tcctaagagt gggaccatgg ataaacac 28
<210> 109
   <211> 25
   <212> DNA
   <213> Artificial Sequence
<220>
   <223> Primer
<400> 109
   tcctaagagt gggaccatgg ataaa 25
<210> 110
   <211> 25
   <212> DNA
   <213> Artificial Sequence
<220>
   <223> Primer
<400> 110
   taagagtggg accatggata aacac 25
<210> 111
   <211> 27
   <212> DNA
   <213> Artificial Sequence
<220>
   <223> Primer
<400> 111
   tcagtgtagg tagaatgttt gcaatgc 27
<210> 112
   <211> 25
   <212> DNA
   <213> Artificial Sequence
<220>
   <223> Primer
<400> 112
   taggtagaat gtttgcaatg caacc 25
<210> 113
   <211> 23
   <212> DNA
   <213> Artificial Sequence
<220>
   <223> Primer
<400> 113
   tgtaggtaga atgtttgcaa tgc 23
<210> 114
   <211> 26
   <212> DNA
   <213> Artificial Sequence
<220>
   <223> Primer
<400> 114
   tgccttaatt ggagatatga gaccat 26
<210> 115
   <211> 26
   <212> DNA
   <213> Artificial Sequence
<220>
   <223> Primer
<400> 115
   tgcctgaatt ggagatatga gaccat 26
<210> 116
   <211> 32
   <212> DNA
   <213> Artificial Sequence
<220>
   <223> Primer
<400> 116
   taggatggtg atatggttga tacaggcttt gg 32
<210> 117
   <211> 32
   <212> DNA
   <213> Artificial Sequence
<220>
   <223> Primer
<400> 117
   taggatggcg atatggttga cacaggcttt gg 32
<210> 118
   <211> 27
   <212> DNA
   <213> Artificial Sequence
<220>
   <223> Primer
<400> 118
   tactgttatt caggatggtg atatggt 27
<210> 119
   <211> 24
   <212> DNA
   <213> Artificial Sequence
<220>
   <223> Primer
<400> 119
   ttcagatgtc tgtgtggcgg ccta 24
<210> 120
   <211> 28
   <212> DNA
   <213> Artificial Sequence
<220>
   <223> Primer
<400> 120
   tggaaatcct ttttctcaag gacgtggt 28
<210> 121
   <211> 32
   <212> DNA
   <213> Artificial Sequence
<220>
   <223> Primer
<400> 121
   tagatgatag tgacattgca tataaatatg ca 32

<210> 122
   <211> 26
   <212> DNA
   <213> Artificial Sequence
<220>
   <223> Primer
<400> 122
   tatggtgcag tgggcatttg ataatg 26
<210> 123
   <211> 24
   <212> DNA
   <213> Artificial Sequence
<220>
   <223> Primer
<400> 123
   tccacctgtg gttattgaac ctgt 24
<210> 124
   <211> 20
   <212> DNA
   <213> Artificial Sequence
<220>
   <223> Primer
<400> 124
   tgacgaacca cagcgtcaca 20
<210> 125
   <211> 20
   <212> DNA
   <213> Artificial Sequence
<220>
   <223> Primer
<400> 125
   tcgggatgta atggctggtt 20
<210> 126
   <211> 29
   <212> DNA
   <213> Artificial Sequence
<220>
   <223> Primer
<400> 126
   tcaggatggt ttttggtaga ggctatagt 29
<210> 127
   <211> 28
   <212> DNA
   <213> Artificial Sequence
<220>
   <223> Primer
<400> 127
   tacacacaat tccttggaag cacaggca 28
<210> 128
   <211> 25
   <212> DNA
   <213> Artificial Sequence
<220>
   <223> Primer
<400> 128
   tgaactaacg gacagtggat atggc 25
<210> 129
   <211> 29
   <212> DNA
   <213> Artificial Sequence
<220>
   <223> Primer
<400> 129
   tcaatgtatc ttatcatgtc tgggtcccc 29
<210> 130
   <211> 29
   <212> DNA
   <213> Artificial Sequence
<220>
   <223> Primer
<400> 130
   tatcttatca tgtctgggtc cccaggaag 29
<210> 131
   <211> 22
   <212> DNA
   <213> Artificial Sequence
<220>
   <223> Primer
<400> 131
   tcatgtctgg gtcccctgga ag 22
<210> 132
   <211> 24
   <212> DNA
   <213> Artificial Sequence
<220>
   <223> Primer
<400> 132
   tgtgccctca aaaaccctga cctc 24
<210> 133
   <211> 24
   <212> DNA
   <213> Artificial Sequence
<220>
   <223> Primer
<400> 133
   tgtgccctca aaaaccctaa cctc 24
<210> 134
   <211> 22
   <212> DNA
   <213> Artificial Sequence
<220>
   <223> Primer
<400> 134
   tgccattcat aggctgccca tc 22
<210> 135
   <211> 25
   <212> DNA
   <213> Artificial Sequence
<220>
   <223> Primer
<400> 135
   tgaggcctat agcagctata gcctc 25
<210> 136
   <211> 26
   <212> DNA
   <213> Artificial Sequence
<220>
   <223> Primer
<400> 136
   tccctctaca gtagcaacgg atgcaa 26
<210> 137
   <211> 22
   <212> DNA
   <213> Artificial Sequence
<220>
   <223> Primer
<400> 137
   tctacagtag caacggatgc aa 22
<210> 138
   <211> 23
   <212> DNA
   <213> Artificial Sequence
<220>
   <223> Primer
<400> 138
   taggggccca accccatttt cat 23
<210> 139
   <211> 20
   <212> DNA
   <213> Artificial Sequence
<220>
   <223> Primer
<400> 139
   tcacctttgc aaaggggccc 20
<210> 140
   <211> 21
   <212> DNA
   <213> Artificial Sequence
<220>
   <223> Primer
<400> 140
   tacggtcaca gcttcccaca t 21
<210> 141
   <211> 24
   <212> DNA
   <213> Artificial Sequence
<220>
   <223> Primer
<400> 141
   tcctagaagt agaggcagca tcca 24
<210> 142
   <211> 24
   <212> DNA
   <213> Artificial Sequence
<220>
   <223> Primer
<400> 142
   tggaggctcc gttgtctgca tgta 24
<210> 143
   <211> 32
   <212> DNA
   <213> Artificial Sequence
<220>
   <223> Primer
<220>
   <221> misc_feature
   <222> 29
   <223> n = I
<400> 143
   taggatggtg atatggttga tacaggctnt gg 32
<210> 144
   <211> 32
   <212> DNA
   <213> Artificial Sequence
<220>
   <223> Primer
<220>
   <221> misc_feature
   <222> 27, 29
   <223> n = I
<400> 144
   taggatggtg atatggttga tacaggntnt gg 32
<210> 145
   <211> 32
   <212> DNA
   <213> Artificial Sequence
<220>
   <223> Primer
<220>
   <221> misc_feature
   <222> 24, 27, 29
   <223> n = I
<400> 145
   taggatggtg atatggttga tacnggntnt gg 32
<210> 146
   <211> 27
   <212> DNA
   <213> Artificial Sequence
<220>
   <223> Primer
<220>
   <221> misc_feature
   <222> 10
   <223> n = I
<400> 146
   tactgttatn caggatggtg atatggt 27
<210> 147
   <211> 27
   <212> DNA
   <213> Artificial Sequence
<220>
   <223> Primer
<220>
   <221> misc_feature
   <222> 19
   <223> n = I
<400> 147
   tactgttatt caggatggng atatggt 27
<210> 148
   <211> 27
   <212> DNA
   <213> Artificial Sequence
<220>
   <223> Primer
<220>
   <221> misc_feature
   <222> 10, 19
   <223> n = I
<400> 148
   tactgttatn caggatggng atatggt 27
<210> 149
   <211> 24
   <212> DNA
   <213> Artificial Sequence
<220>
   <223> Primer
<220>
   <221> misc_feature
   <222> 19
   <223> n = I
<400> 149
   ttcagatgtc tgtgtggcng ccta 24
<210> 150
   <211> 24
   <212> DNA
   <213> Artificial Sequence
<220>
   <223> Primer
<220>
   <221> misc_feature
   <222> 12, 19
   <223> n = I
<400> 150
   ttcagatgtc tntgtggcng ccta 24
<210> 151
   <211> 32
   <212> DNA
   <213> Artificial Sequence
<220>
   <223> Primer
<220>
   <221> misc_feature
   <222> 14, 17, 24
   <223> n = I
<400> 151
   tagatgatag tganatngca tatnaatatg ca 32
<210> 152
   <211> 26
   <212> DNA
   <213> Artificial Sequence
<220>
   <223> Primer
<220>
   <221> misc_feature
   <222> 18
   <223> n = I
<400> 152
   tatggtgcag tgggcatntg ataatg 26
<210> 153
   <211> 28
   <212> DNA
   <213> Artificial Sequence
<220>
   <223> Primer
<400> 153
   tagggatttt tgacccatct ttttctca 28
<210> 154
   <211> 29
   <212> DNA
   <213> Artificial Sequence
<220>
   <223> Primer
<400> 154
   ttacaaagag ggccaacgcc attctcatc 29
<210> 155
   <211> 21
   <212> DNA
   <213> Artificial Sequence
<220>
   <223> Primer
<400> 155
   tcctcccaca gcaaacatgt g 21
<210> 156
   <211> 26
   <212> DNA
   <213> Artificial Sequence
<220>
   <223> Primer
<400> 156
   tgtaaatcta gtggctctcc tcccac 26
<210> 157
   <211> 28
   <212> DNA
   <213> Artificial Sequence
<220>
   <223> Primer
<400> 157
   ttgcaaagag gcccaacccc attctcat 28
<210> 158
   <211> 28
   <212> DNA
   <213> Artificial Sequence
<220>
   <223> Primer
<220>
   <221> misc_feature
   <222> 24
   <223> n = I
<400> 158
   ttgcaaagag gcccaacccc attntcat 28
<210> 159
   <211> 24
   <212> DNA
   <213> Artificial Sequence
<220>
   <223> Primer
<400> 159
   tgccacgacc ggcaagacca acat 24
<210> 160
   <211> 29
   <212> DNA
   <213> Artificial Sequence
<220>
   <223> Primer
<400> 160
   tgcgttaact ggaccaatga gaactttcc 29
<210> 161
   <211> 27
   <212> DNA
   <213> Artificial Sequence
<220>
   <223> Primer
<220>
   <221> misc_feature
   <222> 22
   <223> n = I
<400> 161
   taccaacatc gcggaggcta tngccca 27
<210> 162
   <211> 26
   <212> DNA
   <213> Artificial Sequence
<220>
   <223> Primer
<400> 162
   tgggtcctgc ccacctacaa caacca 26
<210> 163
   <211> 22
   <212> DNA
   <213> Artificial Sequence
<220>
   <223> Primer
<400> 163
   tcctgcccac ctacaacaac ca 22
<210> 164
   <211> 18
   <212> DNA
   <213> Artificial Sequence
<220>
   <223> Primer
<400> 164
   tggtgccgat ggagtggg 18
<210> 165
   <211> 18
   <212> DNA
   <213> Artificial Sequence
<220>
   <223> Primer
<400> 165
   tggtgccgac ggagtggg 18
<210> 166
   <211> 21
   <212> DNA
   <213> Artificial Sequence
<220>
   <223> Primer
<400> 166
   taccccctgg gggtactttg a 21
<210> 167
   <211> 30
   <212> DNA
   <213> Artificial Sequence
<220>
   <223> Primer
<400> 167
   tgggggtatt ttgacttcaa ccgattccac 30
<210> 168
   <211> 30
   <212> DNA
   <213> Artificial Sequence
<220>
   <223> Primer
<220>
   <221> misc_feature
   <222> 22, 24
   <223> n = I
<400> 168
   tgggggtatt ttgacttcaa cngnttccac 30
<210> 169
   <211> 24
   <212> DNA
   <213> Artificial Sequence
<220>
   <223> Primer
<400> 169
   tcctcgggaa attggcattg cgat 24
<210> 170
   <211> 23
   <212> DNA
   <213> Artificial Sequence
<220>
   <223> Primer
<400> 170
   tgattgtcgc cttgaaccat tgc 23
<210> 171
   <211> 25
   <212> DNA
   <213> Artificial Sequence
<220>
   <223> Primer
<400> 171
   tgttgtgcac ttttggagaa tattt 25
<210> 172
   <211> 22
   <212> DNA
   <213> Artificial Sequence
<220>
   <223> Primer
<400> 172
   ttttggcgaa tattttgttt gg 22
<210> 173
   <211> 20
   <212> DNA
   <213> Artificial Sequence
<220>
   <223> Primer
<400> 173
   tcctttgcca tcccattgtc 20
<210> 174
   <211> 18
   <212> DNA
   <213> Artificial Sequence
<220>
   <223> Primer
<400> 174
   tgggcaatga gggtcact 18
<210> 175
   <211> 20
   <212> DNA
   <213> Artificial Sequence
<220>
   <223> Primer
<400> 175
   tcatttagcc tctgtgcaaa 20
<210> 176
   <211> 22
   <212> DNA
   <213> Artificial Sequence
<220>
   <223> Primer
<400> 176
   ttgtcaatat catccagttg gc 22
<210> 177
   <211> 18
   <212> DNA
   <213> Artificial Sequence
<220>
   <223> Primer
<400> 177
   tactctaaca gcatccat 18
<210> 178
   <211> 23
   <212> DNA
   <213> Artificial Sequence
<220>
   <223> Primer
<400> 178
   ttctcagcta acaatttctc agc 23
<210> 179
   <211> 18
   <212> DNA
   <213> Artificial Sequence
<220>
   <223> Primer
<400> 179
   tgctaacaat ttctcagc 18
<210> 180
   <211> 21
   <212> DNA
   <213> Artificial Sequence
<220>
   <223> Primer
<400> 180
   tctttcccct ctgtattcta a 21
<210> 181
   <211> 24
   <212> DNA
   <213> Artificial Sequence
<220>
   <223> Primer
<400> 181
   tgaaccaatt gcattagtct cact 24
<210> 182
   <211> 19
   <212> DNA
   <213> Artificial Sequence
<220>
   <223> Primer
<400> 182
   taattgcatt agtctcact 19
<210> 183
   <211> 23
   <212> DNA
   <213> Artificial Sequence
<220>
   <223> Primer
<400> 183
   tgtaaccagc agaataggct ttg 23
<210> 184
   <211> 16
   <212> DNA
   <213> Artificial Sequence
<220>
   <223> Primer
<400> 184
   tacagaatag gctttg 16
<210> 185
   <211> 21
   <212> DNA
   <213> Artificial Sequence
<220>
   <223> Primer
<400> 185
   tgtttatcca tggtcccact c 21
<210> 186
   <211> 20
   <212> DNA
   <213> Artificial Sequence
<220>
   <223> Primer
<400> 186
   tctaatattg tgtttatcca 20
<210> 187
   <211> 17
   <212> DNA
   <213> Artificial Sequence
<220>
   <223> Primer
<400> 187
   tcaggggtcc ttctata 17
<210> 188
   <211> 25
   <212> DNA
   <213> Artificial Sequence
<220>
   <223> Primer
<400> 188
   tgaggatgtg tccaagatgg ctgcg 25
<210> 189
   <211> 25
   <212> DNA
   <213> Artificial Sequence
<220>
   <223> Primer
<400> 189
   tgcgggaaag gcgggagttg aagat 25
<210> 190
   <211> 25
   <212> DNA
   <213> Artificial Sequence
<220>
   <223> Primer
<400> 190
   tgccgaggcc tatgtggtcg acatt 25
<210> 191
   <211> 25
   <212> DNA
   <213> Artificial Sequence
<220>
   <223> Primer
<400> 191
   tagggagatt gggagctccc gtatt 25
<210> 192
   <211> 23
   <212> DNA
   <213> Artificial Sequence
<220>
   <223> Primer
<400> 192
   tcgggcccac tatgacgtgt aca 23
<210> 193
   <211> 25
   <212> DNA
   <213> Artificial Sequence
<220>
   <223> Primer
<400> 193
   tggtaatcaa aatactgcgg gccaa 25
<210> 194
   <211> 27
   <212> DNA
   <213> Artificial Sequence
<220>
   <223> Primer
<400> 194
   tccgtggatt gttctgtagc agtcttc 27
<210> 195
   <211> 27
   <212> DNA
   <213> Artificial Sequence
<220>
   <223> Primer
<400> 195
   tcagcaaatt gttctgctgc tgcacta 27
<210> 196
   <211> 26
   <212> DNA
   <213> Artificial Sequence
<220>
   <223> Primer
<400> 196
   tagcaaattg ctttgctgtt gcacta 26
<210> 197
   <211> 24
   <212> DNA
   <213> Artificial Sequence
<220>
   <223> Primer
<400> 197
   tggtcttctg gggcttgttc catc 24
<210> 198
   <211> 27
   <212> DNA
   <213> Artificial Sequence
<220>
   <223> Primer
<400> 198
   tttggtcctt gtcttatgtc cagaatg 27
<210> 199
   <211> 25
   <212> DNA
   <213> Artificial Sequence
<220>
   <223> Primer
<400> 199
   tctaaacgca catccccatg aattt 25
<210> 200
   <211> 27
   <212> DNA
   <213> Artificial Sequence
<220>
   <223> Primer
<400> 200
   tgtctaaacg cacatcccca tgaattt 27
<210> 201
   <211> 28
   <212> DNA
   <213> Artificial Sequence
<220>
   <223> Primer
<400> 201
   tgtcatatcc cctattcctc cccttctt 28
<210> 202
   <211> 32
   <212> DNA
   <213> Artificial Sequence
<220>
   <223> Primer <400> 202

   tcctattcct ccccttcttt taaaattcat gc 32
<210> 203
   <211> 24
   <212> DNA
   <213> Artificial Sequence
<220>
   <223> Primer <400> 203

   tggtctggaa aagacagggt tggg 24
<210> 204
   <211> 25
   <212> DNA
   <213> Artificial Sequence
<220>
   <223> Primer
<400> 204
   tgaggggagt cgagggataa ggaac 25
<210> 205
   <211> 28
   <212> DNA
   <213> Artificial Sequence
<220>
   <223> Primer
<400> 205
   tcgtttgtag ggaacattgg tgaggaag 28
<210> 206
   <211> 23
   <212> DNA
   <213> Artificial Sequence
<220>
   <223> Primer
<400> 206
   tggggctcat ggtcattgtc atc 23
<210> 207
   <211> 25
   <212> DNA
   <213> Artificial Sequence
<220>
   <223> Primer
<400> 207
   tgggaaagct ggtagaggta catgc 25
<210> 208
   <211> 23
   <212> DNA
   <213> Artificial Sequence
<220>
   <223> Primer
<400> 208
   tgagtgattg gcggggtaag gac 23
<210> 209
   <211> 26
   <212> DNA
   <213> Artificial Sequence
<220>
   <223> Primer
<400> 209
   tacttgggat tgtttgtgtg agacgg 26
<210> 210
   <211> 22
   <212> DNA
   <213> Artificial Sequence
<220>
   <223> Primer
<400> 210
   tcattgtggt gggtaggtcg tc 22
<210> 211
   <211> 25
   <212> DNA
   <213> Artificial Sequence
<220>
   <223> Primer
<400> 211
   tggtgtttgg agtggctatt ggcag 25
<210> 212
   <211> 25
   <212> DNA
   <213> Artificial Sequence
<220>
   <223> Primer
<400> 212
   tcttgctttg acatgttgtg gtgga 25
<210> 213
   <211> 25
   <212 DNA
   <213> Artificial Sequence
<220>
   <223> Primer
<400> 213
   tgttgaggac ggctgctaat tgttg 25
<210> 214
   <211> 23
   <212> DNA
   <213> Artificial Sequence
<220>
   <223> Primer
<400> 214
   tttgagttgt gggcagtccc ttt 23
<210> 215
   <211> 24
   <212> DNA
   <213> Artificial Sequence
<220>
   <223> Primer
<400> 215
   tcctgcttga tggcctgtaa gtct 24
<210> 216
   <211> 24
   <212> DNA
   <213> Artificial Sequence
<220>
   <223> Primer
<400> 216
   tgaggctgga tctatccacg caaa 24
<210> 217
   <211> 25
   <212> DNA
   <213> Artificial Sequence
<220>
   <223> Primer
<400> 217
   tagtcgttgg tccgttgtta gggaa 25
<210> 218
   <211> 25
   <212> DNA
   <213> Artificial Sequence
<220>
   <223> Primer
<400> 218
   tgttccgcag actactatgg ctctc 25
<210> 219
   <211> 22
   <212> DNA
   <213> Artificial Sequence
<220>
   <223> Primer
<400> 219
   tggcaattcc ggtgtactca cc 22
<210> 220
   <211> 26
   <212> DNA
   <213> Artificial Sequence
<220>
   <223> Primer
<400> 220
   tactcaccgg ttccgcagac cactat 26
<210> 221
   <211> 25
   <212> DNA
   <213> Artificial Sequence
<220>
   <223> Primer
<400> 221
   tgttccgcag accactatgg ctctc 25
<210> 222
   <211> 22
   <212> DNA
   <213> Artificial Sequence
<220>
   <223> Primer
<400> 222
   tcgcaagcac cctatcaggc ag 22
<210> 223
   <211> 23
   <212> DNA
   <213> Artificial Sequence
<220>
   <223> Primer
<400> 223
   tgaatgtacc ccatgaggtc ggc 23
<210> 224
   <211> 27
   <212> DNA
   <213> Artificial Sequence
<220>
   <223> Primer
<400> 224
   tcgacacatt ggaggagcat gatgtta 27
<210> 225
   <211> 30
   <212> DNA
   <213> Artificial Sequence
<220>
   <223> Primer
<400> 225
   tgttccactt cccaagttta catcttcctc 30
<210> 226
   <211> 23
   <212> DNA
   <213> Artificial Sequence
<220>
   <223> Primer
<400> 226
   tcaggagctt tcgtgtccac ctt 23
<210> 227
   <211> 21
   <212> DNA
   <213> Artificial Sequence
<220>
   <223> Primer
<400> 227
   tagctccgag ccacatgaac c 21
<210> 228
   <211> 22
   <212> DNA
   <213> Artificial Sequence
<220>
   <223> Primer
<400> 228
   tcaggctgcc acaccagatg tc 22
<210> 229
   <211> 25
   <212> DNA
   <213> Artificial Sequence
<220>
   <223> Primer
<400> 229
   tccacatgaa ccaaatggct ctgct 25
<210> 230
   <211> 26
   <212> DNA
   <213> Artificial Sequence
<220>
   <223> Primer
<400> 230
   tactccgtct cgtacgactt tctgtt 26
<210> 231
   <211> 31
   <212> DNA
   <213> Artificial Sequence
<220>
   <223> Primer
<400> 231
   tgttgtgata acaaagtccc aatcatcgtt c 31
<210> 232
   <211> 26
   <212> DNA
   <213> Artificial Sequence
<220>
   <223> Primer
<400> 232
   tcgatcaggc tcaacatagc cctctt 26
<210> 233
   <211> 26
   <212> DNA
   <213> Artificial Sequence
<220>
   <223> Primer
<400> 233
   tgcatgttga tgttgtccag catgat 26
<210> 234
   <211> 23
   <212> DNA
   <213> Artificial Sequence
<220>
   <223> Primer
<400> 234
   tgctgatctt ggctgtccac ctc 23
<210> 235
   <211> 24
   <212> DNA
   <213> Artificial Sequence
<220>
   <223> Primer
<400> 235
   tatatgataa aacgccgcag acac 24
<210> 236
   <211> 28
   <212> DNA
   <213> Artificial Sequence
<220>
   <223> Primer
<400> 236
   taggaatatg ataaaacgcc gcagacac 28
<210> 237
   <211> 29
   <212> DNA
   <213> Artificial Sequence
<220>
   <223> Primer
<400> 237
   tgaagaggaa tatgataaaa cgccgcaga 29
<210> 238
   <211> 25
   <212> DNA
   <213> Artificial Sequence z
<220>
   <223> Primer
<400> 238
   tacgggacgt aaacaaagga cgtcc 25
<210> 239
   <211> 19
   <212> DNA
   <213> Artificial Sequence
<220>
   <223> Primer
<400> 239
   tcaggcacag cttggaggc 19
<210> 240
   <211> 27
   <212> DNA
   <213> Artificial Sequence
<220>
   <223> Primer
<400> 240
   tgtctagact ctgtggtatt gtgagga 27
<210> 241
   <211> 28
   <212> DNA
   <213> Artificial Sequence
<220>
   <223> Primer
<400> 241
   tgctccccct agaaaattga gagaagtc 28
<210> 242
   <211> 28
   <212> DNA
   <213> Artificial Sequence
<220>
   <223> Primer
<400> 242
   tccagaagaa ccaacaagaa gatgaggc 28
<210> 243
   <211> 30
   <212> DNA
   <213> Artificial Sequence

<220>
   <223> Primer
<400> 243
   taatccagaa gaaccaacaa gaagatgagg 30
<210> 244
   <211> 23
   <212> DNA
   <213> Artificial Sequence
<220>
   <223> Primer
<400> 244
   tgataaaacg ccgcagacac atc 23
<210> 245
   <211> 24
   <212> DNA
   <213> Artificial Sequence
<220>
   <223> Primer
<400> 245
   tctctgggtg gggaaggagg ggag 24
<210> 246
   <211> 24
   <212> DNA
   <213> Artificial Sequence
<220>
   <223> Primer
<400> 246
   ttggcggggt gaggaccttg aggg 24
<210> 247
   <211> 21
   <212> DNA
   <213> Artificial Sequence
<220>
   <223> Primer
<400> 247
   tccatcgatg gggtcccagg t 21
<210> 248
   <211> 21
   <212> DNA
   <213> Artificial Sequence
<220>
   <223> Primer
<400> 248
   tggcatggtg ccaaactgat t 21
<210> 249
   <211> 21
   <212> DNA
   <213> Artificial Sequence
<220>
   <223> Primer
<220>
   <221> misc_feature
   <222> 7
   <223> n = I
<400> 249
   tggcatngtg ccaaactgat t 21
<210> 250
   <211> 21
   <212> DNA
   <213> Artificial Sequence
<220>
   <223> Primer
<400> 250
   tgtgttttcc caagctcttc c 21
<210> 251
   <211> 19
   <212> DNA
   <213> Artificial Sequence
<220>
   <223> Primer
<220>
   <221> misc_feature
   <222> 11
   <223> n = I
<400> 251
   tgttttccca ngccctccc 19
<210> 252
   <211> 23
   <212> DNA
   <213> Artificial Sequence
<220>
   <223> Primer
<400> 252
   tgctggcatg aaccaaactg att 23
<210> 253
   <211> 24
   <212> DNA
   <213> Artificial Sequence
<220>
   <223> Primer
<400> 253
   tggtcaaagc tggcatgaac caaa 24
<210> 254
   <211> 24
   <212> DNA
   <213> Artificial Sequence
<220>
   <223> Primer
<220>
   <221> misc_feature
   <222> 7, 11
   <223> n = I
<400> 254
   tggtcanagc nggcatgaac caaa 24
<210> 255
   <211> 24
   <212> DNA
   <213> Artificial Sequence
<220>
   <223> Primer
<220>
   <221> misc_feature
   <222> 7, 11
   <223> n = A,T,C or G
<220>
   <221> misc_feature
   <222> 20, 21
   <223> n = propynylated C
<400> 255
   tggtcanagc nggcatgaan naaa 24
<210> 256
   <211> 20
   <212> DNA
   <213> Artificial Sequence
<220>
   <223> Primer
<400> 256
   tcaacactgg tgttgtccca 20
<210> 257
   <211> 20
   <212> DNA
   <213> Artificial Sequence
<220>
   <223> Primer
<220>
   <221> misc_feature
   <222> 13, 14
   <223> n = propynylated T
<220>
   <221> misc_feature
   <222> 17, 18
   <223> n = propynylated C
<400> 257
   tcaacactgg tgnngtnnca 20
<210> 258
   <211> 26
   <212> DNA
   <213> Artificial Sequence
<220>
   <223> Primer
<400> 258
   tcaatctctc ccttaaccat cccatt 26
<210> 259
   <211> 30
   <212> DNA
   <213> Artificial Sequence
<220>
   <223> Primer
<400> 259
   tgtccataat ttctggcaat aaccttctat 30
<210> 260
   <211> 28
   <212> DNA
   <213> Artificial Sequence
<220>
   <223> Primer
<400> 260
   tggcttgatt gtctccttga accattgc 28
<210> 261
   <211> 30
   <212> DNA
   <213> Artificial Sequence
<220>
   <223> Primer
<400> 261
   tactcttgtt gtcacagcta tagcttgatt 30
<210> 262
   <211> 33
   <212> DNA
   <213> Artificial Sequence
<220>
   <223> Primer
<400> 262
   tggtactctt gatgtcacag ctatagcttg att 33
<210> 263
   <211> 27
   <212> DNA
   <213> Artificial Sequence
<220>
   <223> Primer
<400> 263
   tctcccatca tagtatatcc ttttgct 27
<210> 264
   <211> 25
   <212> DNA
   <213> Artificial Sequence
<220>
   <223> Primer
<400> 264
   tgcatgaata agggtctgaa gccca 25
<210> 265
   <211> 24
   <212> DNA
   <213> Artificial Sequence
<220>
   <223> Primer
<400> 265
   tgcttccatc cacgatatct catc 24
<210> 266
   <211> 33
   <212> DNA
   <213> Artificial Sequence
<220>
   <223> Primer
<400> 266
   tgcactagaa aacttcatct gggttgcagt atc 33
<210> 267
   <211> 33
   <212> DNA
   <213> Artificial Sequence
<220>
   <223> Primer
<400> 267
   tgcactagag aatttcatct gggttgcagt atc 33
<210> 268
   <211> 27
   <212> DNA
   <213> Artificial Sequence
<220>
   <223> Primer
<400> 268
   ttctgcaatt acttgacacg acctcat 27
<210> 269
   <211> 25
   <212> DNA
   <213> Artificial Sequence
<220>
   <223> Primer
<400> 269
   tagggtcact tggaggattg aaagg 25
<210> 270
   <211> 25
   <212> DNA
   <213> Artificial Sequence
<220>
   <223> Primer
<400> 270
   ttgcagagat ggagatcatt gtcca 25
<210> 271
   <211> 24
   <212> DNA
   <213> Artificial Sequence
<220>
   <223> Primer
<220>
   <221> misc_feature
   <222> 12
   <223> n = propynylated T
<220>
   <221> misc_feature
   <222> 13, 15, 16, 22
   <223> n = propynylated C
<400> 271
   ttgcttggtt annannctga anca 24
<210> 272
   <211> 24
   <212> DNA
   <213> Artificial Sequence
<220>
   <223> Primer
<400> 272
   tggaccatac aggcaacccg acaa 24
<210> 273
   <211> 25
   <212> DNA
   <213> Artificial Sequence
<220>
   <223> Primer
<400> 273
   tatccccgaa agcccagata tatac 25
<210> 274
   <211> 21
   <212> DNA
   <213> Artificial Sequence
<220>
   <223> Primer
<400> 274
   ttgtgcacca tgcagttcat c 21
<210> 275
   <211> 28
   <212> DNA
   <213> Artificial Sequence
<220>
   <223> Primer
<400> 275
   tgaagtcatc cagtatagtg tttatcca 28
<210> 276
   <211> 21
   <212> DNA
   <213> Artificial Sequence
<220>
   <223> Primer
<400> 276
   tgtttatcca cggtcccact c 21
<210> 277
   <211> 30
   <212> DNA
   <213> Artificial Sequence
<220>
   <223> Primer
<400> 277
   taatagactt tcccctctat attctaattc 30
<210> 278
   <211> 34
   <212> DNA
   <213> Artificial Sequence
<220>
   <223> Primer
<400> 278
   tactgcataa taggctttcc cctctaaatt ctaa 34
<210> 279
   <211> 30
   <212> DNA
   <213> Artificial Sequence
<220>
   <223> Primer
<400> 279
   taataggctt tcccctctat attctaattc 30
<210> 280
   <211> 29
   <212> DNA
   <213> Artificial Sequence
<220>
   <223> Primer
<400> 280
   tcagctatca atttctctgc caatatttg 29
<210> 281
   <211> 29
   <212> DNA
   <213> Artificial Sequence
<220>
   <223> Primer
<400> 281
   tcagctatca ttttctcagc caagatttg 29
<210> 282
   <211> 26
   <212> DNA
   <213> Artificial Sequence
<220>
   <223> Primer
<400> 282
   tagatttcat ttagcctctg tgcaaa 26
<210> 283
   <211> 20
   <212> DNA
   <213> Artificial Sequence
<220>
   <223> Primer
<400> 283
   tcgggagggc aatgagggtc 20
<210> 284
   <211> 34
   <212> DNA
   <213> Artificial Sequence
<220>
   <223> Primer
<400> 284
   tactgcataa tagactttcc cctctatatt ctaa 34
<210> 285
   <211> 35
   <212> DNA
   <213> Artificial Sequence
<220>
   <223> Primer
<400> 285
   tctgcaacca tttgcaaata atctggatat ttgca 35
<210> 286
   <211> 32
   <212> DNA
   <213> Artificial Sequence
<220>
   <223> Primer
<400> 286
   tctgcaacca tttgcaaata atctggatat tt 32
<210> 287
   <211> 32
   <212> DNA
   <213> Artificial Sequence
<220>
   <223> Primer
<400> 287
   tcggcagcca tttgcaaata atcaggatat tt 32
<210> 288
   <211> 30
   <212> DNA
   <213> Artificial Sequence
<220>
   <223> Primer
<400> 288
   tacatattca tccgtgctta caaccttaga 30
<210> 289
   <211> 30
   <212> DNA
   <213> Artificial Sequence
<220>
   <223> Primer
<400> 289
   tagtattttg tcctgccacg catttaaacg 30
<210> 290
   <211> 28
   <212> DNA
   <213> Artificial Sequence
<220>
   <223> Primer
<400> 290
   tttctgctcg tttataatgt ctacacat 28
<210> 291
   <211> 26
   <212> DNA
   <213> Artificial Sequence
<220>
   <223> Primer
<400> 291
   ttgcttttta aaaatgcagc tgcatt 26
<210> 292
   <211> 28
   <212> DNA
   <213> Artificial Sequence
<220>
   <223> Primer
<400> 292
   tatttgcctg ccaattgctt tttaaaaa 28
<210> 293
   <211> 23
   <212> DNA
   <213> Artificial Sequence
<220>
   <223> Primer
<400> 293
   tcaaacccag aggtgcctgt aaa 23
<210> 294
   <211> 21
   <212> DNA
   <213> Artificial Sequence
<220>
   <223> Primer
<400> 294
   tgcacacaac ggacacacaa a 21
<210> 295
   <211> 24
   <212> DNA
   <213> Artificial Sequence
<220>
   <223> Primer
<400> 295
   taccatgtcc gaacctgtat ctgt 24
<210> 296
   <211> 31
   <212> DNA
   <213> Artificial Sequence
<220>
   <223> Primer
<400> 296
   tgcctgtgct tccaaggaat tgtgtgtaat a 31
<210> 297
   <211> 25
   <212> DNA
   <213> Artificial Sequence
<220>
   <223> Primer
<400> 297
   ttaggtcctg cacagccgca taatg 25
<210> 298
   <211> 22
   <212> DNA
   <213> Artificial Sequence
<220>
   <223> Primer
<400> 298
   tcgccatgtc tctctacctg cg 22
<210> 299
   <211> 23
   <212> DNA
   <213> Artificial Sequence
<220>
   <223> Primer
<400> 299
   tgagagtgga tgggcagcct atg 23
<210> 300
   <211> 19
   <212> DNA
   <213> Artificial Sequence
<220>
   <223> Primer
<400> 300
   tctggaaccc agcagtgga 19
<210> 301
   <211> 24
   <212> DNA
   <213> Artificial Sequence
<220>
   <223> Primer
<400> 301
   tagctgaaat tgctgctgga gagg 24
<210> 302
   <211> 28
   <212> DNA
   <213> Artificial Sequence
<220>
   <223> Primer
<400> 302
   tgggggacct aattgctact gtatctga 28
<210> 303
   <211> 21
   <212> DNA
   <213> Artificial Sequence
<220>
   <223> Primer
<400> 303
   tgtatctgaa gctgctgctg c 21
<210> 304
   <211> 27
   <212> DNA
   <213> Artificial Sequence
<220>
   <223> Primer
<400> 304
   tccagaccct gcaaaaaatg agaacac 27
<210> 305
   <211> 29
   <212> DNA
   <213> Artificial Sequence
<220>
   <223> Primer
<400> 305
   tgggtccctg atccaactag aaatgaaaa 29
<210> 306
   <211> 23
   <212> DNA
   <213> Artificial Sequence
<220>
   <223> Primer
<400> 306
   tctaaatgag gacctgacct gtg 23
<210> 307
   <211> 25
   <212> DNA
   <213> Artificial Sequence
<220>
   <223> Primer
<400> 307
   tgctccagga ggtgcaaatc aaaga 25
<210> 308
   <211> 35
   <212> DNA
   <213> Artificial Sequence
<220>
   <223> Primer
<220>
   <221> misc_feature
   <222> 33
   <223> n = I
<400> 308
   tctgcaacca tttgcaaata atctggatat ttnca 35
<210> 309
   <211> 35
   <212> DNA
   <213> Artificial Sequence
<220>
   <223> Primer
<220>
   <221> misc_feature
   <222> 15, 33
   <223> n = I
<400> 309
   tctgcaacca tttgnaaata atctggatat ttnca 35
<210> 310
   <211> 35
   <212> DNA
   <213> Artificial Sequence
<220>
   <223> Primer
<220>
   <221> misc_feature
   <222> 14, 15, 33
   <223> n = I
<400> 310
   tctgcaacca tttnnaaata atctggatat ttnca 35
<210> 311
   <211> 32
   <212> DNA
   <213> Artificial Sequence
<220>
   <223> Primer
<220>
   <221> misc_feature
   <222> 11, 12, 13
   <223> n = propynylated T
<400> 311
   tctgcaacca nnngaaaata atctggatat tt 32
<210> 312
   <211> 32
   <212> DNA
   <213> Artificial Sequence
<220>
   <223> Primer
<400> 312
   tctgcaacca tttgaaaata atctggatat tt 32
<210> 313
   <211> 32
   <212> DNA
   <213> Artificial Sequence
<220>
   <223> Primer
<220>
   <221> misc_feature
   <222> 15
   <223> n = I'
<400> 313
   tctgcaacca tttgnaaata atctggatat tt 32
<210> 314
   <211> 32
   <212> DNA
   <213> Artificial Sequence
<220>
   <223> Primer
<220>
   <221> misc_feature
   <222> 14, 15
   <223> n = I
<400> 314
   tctgcaacca tttnnaaata atctggatat tt 32
<210> 315
   <211> 32
   <212> DNA
   <213> Artificial Sequence
<220>
   <223> Primer
<220>
   <221> misc_feature
   <222> 14, 15, 17
   <223> n = I
<400> 315
   tctgcaacca tttnnanata atctggatat tt 32
<210> 316
   <211> 30
   <212> DNA
   <213> Artificial Sequence
<220>
   <223> Primer
<220>
   <221> misc_feature
   <222> 20
   <223> n = I
<400> 316
   tagtattttg tcctgccacn catttaaacg 30
<210> 317
   <211> 30
   <212> DNA
   <213> Artificial Sequence
<220>
   <223> Primer
<220>
   <221> misc_feature
   <222> 19, 20
   <223> n = I
<400> 317
   tagtattttg tcctgccann catttaaacg 30
<210> 318
   <211> 30
   <212> DNA
   <213> Artificial Sequence
<220>
   <223> Primer
<220>
   <221> misc_feature
   <222> 18, 19, 20
   <223> n = I
<400> 318
   tagtattttg tcctgccnnn catttaaacg 30
<210> 319
   <211> 26
   <212> DNA
   <213> Artificial Sequence
<220>
   <223> Primer
<220>
   <221> misc_feature
   <222> 20, 21
   <223> n = I <400> 319

   ttgcttttta aaaatgcagn ngcatt 26
<210> 320
   <211> 26
   <212> DNA
   <213> Artificial Sequence
<220>
   <223> Primer
<220>
   <221> misc feature
   <222> 18, 19, 20, 21
   <223> n = I
<400> 320
   ttgcttttta aaaatgcnnn ngcatt 26
<210> 321
   <211> 28
   <212> DNA
   <213> Artificial Sequence
<220>
   <223> Primer
<220>
   <221> misc_feature
   <222> 12, 13, 20
   <223> n = I
<400> 321
   tatttgcctg cnnattgctn tttaaaaa 28
<210> 322
   <211> 21
   <212> DNA
   <213> Artificial Sequence
<220>
   <223> Primer
<400> 322
   tgggcctctc tgcaaaggag a 21
<210> 323
   <211> 37
   <212> DNA
   <213> Artificial Sequence
<220>
   <223> Primer
<400> 323
   tgaaaacaca agatactttg gaacatacac aggaggt 37
<210> 324
   <211> 28
   <212> DNA
   <213> Artificial Sequence
<220>
   <223> Primer
<400> 324
   tgtgtctgta aagactgaag ttgttgga 28
<210> 325
   <211> 31
   <212> DNA
   <213> Artificial Sequence
<220>
   <223> Primer
<400> 325
   tgtaactgtt aaaactgagg ttgttggagt g 31
<210> 326
   <211> 36
   <212> DNA
   <213> Artificial Sequence
<220>
   <223> Primer
<220>
   <221> misc_feature
   <222> 18, 27, 34
   <223> n = I
<400> 326
   tgagaacaca agatactntg gaaactncac aggngg 36
<210> 327
   <211> 33
   <212> DNA
   <213> Artificial Sequence
<220>
   <223> Primer
<220>
   <221> misc_feature
   <222> 11
   <223> n = I
<400> 327
   tccagaccca nccaagaatg agaacacaag ata 33
<210> 328
   <211> 24
   <212> DNA
   <213> Artificial Sequence
<220>
   <223> Primer
<400> 328
   tgtcatcttg ccctcctccc acca 24
<210> 329
   <211> 24
   <212> DNA
   <213> Artificial Sequence
<220>
   <223> Primer
<400> 329
   tgtcattttg ccctcctccc acca 24
<210> 330
   <211> 28
   <212> DNA
   <213> Artificial Sequence
<220>
   <223> Primer
<400> 330
   tgaagggaaa gttctcattg gtccagtt 28
<210> 331
   <211> 28
   <212> DNA
   <213> Artificial Sequence
<220>
   <223> Primer
<400> 331
   tgttgaagtc aaaatacccc cagggggt 28
<210> 332
   <211> 26
   <212> DNA
   <213> Artificial Sequence
<220>
   <223> Primer
<400> 332
   tggcagtgga atcggttgaa gtcaaa 26
<210> 333
   <211> 24
   <212> DNA
   <213> Artificial Sequence
<220>
   <223> Primer
<400> 333
   tccatttgga atcgcaatgc caat 24
<210> 334
   <211> 24
   <212> DNA
   <213> Artificial Sequence
<220>
   <223> Primer
<400> 334
   tccatgggga atcgcaatgc caat 24
<210> 335
   <211> 25
   <212> DNA
   <213> Artificial Sequence
<220>
   <223> Primer
<400> 335
   tgttgttgat gagtctctgc cagtc 25
<210> 336
   <211> 25
   <212> DNA
   <213> Artificial Sequence
<220>
   <223> Primer
<220>
   <221> misc_feature
   <222> 16
   <223> n = I
<400> 336
   tgttgttgat gagtcnctgc cagtc 25
<210> 337
   <211> 25
   <212> DNA
   <213> Artificial Sequence
<220>
   <223> Primer
<400> 337
   tgtagaggtg gttgttgtag gtggg 25
<210> 338
   <211> 3215
   <212> DNA
   <213> Hepatitis B virus
<400> 338
   ctccacaaca ttccaccaag ctctgctaga tcccagagtg aggggcctat attttcctgc 60
   tggtggctcc agttccggaa cagtaaaccc tgttccgact actgcctcac ccatatcgtc 120
   aatcttctcg aggactgggg accctgcacc gaacatggag agcacaacat caggattcct 180
   aggacccctg ctcgtgttac aggcggggtt tttcttgttg acaagaatcc tcacaatacc 240
   acagagtcta gactcgtggt ggacttctct caattttcta gggggagcac ccacgtgtcc 300
   tggccaaaat tcgcagtccc caacctccaa tcactcacca acctcttgtc ctccaacttg 360
   tcctggctat cgctggatgt gtctgcggcg ttttatcata ttcctcttca tcctgctgct 420
   atgcctcatc ttcttgttgg ttcttctgga ctaccaaggt atgttgcccg tttgtcctct 480
   acttccagga acatcaacta ccagcacggg accatgcaga acctgcacga ttcctgctca 540
   aggaacctct atgtttccct cttgttgctg tacaaaacct tcggacggaa actgcacttg 600
   tattcccatc ccatcatcct gggctttcgc aagattccta tgggagtggg cctcagtccg 660
   tttctcctgg ctcagtttac tagtgccatt tgttcagtgg ttcgtagggc tttcccccac 720
   tgtttggctt tcagctatat ggatgatgtg gtattggggg ccaagtctgt acaacatctt 780
   gagtcccttt ttacctctat taccaatttt cttttgtctt tgggtataca tttgaaccct 840
   aataaaacca aacgttgggg ctactccctt aacttcatgg gatatgtaat tggaagttgg 900
   ggtactttac cgcaggaaca tattgtacaa aaactcaagc aatgttttcg aaaattgcct 960
   gtaaatagac ctattgattg gaaagtatgt caaagaattg tgggtctttt gggctttgct 1020
   gcccctttta cacaatgtgg ctatcctgcc ttgatgcctt tatatgcatg tatacaatct 1080
   aagcaggctt tcactttctc gccaacttac aaggcctttc tgtgtaaaca atatctaaac 1140
   ctttaccccg ttgcccggca acggtcaggt ctctgccaag tgtttgctga cgcaaccccc 1200
   acgggttggg gcttggccat aggccatcgg cgcatgcgtg gaacctttgt ggctcctctg 1260
   ccgatccata ctgcggaact cctagcagct tgttttgctc gcagccggtc tggagcgaaa 1320
   cttatcggaa ccgacaactc agttgtcctc tctcggaaat acacctcctt tccatggctg 1380
   ctaggctgtg ctgccaactg gatcctgcgc gggacgtcct ttgtctacgt cccgtcggcg 1440
   ctgaatcccg cggacgaccc gtctcggggc cgtttgggcc tctaccgtcc ccttcttcat 1500
   ctgccgttcc ggccgaccac ggggcgcacc tctctttacg cggtctcccc gtctgtgcct 1560
   tctcatctgc cggaccgtgt gcacttcgct tcacctctgc acgtagcatg gagaccaccg 1620
   tgaacgccca ccaggtcttg cccaaggtct tacacaagag gactcttgga ctctcagcaa 1680
   tgtcaacgac cgaccttgag gcatacttca aagactgttt gtttaaagac tgggaggagt 1740
   tgggggagga gattaggtta aaggtctttg tactaggagg ctgtaggcat aaattggtct 1800
   gttcaccagc accatgcaac tttttcccct ctgcctaatc atctcatgtt catgtcctac 1860
   tgttcaagcc tccaagctgt gccttgggtg gctttggggc atggacattg acccgtataa 1920
   agaatttgga gcttctgtgg agttactctc ttttttgcct tctgacttct ttccttctat 1980
   tcgagatctc ctcgacaccg cctctgctct gtatcgggag gccttagagt ctccggaaca 2040
   ttgttcacct caccatacag cactcaggca agctattctg tgttggggtg agttgatgaa 2100
   tctggccacc tgggtgggaa gtaatttgga agacccagca tccagggaat tagtagtcag 2160
   ctatgtcaat gttaatatgg gcctaaaaat tagacaacta ttgtggtttc acatttcctg 2220
   ccttactttt ggaagagaaa ctgtccttga gtatttggtg tcttttggag tgtggattcg 2280
   cactcctccc gcttacagac caccaaatgc ccctatctta tcaacacttc cggaaactac 2340
   tgttgttaga cgacgaggca ggtcccctag aagaagaact ccctcgcctc gcagacgaag 2400
   gtctcaatcg ccgcgtcgca gaagatctca atctcgggaa tctcaatgtt agtatccctt 2460
   ggactcataa ggtgggaaac tttactgggc tttattcttc tactgtacct gtctttaatc 2520
   ctgattggaa aactccctcc tttcctcaca ttcatttaca ggaggacatt attaatagat 2580
   gtcaacaata tgtgggccct ctgacagtta atgaaaaaag gagattaaaa ttaattatgc 2640
   ctgctaggtt ctatcctaac cttaccaaat atttgccctt ggacaaaggc attaaaccgt 2700
   attatcctga atatgcagtt aatcattact tcaaaactag gcattattta catactctgt 2760
   ggaaggctgg cattctatat aagagagaaa ctacacgcag cgcctcattt tgtgggtcac 2820
   catattcttg ggaacaagag ctacagcatg ggaggttggt cttccaaacc tcgacaaggc 2880
   atggggacga atctttctgt tcccaatcct ctgggattct ttcccgatca ccagttggac 2940
   cctgcgttcg gagccaactc aaacaatcca gattgggact tcaaccccaa caaggatcac 3000
   tggccagagg caaatcaggt aggagcggga gcatttggtc cagggttcac cccaccacac 3060
   ggaggccttt tggggtggag ccctcaggct cagggcatat tgacaacact gccagcagca 3120
   cctcctcctg cctccaccaa tcggcagtca ggaagacagc ctactcccat ctctccacct 3180
   ctaagagaca gtcatcctca ggccatgcag tggaa                            3215

## Description

### FIELD OF THE INVENTION

The present invention relates generally to the field of genetic identification and quantification of adventitious contaminant viruses and provides methods, compositions and kits useful for this purpose when combined with molecular mass or base composition analysis.

### BACKGROUND OF THE INVENTION

Adventitious viral agents represent a major risk associated with the use of cultured cells, cell lines, cell-substrate derived biologicals, including vaccines and antibodies for human use. Thus, methods are needed for detecting these viruses in cells and biologicals, as well as in human samples. The possibility for viral contamination exists in primary cultures and established cultures, as well as Master Cell Banks, end-of-production cells, and bulk harvest fluids. A major obstacle to the use of neoplastic-immortalized cells for which the mechanism of transformation is unknown is that these cells could have a higher risk of containing oncogenic viruses. Extensive testing for the presence of potential extraneous agents is therefore required to ensure the safety of the vaccines. Among the methods used for this purpose are animal inoculations, electron microscopy and in vitro molecular and antibody assays that provide a screen for viral agents.

A critical consideration for assessing safety is detection of endogenous retroviral sequences while using avian, murine, non-human primate, and human cell lines. Endogenous retroviral sequences are an integral part ofeukaryotic genomes, and while the majority of these sequences are defective, a few can produce infectious virus, either spontaneously upon long-term culture or inducibly following treatment with various chemical or other agents that may be part of normal production systems. The potential for activation of endogenous, infectious retrovirus in a cell substrate used for the production of biologics is an important safety concern, especially in the case of live viral vaccines, where purification and inactivation steps are minimized in order to preserve high vaccine potency.

The currently established methods for measuring reverse transcriptase (RT) activity include the highly sensitive, product-enhanced reverse transcriptase assays (PERT), which can detect 1-10 virions, and transmission electron microscopy (TEM), used to identify infective retroviruses particles. However, these techniques are not specific and do not provide any information regarding the source of the RT activity. PCR-based detection of retroviruses can be used in combination with other assays such as reverse transcriptase, electron microscopy infectivity or co-cultivation to increase the sensitivity of detection or to identify a particular adventitious agent present in the test sample. While some studies demonstrate that a low level of RT activity is not generally associated with a replicating agent, major concerns remain regarding the effects of such non-productive, non-replicating defective infections in the vaccine, which have the potential for host genome integration.

Retrovirus-induced tumorigenesis can involve the generation of a novel pathogenic virus by recombination between replication-competent and -defective sequences and/or activation of a cellular oncogene by a long terminal repeat (LTR) due to upstream or downstream insertion of retrovirus sequences. To address the possible integration of extraneous retroviral sequences in human cells by RT-containing particles, multiple PCR strategies have been used. These include direct PCR of DNase-treated inoculum using primers from the highly conserved pol region and Alu PCR using LTR primers in conjunction with Alu primers that specifically amplify viral-cellular DNA junctions of integrants.

Future strategies to detect adventitious agents will ideally address three fundamental problems. First, there are large numbers of known viral agents that are potential contaminants, each with a large number of potential strain variants. Second, history has shown that not all adventitious agents fall into anticipated families of viruses, so unanticipated virus families must also be considered. Third, the test must be practical to perform on a large number of samples in a standardized, high-throughput, quality-controlled fashion. The compounds, compositions and methods disclosed herein are based on use of recently developed and validated methods encompassing mass spectrometry analysis of broad-range PCR reactions for rapid, sensitive, cost-effective detection of broad ranges of bioagents to detect a broad range of adventitious viral agents, including previously unknown/uncharacterized viruses and endogenous retroviruses.

The Parvoviridae family includes the Parvovirinae sub-family, which comprises small single stranded DNA viruses that are about 4-5 kilobases long and includes multiple genera. For example, the *Dependovirus* genus includes the human helper-dependent adeno-associated virus (AAV) serotypes 1 to 8 (AAV 1-8) and the autonomous avian parvoviruses, the *Erythrovirus* genus includes the bovine, chipmunk, and autonomous primate parvoviruses, including human viruses B19 and V9, and the *Parvovirus* genus includes parvoviruses of other animals and rodents (except for chipmunks), carnivores, and pigs, including murine minute virus (MMV). These Parvovirinae members, or parvoviruses, can infect several cell types and have been described in clinical samples. AAVs in particular, have been implicated in decreased replication, propagation, and growth of other viruses

Exogenous retroviruses are known to cause various malignant and non-malignant diseases in animals over a wide range of species. These viruses infect most known animals and rodents. Examples include, but are not limited to: Deltaretroidvirus (HTLV 1-4, STLV 1-3), Gammaretrovirus (Murine leukemia virus, PERV), Alpharetrovirus: (Avian leucosis virus and Avian endogenous virus) and Human immunodeficiency viruses 1 and 2).

Polyomaviruses are small dsDNA viruses that can infect several species including humans, primates, rodents, rabbits and birds. Because of their tumorigenic and oncogenic potential, it is important to test for these viruses in cell substrates used for vaccine production.

The Papillomaviridae family of viruses contains more that 150 known species representing varying host-specificity and sequence homology. They have been identified in mammals (humans, simians, bovines, canines, ovines) and in birds. Majority of the human Papillomaviruses (HPVs), including all HPV types traditionally called genital and mucosal HPVs belong to supergroup A. Within supergroup A, there are 11 groups; the most medically important of these are the human Papillomaviruses HPV 16, HPV 18, HPV 31, HPV 45, HPV 11, HPV 6 and HPV 2. Each of these has been reported as "high risk" viruses in the medical literature.

Other viral families which are potential adventitious contaminants include, but are not limited to: Herpesviridae (Human herpesviruses 1 through 8, Bovine herpesvirus, Canine herpesvirus and Simian cytomegalovirus), Hepadnaviridae (including Hepatitis B virus[HBV]), Hepeviridae (Hepatitis E virus), Deltavirus (Hepatitus delta virus), Adenoviridae (Human adenoviruses A-F and murine adenovirus), Flaviviridae (Bovine viral diarrhea virus, TBE, Yellow fever virus, Dengue viruses 1-4, WNV and hepatitis C virus), , Togaviridae (Westemequine encephalomyelitis virus), Picornaviridae (Polio (types 1-13), Human hepatitis A, Human coxsackievirus, Human cardiovirus, Human rhinovirus and Bovine rhinovirus), Reoviridae (Mouse rotavirus, reovirus type 3 and Colorado tick fever virus), and Rhabdoviridae (vesicular stomatitis virus).

Paramyxoviridae represents two sub-families and seven genera that are distinct from one another. Some of the major viral genera in this family include Respirovirus (key species: human and simian parainfluenza virus 1 and 3-PIV1, PIV3, and Sendai virus), Pneumovirus, Rumulavirus and Avulavirus. A sub-family, Pneumovirinae, includes both Pneumovirus and Metapneumovirus genera. Some key members of these groups include human and bovine respiratory syncitial virus (RSV), human metapneumovirus (HMPV) and pneumonia virus of mice. The Paramyxoviridae family also includes Simian parainfluenza virus 5.

Provided herein are *inter alia*, methods of identifying adventitious contaminant viruses. Also provided are oligonucleotide primers, primer pairs, compositions and kits containing the oligonucleotide primers, which define viral bioagent identifying amplicons and, upon amplification, produce corresponding amplification products whose molecular masses provide the means to identify adventitious contaminant viruses at the species or sub-species level.

### Summary of the invention

The invention provides a method for identifying or determining the presence or absence of an adventitious contaminant virus in a sample, wherein said adventitious virus is a member of the *Dependovirus* genus, said method comprising: contacting nucleic acids from the sample with at least one primer pair, wherein each of the two primers of each of said at least one primer pair hybridizes to a conserved sequence region of a *Dependovirus* nucleic acid, wherein the two conserved sequence regions flank a variable region of *Dependovirus,* wherein each of said conserved sequence regions comprises at least a portion of a gene, wherein each of the at least one primer pair comprises at least 100% sequence identity with any primer pair selected from the group represented by SEQ ID NOs: 160:329, 161:329, 161:330, 163:332, 164:333, 165:334, and 168:336; amplifying the two conserved sequence regions and the variable region to produce an amplification product, determining the molecular mass of said amplification product by mass spectrometry; and comparing said determined molecular mass to a database comprising indexed molecular masses; wherein a match between said determined molecular mass and a molecular mass in the database indicates the presence of said *Dependovirus* in said sample.

The invention also provides a method for identifying or determining the presence or absence of an adventitious contaminant virus in a sample, wherein said adventitious virus is a member of the *Dependovirus* genus, said method comprising: contacting nucleic acids from the sample with at least one primer pair, wherein each of the two primers of each of said at least one primer pair hybridizes to a conserved sequence region of a *Dependovirus* nucleic acid, wherein the two conserved sequence regions flank a variable region of a *Dependovirus,* and wherein each of said conserved sequence regions comprises at least a portion of a gene, wherein each of the at least one primer pair comprises at least 100% sequence identity with any primer pair selected from the group represented by SEQ ID NOs: 160:329, 161:329, 161:330, 163:332, 164:333, 165:334, and 168:336; amplifying the two conserved sequence regions and the variable region to produce an amplification product, determining the molecular mass of said amplification product by mass spectrometry; calculating the base composition of said amplification product from said molecular mass; and comparing said calculated base composition to a database comprising indexed base compositions; wherein a match between said calculated base composition and a base composition in the database indicates the presence of said *Dependovirus* in said sample.

The invention also provides an oligonucleotide primer pair for identifying a contaminating *Dependovirus* comprising a forward and a reverse primer, wherein each primer hybridize to a nucleic acid encoding an NS1 protein or a nucleic acid encoding a VP1 protein, said oligonucleotide primer pair comprising at least 100% sequence identity with any primer pair selected from the group represented by SEQ ID NOs: 160:329, 161:329, 161:330, 163:332, 164:333, 165:334, and 168:336.

The invention also provides a composition comprising an oligonucleotide primer pair of the invention.

The invention also provides a kit for identification or detection of a contaminating *Dependovirus* comprising a composition of the invention.

The invention also provides a kit for identification or detection of one or more *Dependovirus* comprising: a first primer pair having at least 100% sequence identity with the primer pair represented by SEQ ID NOs: 160:329, a second primer pair having at least 100% sequence identity with the primer pair represented by SEQ ID NOs: 161:329, a third primer pair having at least 100% sequence identity with the primer pair represented by SEQ ID NOs: 161:330, a fourth primer pair having at least 100% sequence identity with the primer pair represented by SEQ ID NOs: 163:332, a fifth primer pair having at least 100% sequence identity with the primer pair represented by SEQ ID NOs: 164:333, a sixth primer pair having at least 100% sequence identity with the primer pair represented by SEQ ID NOs: 165:334, and a seventh primer pair having at least 100% sequence identity with the primer pair represented by SEQ ID NOs: 168:336.

### SUMMARY OF THE DISCLOSURE

Disclosed herein are *inter alia* methods of detecting, determining the presence or absence of, identifying or quantifying adventitious contaminant viruses. Also disclosed are compounds, including oligonucleotide primers and primer pairs that hybridize to conserved sequence regions, each pair of which flanks a variable region, or intervening variable region, defining a viral bioagent identifying amplicon. Also disclosed are compositions and kits comprising the compounds disclosed herein, and methods for using such primers, primer pairs, compositions and kits for identification, quantification, detection and/or determination of the presence or absence of adventitious contaminant viruses. The primers are designed to amplify the conserved sequence regions and variable region to produce viral bioagent identifying amplicons, or amplification products, of nucleic acids encoding viral genes. Compositions comprising pairs of primers and kits containing the same are designed to provide species and sub-species identification, detection and characterization of adventitious contaminant viruses. For example, compositions, primer pairs, kits and methods are disclosed herein to identify and For example, compositions, primer pairs, kits and methods are disclosed herein to identify and detect members of the Families Parvoviridae, Hepadnaviridae and Paramyxoviridae, and the sub-families, genera and species Parvovirinae, Dependovirus, Parvovirus, Erythrovirus, HBV, Pneumovirinae, Respirovirus, Avulavirus and Rubulavirus, and sub-species and strains thereof.

In some embodiments, methods for identification, detection or determination of the presence or absence of an adventitious contaminant virus in a sample are disclosed. In some embodiments, nucleic acid from the virus is amplified using the compounds disclosed herein to obtain an amplification product. The molecular mass of the amplification product is determined. The determined molecular mass is compared with a database comprising a plurality of indexed molecular masses of adventitious contaminant virus identifying amplicons, wherein a match between the determined molecular mass and a molecular masse in the database identifies or indicates the presence of the adventitious contaminant virus. In some embodiments, the molecular mass is measured by mass spectrometry. In some embodiments, the mass spectrometry is Fourier transform ion cyclotron resonance mass spectrometry (FT-IRC-MS). In some embodiments the mass spectrometry is time of flight mass spectrometry (TOF-MS). In some embodiments, at least one primer pair is used. In some embodiments, at least two primer pairs are used. In some embodiments at least four primer pairs are used. In some embodiments, the amplifying step is carried out by multiplex PCR.

In some embodiments the base composition of the amplification product is calculated from the determined molecular mass. The calculated base composition is compared with a database comprising a plurality of indexed base compositions of adventitious contaminant virus identifying amplicons, wherein a match between the calculated base composition and a base composition in the database identifies or indicates the presence of the the adventitious contaminant virus.

In some embodiments, the adventitious contaminant virus is a member of the Parvoviridae family, or a Parvoviridae virus. In some disclosures the member of the Parvoviridae family is a member of the Dependovirus genus. In some aspects, it is a member of the Erythrovirus genus. In some embodiments, it is a member of the Parvovirus genus. In some embodiments, the conserved sequence regions used in the methods for identifying Parvoviridae viruses comprise a portion of the gene encoding an NS I protein. In some embodiments, the conserved sequence regions used in the methods for identifying Parvoviridae viruses comprise a portion of the gene encoding a VP1 protein. Thus, in some cases the primers hybridize to a nucleic acid encoding an NS 1 protein, while in some cases they hybridize to a nucleic acid encoding a VP1 protein.

In some embodiments, the adventitious contaminant virus is a member of the Paramyxoviridae family, or a Paramyxoviridae virus. Disclosed herein, the member of the Paramyxoviridae family is a member of the Respirovirus genus. In some disclosures, it is a member of the Avulavirus genus. In some embodiments, it is a member of the Rubulavirus genus. In some embodiments, the member of the Paramyxoviridae family is a member of the Pneumovirinae sub-family. In some embodiments, the conserved sequence regions used in the methods for identifying Paramyxoviridae viruses comprise a portion of the gene encoding an RbRp protein. Thus, in some cases the primers hybridize to a nucleic acid encoding an RbRp protein.

In some embodiments, the adventitious contaminant virus is a member of the Hepadnaviridae family, or a Hepadnaviridae virus. Disclosed herein, the member of the Hepadnaviridae family is an HBV virus. In some embodiments, the conserved sequence regions used in the methods for identifying Hepadnaviridae viruses comprise a portion of nucleotides 180-453 of SEQ ID NO:338, a portion of nucleotides 1375-1436 of SEQ ID NO:338, or a portion of nucleotides 1777-1876 of SEQ ID NO: 338. Thus, in some embodiments, the primer pairs used for identifying Hepadnaviridae viruses hybridize to a portion of nucleotides 180-453 of SEQ ID NO:338, a portion of nucleotides 1375-1436 of SEQ ID NO:338, or a portion of nucleotides 1777-1876 of SEQ ID NO: 338. This sequence is presented as a reference sequence only. Since compounds disclosed herein hybridize to conserved sequence regions, they are not necessarily 100% complementary with target sequences and can often bind to multiple sequences, for example, in one embodiment, to all species within a genome.

In some embodiments, compounds, compositions, kits and methods for determination of the quantity of an adventitious contaminant virus in a sample are disclosed. The sample is contacted with a composition described herein and a known quantity of a calibration polynucleotide, or calibrant comprising a calibration sequence. Nucleic acid from the adventitious contaminant virus in the sample and nucleic acid from the calibration polynucleotide are concurrently amplified with the compounds disclosed hereinabove to obtain both a first amplification product comprising an adventitious contaminant virus identifying amplicon and a second amplification product comprising a calibration amplicon. The molecular mass and abundance for the adventitious contaminant virus identifying amplicon and the calibration amplicon is determined. The adventitious contaminant virus identifying amplicon is distinguished from the calibration amplicon based on molecular mass, wherein comparison of adventitious contaminant virus identifying amplicon abundance and calibration amplicon abundance indicates the quantity of adventitious contaminant virus in the sample. In some embodiments, the base composition of the adventitious contaminant virus identifying amplicon is determined.

In some embodiments, kits are disclosed herein for use in the methods disclosed herein, and using compounds disclosed herein. Disclosed herein, the kits comprise at least one calibration polynucleotide. In some disclosures, they comprise at least one ion exchange resin linked to magnetic beads. In some disclosures, the kits can be used in multiplex reaction, and the primer pairs disclosed herein are designed to be used in multiplex PCR reaction.

In some embodiments, the compounds and compositions disclosed comprise at least one modified nucleobase. In some disclosures the modified nucleobase comprises a mass-modified nucleobase or a universal nucleobase. In some disclosures, it is a 5-iodo-C. In some disclosures it is a 5-propynyluracil or 5-propynylcytosine. In some disclosures, it is inosine. In some embodiments, the primers comprise non-templated T residues on the 5' ends. In some embodiments, they comprise at least one non-template tag. In some embodiments, they comprise at least one molecular mass modifying tag.

In some embodiments, the compounds, compositions or kits comprise primer pairs having at least 70%, at least 80%, at least 90%, at least 95%, or at least 100% sequence identity with the primer pairs disclosed herein.

### BRIEF DESCRIPTION OF THE DRAWINGS

The foregoing summary of the invention, as well as the following detailed description of the invention, is better understood when read in conjunction with the accompanying drawings which are included by way of example and not by way of limitation.

**Figure 1****:** process diagram illustrating a representative primer pair selection process.

**Figure 2****:** process diagram illustrating a representative primer pair validation process.

**Figure 3****:** mass spectra of amplification products of HTLV-1 and HTLV-2 obtained by amplification of nucleic acid of HTLV-1 and HTLV-2 with primer pair numbers 2293 and 2294.

**Figure 4****:** 3D base composition plots of amplification products of human and simian retroviruses HTLV and STLV.

**Figure 5****:** 3D base composition plot of amplification products of SV40 virus, BK virus, and JC virus.

**Figure 6****:** 3D base composition plot of amplification products of polyomaviruses.

**Figure 7****:** 3D base composition plot of amplification products of papillomaviruses

**Figure 8****:** Determination of the dynamic range of detection of two viruses: cell line derived HPV-18 and plasmid-derived HPV-6b.

**Figure 9** is a process diagram illustrating an embodiment of the calibration method.

**Figure 10** shows testing of primer sensitivities against synthetic DNA calibrant. A subset of primers from Table 4 were used in limiting dilution studies (two-fold dilutions of calibrant construct, beginning with 5,000 copies per well.

**Figure 11****:** a. highly conserved regions of HBV nucleic acid sequence (SEQ ID NO: 338) to which primers were designed. b. Mass spectra and calculated base compositions in experimental validation of primer pairs 1245 and 1247. Each pair produced unique, expected amplification product. 1247 did not detect other, non-HBV, non Hepadnoviridae viruses included in reaction.

**Figure 12****:** 3D base composition plot showing distribution of all known respirovirus isolate in the A G C T space.

**Figure 13****: a.** 3D base composition plot showing distribution of Pneumonovirinae sub-family. b. RSV-B detection with primer pairs 2441 (top) and 2448 (bottom) using three different ATCC culture isolates (VR955, 1400 and 1401), with products matching the expected base composition for RSV-B detection.

### DETAILED DESCRIPTION OF EMBODIMENTS

In the context of the present invention, a "bioagent" is any organism, cell, or virus, living or dead, or a nucleic acid derived from such an organism, cell or virus. Examples of bioagents include, but are not limited, to cells, including but not limited to human clinical samples, cell cultures, bacterial cells and other pathogens, viruses, viroids, fungi, protists, parasites, and pathogenicity markers (including, but not limited to: pathogenicity islands, antibiotic resistance genes, virulence factors, toxin genes and other bioregulating compounds). Samples may be alive or dead or in a vegetative state (for example, vegetative bacteria or spores) and may be encapsulated or bioengineered. In the context of this invention, a "pathogen" is a bioagent which causes a disease or disorder.

As used herein, "intelligent primers" "primers" or "oligonucletide primers" are oligonucleotides that are designed to bind to highly conserved sequence regions of bioagent nucleic acid that flank an intervening variable region. The terms "Oligonucleotide primer pair" or "primer pair" refers to two primers (a forward and a reverse primer) that, upon amplification, yield an amplification product, or bioagent identifying amplicon.

In some embodiments, bioagent identifying amplicons or amplification products comprise from about 45 to about 200 nucleobases (i.e. from about 45 to about 200 linked nucleosides). One of ordinary skill in the art will appreciate that the invention embodies compounds of 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, and 200 nucleobases in length, or any range therewithin.

The amplification products or amplicons ideally provide enough base composition variability to distinguish individual bioagents, and which are amenable to molecular mass analysis. The variability of base composition and molecular mass allows for the identification of one or more individual bioagents based on base composition and/or molecular mass distinction. Primer pairs provided herein are named according to a certain nomenclature. Each primer pair is given a "Primer pair number," and a "primer pair name." Each primer (forward and reverse) within a primer pair is also given a "primer name." Each primer within a primer pair is also assigned a SEQ ID NO, and in some instances herein, the pair of SEQ ID NOs is used to identify a primer pair. For example, "the primer pair represented by SEQ ID NOs: 70:275" or simply "SEQ ID NOs: 70:239" refer to the primer pair, wherein the forward primer comprises SEQ ID NO: 70 and the reverse primer comprises SEQ ID NO: 239.

The "primer names" and "primer pair names" disclosed herein are given according to a standard nomenclature and include the naming of a reference sequence for each primer pair. For example, the forward primer number for primer pair number 377 is RLV_X03614_2256_2279_F. The "_F" indicates that this primer is the Forward primer of the pair. X03614 refers to the reference sequence, an example of a sequence to which the primer hybridizes, or is targeted. In this case, the reference sequence is GenBank Acession number X03614. The numbers following the reference sequence identifier identify the residues in the reference sequence to which the primer hybridizes (2256-2279). "RLV" is the primer pair identifier, and indicates that this primer hybridizes to a respirovirus. Since the primers provided herein hybridize to conserved sequence regions, and thus hybridize to multiple bioagents, often one or more species or strains, the primer pair identifier and reference sequence identifier are only for reference, and are not intended to indicate that the primer hybridizes to only the reference sequence. Table 2 lists the name and description of primer pair virus identifiers used herein.

By the term "highly conserved," it is meant that the sequence regions exhibit between about 80-100%, between about 90-100%, or between about 95-100% identity among all or at least 70%, at least 80%, at least 90%, at least 95%, or at least 99% of genera, species or strains. Further, the sequences of the primer members of the primer pairs provided herein are not necessarily fully complementary to the conserved sequence region of the reference sequence. Rather, the primers are designed to be "best fit" amongst a plurality of bioagents comprising these conserved sequence regions. Therefore, the primer pair members have substantial complementarity with the conserved regions of bioagent sequences, including the reference sequence.

As used herein, the term "substantial complementarity" refers to between about 70% and about 100%, between about 80% and about 100%, between about 90% and about 100%, between about 95% and about 100%, or between about 99% and about 100% complementarity to a target or reference sequence. These ranges of complementarity are inclusive of all whole or partial numbers embraced within the recited range numbers. For example, and not limitation, 75.667%, 82%, 91.2435%, and 97% all fall within the above recited range of about 70% and about 100%, therefore forming part of this description. Thus primers disclosed herein can comprise between about 70% and about 100%, between about 80% and about 100%, between about 90% and about 100%, between about 95% and about 100%, or between about 99% and about 100% sequence identity with the primer sequences disclosed herein.

As used herein, "housekeeping gene" refers to a gene encoding a protein or RNA involved in basic functions required for survival and reproduction of a bioagent. Housekeeping genes include, but are not limited to genes encoding RNA or proteins involved in translation, replication, recombination and repair, transcription, nucleotide metabolism, amino acid metabolism, lipid metabolism, energy generation, uptake, secretion and the like.

As used herein, "broad range survey primers" are intelligent primers designed to identify an unknown bioagent as a member of a particular division (e.g., an order, family, class, clade, genus or other such grouping of bioagents above the species level of bioagents). In some cases, broad range survey primers are able to identify unknown bioagents at the species or sub-species level. As used herein, "division-wide primers" are intelligent primers designed to identify a bioagent at the species level and "drill-down" primers are intelligent primers designed to identify a bioagent at the sub-species level. As used herein, the "sub-species" level of identification includes, but is not limited to, strains, subtypes, variants, and isolates.

As used herein, a "bioagent division" is defined as group of bioagents above the species level and includes but is not limited to, orders, families, classes, clades, genera or other such groupings of bioagents above the species level.

As used herein, a "sub-species characteristic" is a genetic characteristic that provides the means to distinguish two members of the same bioagent species. For example, one viral strain could be distinguished from another viral strain of the same species by possessing a genetic change (e.g., for example, a nucleotide deletion, addition or substitution) in one of the viral genes, such as the RNA-dependent RNA polymerase.

As used herein, the term "bioagent identifying amplicon" refers to a polynucleotide that is amplified from a bioagent in an amplification reaction and which 1) provides enough variability to distinguish each individual bioagent and 2) whose molecular mass is amenable to base composition calculation.

As used herein, a "base composition" is the exact number of each nucleobase (A, T, C and G) in a given nucleic acid sequence. Base compositions can be calculated from molecular masses of bioagent identifying amplicons.

As used herein, a "base composition probability cloud" is a representation of the diversity in base composition resulting from a variation in sequence that occurs among different isolates of a given species. The "base composition probability cloud" represents the base composition constraints for each species and is typically visualized using a pseudo four-dimensional plot.

As used herein, the term "database" is used to refer to a collection of molecular mass and/or base composition data. The base composition and molecular mass data in the database is indexed to bioagents and to primer pairs. The base composition data reported in the database comprises the number of each nucleoside in an amplicon that would be generated for each bioagent using each primer. The database can be populated by empirical data. In this aspect of populating the database, a bioagent is selected and a primer pair is used to generate an amplicon. The amplicon's molecular mass is determined using a mass spectrometer and the base composition calculated therefrom. An entry in the database is made to associate the base composition with the bioagent and the primer pair used. The database may also be populated using other databases comprising bioagent information. For example, using the GenBank database it is possible to perform electronic PCR using an electronic representation of a primer pair. This *in silico* method will provide the base composition for any or all selected bioagent(s) stored in the GenBank database. The information is then used to populate the base composition database as described above. A base composition database can be *in silico,* a written table, a reference book, a spreadsheet or any form generally amenable to databases. Preferably, it is *in silico.*

As used herein, a "wobble base" is a variation in a codon found at the third nucleotide position of a DNA triplet. Variations in conserved regions of sequence are often found at the third nucleotide position due to redundancy in the amino acid code.

In the context of the present disclosure, the term "unknown bioagent" may mean either: (i) a bioagent whose existence is known (such as the well known bacterial species *Staphylococcus aureus* for example) but which is not known to be in a sample to be analyzed, or (ii) a bioagent whose existence is not known (for example, the SARS coronavirus was unknown prior to April 2003). For example, if the method for identification of coronaviruses disclosed in commonly owned U.S. Patent Serial No. 10/829,826 was to be employed prior to April 2003 to identify the SARS coronavirus in a clinical sample, both meanings of "unknown" bioagent are applicable since the SARS coronavirus was unknown to science prior to April, 2003 and since it was not known what bioagent (in this case a coronavirus) was present in the sample. On the other hand, if the method of U.S. Patent Serial No. 10/829,826 was to be employed subsequent to April 2003 to identify the SARS coronavirus in a clinical sample, only the first meaning (i) of "unknown" bioagent would apply since the SARS coronavirus became known to science subsequent to April 2003 and since it was not known what bioagent was present in the sample.

As used herein, "triangulation identification" means the employment of more than one bioagent identifying amplicons for identification of a bioagent.

In the context of the present disclosure, "viral nucleic acid" includes, but is not limited to, DNA, RNA, or DNA that has been obtained from viral RNA, such as, for example, by performing a reverse transcription reaction. Viral RNA can either be single-stranded (of positive or negative polarity) or double-stranded.

As used herein, the term "etiology" refers to the causes or origins, of diseases or abnormal physiological conditions.

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

The present disclosure provides methods for detection and identification of bioagents in an unbiased manner using bioagent identifying amplicons. Primers are selected to hybridize to conserved sequence regions of nucleic acids derived from a bioagent and which bracket variable sequence regions to yield a bioagent identifying amplicon which can be amplified and which is amenable to molecular mass determination. The molecular mass then provides a means to uniquely identify the bioagent without a requirement for prior knowledge of the possible identity of the bioagent. The molecular mass or corresponding base composition signature of the amplification product is then matched against a database of molecular masses or base composition signatures. Furthermore, the method can be applied to rapid parallel multiplex analyses, the results of which can be employed in a triangulation identification strategy. The present method provides rapid throughput and does not require nucleic acid sequencing of the amplified target sequence for bioagent detection and identification.

Despite enormous biological diversity, all forms of life on earth share sets of essential, common features in their genomes. Since genetic data provide the underlying basis for identification of bioagents by the methods of the present disclosure, it is necessary to select segments of nucleic acids which ideally provide enough variability to distinguish each individual bioagent and whose molecular mass is amenable to molecular mass determination.

Unlike bacterial genomes, which exhibit conversation of numerous genes (i.e. housekeeping genes) across all organisms, viruses do not share a gene that is essential and conserved among all virus families. Therefore, viral identification is achieved within smaller groups of related viruses, such as members of a particular virus family or genus. For example, RNA-dependent RNA polymerase is present in all single-stranded RNA viruses and can be used for broad priming as well as resolution within the virus family.

In some embodiments, at least one viral nucleic acid segment is amplified in the process of identifying the bioagent. Thus, the nucleic acid segments that can be amplified by the primers disclosed herein and that provide enough variability to distinguish each individual bioagent and whose molecular masses are amenable to molecular mass determination are herein described as bioagent identifying amplicons.

It is the combination of the portions of the bioagent nucleic acid segment to which the primers hybridize (hybridization sites) and the variable region between the primer hybridization sites that comprises the bioagent identifying amplicon.

In some embodiments, bioagent identifying amplicons amenable to molecular mass determination which are produced by the primers described herein are either of a length, size or mass compatible with the particular mode of molecular mass determination or compatible with a means of providing a predictable fragmentation pattern in order to obtain predictable fragments of a length compatible with the particular mode of molecular mass determination. Such means of providing a predictable fragmentation pattern of an amplification product include, but are not limited to, cleavage with restriction enzymes or cleavage primers, for example. Thus, in some embodiments, bioagent identifying amplicons, or amplification products are larger than 200 nucleobases and are amenable to molecular mass determination following restriction digestion. Methods of using restriction enzymes and cleavage primers are well known to those with ordinary skill in the art.

In some embodiments, amplification products corresponding to bioagent identifying amplicons are obtained using the polymerase chain reaction (PCR) which is a routine method to those with ordinary skill in the molecular biology arts. Other amplification methods may be used such as ligase chain reaction (LCR), low-stringency single primer PCR, and multiple strand displacement amplification (MDA). These methods are also known to those with ordinary skill.

The primers are designed to bind to highly conserved sequence regions of a bioagent identifying amplicon that flank an intervening variable region and yield amplification products which ideally provide enough variability to distinguish each individual bioagent, and which are amenable to molecular mass analysis. In some embodiments, the highly conserved sequence regions exhibit between about 80-100%, or between about 90-100%, or between about 95-100% identity, or between about 99-100% identity among species, sub-species, strains or genera. The molecular mass of a given amplification product provides a means of identifying the bioagent from which it was obtained, due to the variability of the variable region. Thus design of the primers requires selection of a variable region with appropriate variability to resolve the identity of a given bioagent. Bioagent identifying amplicons are ideally specific to the identity of the bioagent.

Identification ofbioagents can be accomplished at different levels using primers suited to resolution of each individual level of identification. Broad range survey primers are designed with the objective of identifying a bioagent as a member of a particular division (e.g., an order, family, genus or other such grouping of bioagents above the species level of bioagents). In some embodiments, broad range survey intelligent primers are capable of identification of bioagents at the species or sub-species level.

Drill-down primers are designed with the objective of identifying a bioagent at the sub-species level (including strains, subtypes, variants and isolates) based on sub-species characteristics. Drill-down intelligent primers are not always required for identification at the sub-species level because broad range survey intelligent primers may, in some cases provide sufficient identification resolution to accomplishing this identification objective.

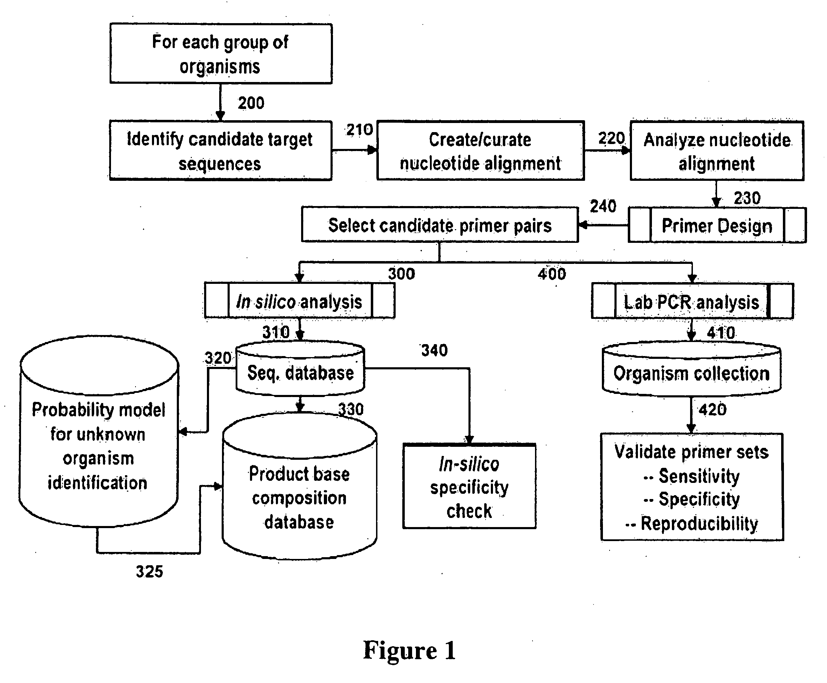

A representative process flow diagram used for primer selection and validation process is outlined in Figure 1. For each group of organisms, candidate target sequences are identified (200) from which nucleotide alignments are created (210) and analyzed (220). Primers are then designed by selecting appropriate priming regions (230) to facilitate the selection of candidate primer pairs (240). The primer pairs are then subjected to *in silico* analysis by electronic PCR (ePCR) (300) wherein bioagent identifying amplicons are obtained from sequence databases such as GenBank or other sequence collections (310) and checked for specificity *in silico* (320). Bioagent identifying amplicons obtained from GenBank sequences (310) can also be analyzed by a probability model which predicts the capability of a given amplicon to identify unknown bioagents such that the base compositions of amplicons with favorable probability scores are then stored in a base composition database (325). Alternatively, base compositions of the bioagent identifying amplicons obtained from the primers and GenBank sequences can be directly entered into the base composition database (330). Candidate primer pairs (240) are validated by in vitro amplification by a method such as PCR analysis (400) of nucleic acid from a collection of organisms (410). Amplification products thus obtained are analyzed to confirm the sensitivity, specificity and reproducibility of the primers used to obtain the amplification products (420).

Many of the important pathogens, including the organisms of greatest concern as biological weapons agents, have been completely sequenced. This effort has greatly facilitated the design of primers and probes for the detection of unknown bioagents. The combination of broad-range priming with division-wide and drill-down priming has been used very successfully in several applications of the technology, including environmental surveillance for biowarfare threat agents and clinical sample analysis for medically important pathogens.

Synthesis of primers is well known and routine in the art. The primers may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed.

The primers are employed as compositions for use in methods for identification of viral bioagents as follows: a primer pair composition is contacted with nucleic acid (such as, for example, DNA from a DNA virus, or DNA reverse transcribed from the RNA of an RNA virus) of an unknown viral bioagent. The nucleic acid is then amplified by a nucleic acid amplification technique, such as PCR for example, to obtain an amplification product that represents a bioagent identifying amplicon. The molecular mass of each strand of the double-stranded amplification product is determined by a molecular mass measurement technique such as mass spectrometry for example, wherein the two strands of the double-stranded amplification product are separated during the ionization process. In some embodiments, the mass spectrometry is electrospray Fourier transform ion cyclotron resonance mass spectrometry (ESI-FTICR-MS) or electrospray time of flight mass spectrometry (ESI-TOF-MS). A list of possible base compositions can be generated for the molecular mass value obtained for each strand and the choice of the correct base composition from the list is facilitated by matching the base composition of one strand with a complementary base composition of the other strand. The molecular mass or base composition thus determined is then compared with a database comprising indexed molecular masses or indexed base compositions of analogous bioagent identifying amplicons for known viral bioagents. A match between the molecular mass or base composition of the amplification product and the molecular mass or base composition of an analogous bioagent identifying amplicon for a known viral bioagent indicates the identity of the unknown bioagent. In some embodiments, the primer pair used is one of the primer pairs of Table 2. In some embodiments, the method is repeated using a different primer pair to resolve possible ambiguities in the identification process or to improve the confidence level for the identification assignment.

In some embodiments, a bioagent identifying amplicon may be produced using only a single primer (either the forward or reverse primer of any given primer pair), provided an appropriate amplification method is chosen, such as, for example, low stringency single primer PCR (LSSP-PCR). Adaptation of this amplification method in order to produce bioagent identifying amplicons can be accomplished by one with ordinary skill in the art without undue experimentation.

In some cases, the molecular mass or base composition of a bioagent identifying amplicon defined by a broad range survey primer pair does not provide enough resolution to unambiguously identify a bioagent at or below the species level. These cases benefit from further analysis of one or more bioagent identifying amplicons generated from at least one additional broad range survey primer pair or from at least one additional division-wide primer pair. The employment of more than one bioagent identifying amplicon for identification of a bioagent is herein referred to as triangulation identification.

In other embodiments, the oligonucleotide primers are division-wide primers which hybridize to nucleic acid encoding genes of species within a genus. In other embodiments, the oligonucleotide primers are drill-down primers which enable the identification of sub-species characteristics. Drill down primers provide the functionality of producing bioagent identifying amplicons for drill-down analyses such as strain typing when contacted with nucleic acid under amplification conditions. Identification of such sub-species characteristics is often critical for determining proper clinical treatment of infections. In some embodiments, sub-species characteristics are identified using only broad range survey primers and division-wide and drill-down primers are not used.

In some embodiments, the primers used for amplification hybridize to and amplify genomic DNA, DNA of bacterial plasmids, DNA of DNA viruses or DNA reverse transcribed from RNA of an RNA virus.

In some embodiments, the primers used for amplification hybridize directly to viral RNA and act as reverse transcription primers for obtaining DNA from direct amplification of viral RNA. Methods of amplifying RNA to produce cDNA using reverse transcriptase are well known to those with ordinary skill in the art and can be routinely established without undue experimentation.

Primers described herein are targeted to particular target nucleic acids. One with ordinary skill in the art of design of amplification primers will recognize that a given primer need not be 100% complementary to a target nucleic acid in order to hybridize to the nucleic acid and effectively prime the synthesis of a complementary nucleic acid strand in an amplification reaction. Moreover, a primer may hybridize over one or more segments such that intervening or adjacent segments are not involved in the hybridization event. (e.g., for example, a loop structure or a hairpin structure). The primers described herein may comprise at least 70%, at least 75%, at least 80%, at least 85%, at least 90%, at least 95% or at least 99% sequence identity with any of the primers listed in Table 2, and may be at least 70%, at least 75%, at least 80%, at least 85%, at least 90%, at least 95% or at least 99% complementary to a target nucleic acid sequence. Thus, in some embodiments, an extent of variation of 70% to 100%, or any percentage therewithin, of the sequence identity is possible relative to the specific primer sequences disclosed herein. Determination of sequence identity is described in the following example: a primer 20 nucleobases in length which shares sequence identity with another 20 nucleobase primer, but having two non-identical residues has 18 residues identical to the other primer, out of 20 total residues (18/20 = 0.9), and thus has 90% sequence identity. In another example, a primer 15 nucleobases in length having all residues identical to a 15 nucleobase segment of a primer 17 nucleobases in length would have 15/17 = 0.88235 or 88.235% sequence identity with the 17 nucleobase primer. Percent complementarity is determined in a similar way. For example, a 20 nucleobase primer hybridizable to a 20 nucleobase segment of a target nucleic acid, with only 18 out of 20 complementary residues has 18/20 or 0.9 complementarity, and is 90% complementary with the target nucleic acid sequence.

Percent homology, sequence identity or complementarity, can be determined by, for example, the Gap program (Wisconsin Sequence Analysis Package, Version 8 for UNIX, Genetics Computer Group, University Research Park, Madison WI), using default settings, which uses the algorithm of Smith and Waterman (Adv. Appl. Math., 1981, 2, 482-489). In some embodiments, complementarity of primers with respect to the conserved priming regions of viral nucleic acid is between about 70% and about 80%. In other embodiments, homology, sequence identity or complementarity, is between about 80% and about 90%. In yet other embodiments, homology, sequence identity or complementarity, is at least 90%, at least 92%, at least 94%, at least 95%, at least 96%, at least 97%, at least 98%, at least 99% or is 100%.

In some embodiments, the primers described herein comprise at least 70%, at least 75%, at least 80%, at least 85%, at least 90%, at least 92%, at least 94%, at least 95%, at least 96%, at least 98%, or at least 99%, or 100% (or any range therewithin) sequence identity with the primer sequences specifically disclosed herein.

One with ordinary skill is able to calculate percent sequence identity or percent sequence homology and able to determine, without undue experimentation, the effects of variation of primer sequence identity on the function of the primer in its role in priming synthesis of a complementary strand of nucleic acid for production of an amplification product of a corresponding bioagent identifying amplicon.

In some embodiments of the present disclosure, the oligonucleotide primers are 13 to 35 nucleobases in length (13 to 35 linked nucleotide residues). These embodiments comprise oligonucleotide primers 13, 14, 15, 16, 17, 18, 19, 20, 21, 22, 23, 24, 25, 26, 27, 28, 29, 30, 31, 32, 33, 34 or 35 nucleobases in length, or any range therewithin.

In some embodiments, any given primer comprises a modification comprising the addition of a non-templated T residue to the 5' end of the primer (i.e., the added T residue does not necessarily hybridize to the nucleic acid being amplified). The addition of a non-templated T residue has an effect of minimizing the addition of non-templated A residues as a result of the non-specific enzyme activity of Taq polymerase (Magnuson et al., Biotechniques, 1996, 21, 700-709), an occurrence which may lead to ambiguous results arising from molecular mass analysis.

In some embodiments of the present disclosure, primers may contain one or more universal bases. Because any variation (due to codon wobble in the 3^{rd} position) in the conserved regions among species is likely to occur in the third position of a DNA (or RNA) triplet, oligonucleotide primers can be designed such that the nucleotide corresponding to this position is a base which can bind to more than one nucleotide, referred to herein as a "universal nucleobase." For example, under this "wobble" pairing, inosine (I) binds to U, C or A; guanine (G) binds to U or C, and uridine (U) binds to U or C. Other examples of universal nucleobases include nitroindoles such as 5-nitroindole or 3-nitropyrrole (Loakes et al., Nucleosides and Nucleotides, 1995, 14, 1001-1003), the degenerate nucleotides dP or dK (Hill *et al*.), an acyclic nucleoside analog containing 5-nitroindazole (Van Aerschot et al., Nucleosides and Nucleotides, 1995, 14, 1053-1056) or the purine analog 1-(2-deoxy-β-D-ribofuranosyl)-imidazole-4-carboxamide (Sala et al., Nucl. Acids Res., 1996, 24, 3302-3306).

In some embodiments, to compensate for the somewhat weaker binding by the wobble base, the oligonucleotide primers are designed such that the first and second positions of each triplet are occupied by nucleotide analogs which bind with greater affinity than the unmodified nucleotide. Examples of these analogs include, but are not limited to, 2,6-diaminopurine which binds to thymine, 5-propynyluracil which binds to adenine and 5-propynylcytosine and phenoxazines, including G-clamp, which binds to G. Propynylated pyrimidines are described in U.S. Patent Nos. 5,645,985, 5,830,653 and 5,484,908, Propynylated primers are described in U.S Pre-Grant Publication No. 2003-0170682, which is also commonly owned. . Phenoxazines are described in U.S. Patent Nos. 5,502,177, 5,763,588, and 6,005,096. G-clamps are described in U.S. Patent Nos. 6,007,992 and 6,028,183.

In some embodiments, to enable broad priming of rapidly evolving RNA viruses, primer hybridization is enhanced using primers and probes containing 5-propynyl deoxy-cytidine and deoxy-thymidine nucleotides. These modified primers and probes offer increased affinity and base pairing selectivity.

In some embodiment, non-template primer tags are used to increase the melting temperature (Tₘ) of a primer-template duplex in order to improve amplification efficiency. A non-template tag is at least three consecutive A or T nucleotide residues on a primer which are not complementary to the template. In any given non-template tag, A can be replaced by C or G and T can also be replaced by C or G. Although Watson-Crick hybridization is not expected to occur for a non-template tag relative to the template, the extra hydrogen bond in a G-C pair relative to an A-T pair confers increased stability of the primer-template duplex and improves amplification efficiency for subsequent cycles of amplification when the primers hybridize to strands synthesized in previous cycles.

In other embodiments, propynylated tags may be used in a manner similar to that of the non-template tag, wherein two or more 5-propynylcytidine or 5-propynyluridine residues replace template matching residues on a primer. In other embodiments, a primer contains a modified internucleoside linkage such as a phosphorothioate linkage, for example.

In some embodiments, the primers contain mass-modifying tags. Reducing the total number of possible base compositions of a nucleic acid of specific molecular weight provides a means of avoiding a persistent source of ambiguity in determination of base composition of amplification products. Addition of mass-modifying tags to certain nucleobases of a given primer will result in simplification of *de novo* determination of base composition of a given bioagent identifying amplicons from its molecular mass.

In some embodiments of the present invention, the mass modified nucleobase comprises one or more of the following: for example, 7-deaza-2'-deoxyadenosine-5-triphosphate, 5-iodo-2'-deoxyuridine-5'-triphosphate, 5-bromo-2'-deoxyuridine-5'-triphosphate, 5-bromo-2'-deoxycytidine-5'-triphosphate, 5-iodo-2'-deoxycytidine-5'-triphosphate, 5-hydroxy-2'-deoxyuridine-5'-triphosphate, 4-thiothymidine-5'-triphosphate, 5-aza-2'-deoxyuridine-5'-triphosphate, 5-fluoro-2'-deoxyuridine-5'-triphosphate, O6-methyl-2'-deoxyguanosine-5'-triphosphate, N2-methyl-2'-deoxyguanosine-5'-triphosphate, 8-oxo-2'-deoxyguanosine-5'-triphosphate or thiothymidine-5'-triphosphate. In some embodiments, the mass-modified nucleobase comprises ¹⁵N or ¹³C or both ¹⁵N and ¹³C.

In some cases, a molecular mass of a given bioagent identifying amplicon alone does not provide enough resolution to unambiguously identify a given bioagent. The employment of more than one bioagent identifying amplicon for identification of a bioagent is herein referred to as triangulation identification. Triangulation identification is pursued by analyzing a plurality of bioagent identifying amplicons selected within multiple housekeeping genes. This process is used to reduce false negative and false positive signals, and enable reconstruction of the origin of hybrid or otherwise engineered bioagents. For example, identification of the three part toxin genes typical *of B. anthracis* (Bowen et al., J. Appl. Microbiol., 1999, 87, 270-278) in the absence of the expected signatures from the *B. anthracis* genome would suggest a genetic engineering event.

In some embodiments, the triangulation identification process can be pursued by characterization of bioagent identifying amplicons in a massively parallel fashion using the polymerase chain reaction (PCR), such as multiplex PCR where multiple primers are employed in the same amplification reaction mixture, or PCR in multi-well plate format wherein a different and unique pair of primers is used in multiple wells containing otherwise identical reaction mixtures. Such multiplex and multi-well PCR methods are well known to those with ordinary skill in the arts of rapid throughput amplification of nucleic acids. In other related embodiments, one PCR reaction per well or container may be carried out, followed by an amplicon pooling step wherein the amplification products of different wells are combined in a single well or container which is then subjected to molecular mass analysis. The combination of pooled amplicons can be chosen such that the expected ranges of molecular masses of individual amplicons are not overlapping and thus will not complicate identification of signals.

In some embodiments, the molecular mass of a given bioagent identifying amplicon is determined by mass spectrometry. Mass spectrometry has several advantages, not the least of which is high bandwidth characterized by the ability to separate (and isolate) many molecular peaks across a broad range of mass to charge ratio (m/z). Thus mass spectrometry is intrinsically a parallel detection scheme without the need for radioactive or fluorescent labels, since every amplification product is identified by its molecular mass. The current state of the art in mass spectrometry is such that less than femtomole quantities of material can be readily analyzed to afford information about the molecular contents of the sample. An accurate assessment of the molecular mass of the material can be quickly obtained, irrespective of whether the molecular weight of the sample is several hundred, or in excess of one hundred thousand atomic mass units (amu) or Daltons.

In some embodiments, intact molecular ions are generated from amplification products using one of a variety of ionization techniques to convert the sample to gas phase. These ionization methods include, but are not limited to, electrospray ionization (ES), matrix-assisted laser desorption ionization (MALDI) and fast atom bombardment (FAB). Upon ionization, several peaks are observed from one sample due to the formation of ions with different charges. Averaging the multiple readings of molecular mass obtained from a single mass spectrum affords an estimate of molecular mass of the bioagent identifying amplicon. Electrospray ionization mass spectrometry (ESI-MS) is particularly useful for very high molecular weight polymers such as proteins and nucleic acids having molecular weights greater than 10 kDa, since it yields a distribution of multiply-charged molecules of the sample without causing a significant amount of fragmentation.

The mass detectors used in the methods of the present invention include, but are not limited to, Fourier transform ion cyclotron resonance mass spectrometry (FT-ICR-MS), time of flight (TOF), ion trap, quadrupole, magnetic sector, Q-TOF, and triple quadrupole.

Although the molecular mass of amplification products obtained using intelligent primers provides a means for identification of bioagents, conversion of molecular mass data to a base composition signature is useful for certain analyses. As used herein, "base composition" is the exact number of each nucleobase (A, T, C and G) determined from the molecular mass of a bioagent identifying amplicon. In one embodiment, a base composition provides an index of a specific organism.

In some embodiments, assignment of previously unobserved base compositions to a given phylogeny can be accomplished via the use of pattern classifier model algorithms. Base compositions, like sequences, vary slightly from strain to strain within species, for example. In some embodiments, the pattern classifier model is the mutational probability model. On other embodiments, the pattern classifier is the polytope model. The mutational probability model and polytope model are both commonly owned and described in U.S. Patent application Serial No. 11/073,362.

In one embodiment, it is possible to manage this diversity by building "base composition probability clouds" around the composition constraints for each species. This permits identification of organisms in a fashion similar to sequence analysis. A "pseudo four-dimensional plot" can be used to visualize the concept of base composition probability clouds. Optimal primer design requires optimal choice of bioagent identifying amplicons and maximizes the separation between the base composition signatures of individual bioagents. Areas where clouds overlap indicate regions that may result in a misclassification, a problem which is overcome by a triangulation identification process using bioagent identifying amplicons not affected by overlap of base composition probability clouds.

In some embodiments, base composition probability clouds provide the means for screening potential primer pairs in order to avoid potential misclassifications of base compositions. In other embodiments, base composition probability clouds provide the means for predicting the identity of a bioagent whose assigned base composition was not previously observed and/or indexed in a bioagent identifying amplicon base composition database due to evolutionary transitions in its nucleic acid sequence. Thus, in contrast to probe-based techniques, mass spectrometry determination of base composition does not require prior knowledge of the composition or sequence in order to make the measurement.

The present invention provides bioagent classifying information similar to DNA sequencing and phylogenetic analysis at a level sufficient to identify a given bioagent. Furthermore, the process of determination of a previously unknown base composition for a given bioagent (for example, in a case where sequence information is unavailable) has downstream utility by providing additional bioagent indexing information with which to populate base composition databases. The process of future bioagent identification is thus greatly improved as more BCS indexes become available in base composition databases.

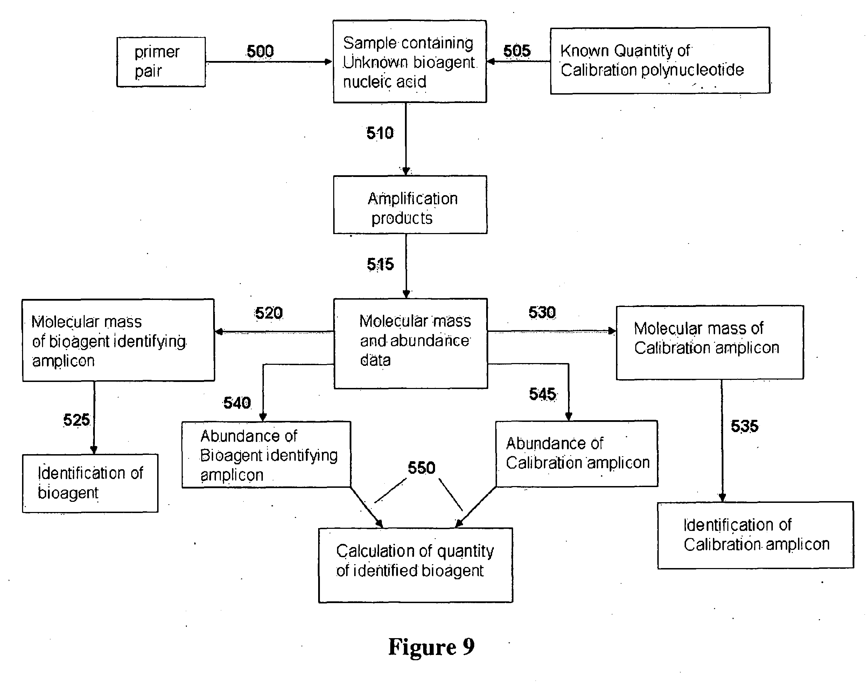

In some embodiments, the identity and quantity of an unknown bioagent can be determined using the process illustrated in Figure 9. Primers (**500**) and a known quantity of a calibration polynucleotide (**505**) are added to a sample containing nucleic acid of an unknown bioagent. The total nucleic acid in the sample is then subjected to an amplification reaction (**510**) to obtain amplification products. The molecular masses of amplification products are determined (**515**) from which are obtained molecular mass and abundance data. The molecular mass of the bioagent identifying amplicon (**520**) provides the means for its identification (**525**) and the molecular mass of the calibration amplicon obtained from the calibration polynucleotide (**530**) provides the means for its identification (**535**). The abundance data of the bioagent identifying amplicon is recorded (**540**) and the abundance data for the calibration data is recorded (**545**), both of which are used in a calculation (**550**) which determines the quantity of unknown bioagent in the sample.

A sample comprising an unknown bioagent is contacted with a pair of primers which provide the means for amplification of nucleic acid from the bioagent, and a known quantity of a polynucleotide that comprises a calibration sequence. The nucleic acids of the bioagent and of the calibration sequence are amplified and the rate of amplification is reasonably assumed to be similar for the nucleic acid of the bioagent and of the calibration sequence. The amplification reaction then produces two amplification products: a bioagent identifying amplicon and a calibration amplicon. The bioagent identifying amplicon and the calibration amplicon should be distinguishable by molecular mass while being amplified at essentially the same rate. Effecting differential molecular masses can be accomplished by choosing as a calibration sequence, a representative bioagent identifying amplicon (from a specific species of bioagent) and performing, for example, a 2-8 nucleobase deletion or insertion within the variable region between the two priming sites. The amplified sample containing the bioagent identifying amplicon and the calibration amplicon is then subjected to molecular mass analysis by mass spectrometry, for example. The resulting molecular mass analysis of the nucleic acid of the bioagent and of the calibration sequence provides molecular mass data and abundance data for the nucleic acid of the bioagent and of the calibration sequence. The molecular mass data obtained for the nucleic acid of the bioagent enables identification of the unknown bioagent and the abundance data enables calculation of the quantity of the bioagent, based on the knowledge of the quantity of calibration polynucleotide contacted with the sample.

In some embodiments, construction of a standard curve where the amount of calibration polynucleotide spiked into the sample is varied provides additional resolution and improved confidence for the determination of the quantity of bioagent in the sample. The use of standard curves for analytical determination of molecular quantities is well known to one with ordinary skill and can be performed without undue experimentation.

In some embodiments, multiplex amplification is performed where multiple bioagent identifying amplicons are amplified with multiple primer pairs which also amplify the corresponding standard calibration sequences. In this or other embodiments, the standard calibration sequences are optionally included within a single vector which functions as the calibration polynucleotide. Multiplex amplification methods are well known to those with ordinary skill and can be performed without undue experimentation.

In some embodiments, the calibrant polynucleotide is used as an internal positive control to confirm that amplification conditions and subsequent analysis steps are successful in producing a measurable amplicon. Even in the absence of copies of the genome of a bioagent, the calibration polynucleotide should give rise to a calibration amplicon. Failure to produce a measurable calibration amplicon indicates a failure of amplification or subsequent analysis step such as amplicon purification or molecular mass determination. Reaching a conclusion that such failures have occurred is in itself, a useful event.

In some embodiments, the calibration sequence is comprised of DNA. In some embodiments, the calibration sequence is comprised of RNA.

In some embodiments, the calibration sequence is inserted into a vector which then itself functions as the calibration polynucleotide. In some embodiments, more than one calibration sequence is inserted into the vector that functions as the calibration polynucleotide. Such a calibration polynucleotide is herein termed a "combination calibration polynucleotide." The process of inserting polynucleotides into vectors is routine to those skilled in the art and can be accomplished without undue experimentation. Thus, it should be recognized that the calibration method should not be limited to the embodiments described herein. The calibration method can be applied for determination of the quantity of any bioagent identifying amplicon when an appropriate standard calibrant polynucleotide sequence is designed and used. The process of choosing an appropriate vector for insertion of a calibrant is also a routine operation that can be accomplished by one with ordinary skill without undue experimentation.

In other embodiments of the present disclosure, the intelligent primers produce bioagent identifying amplicons within stable and highly conserved regions of adventitious contaminant viruses. The advantage to characterization of an amplicon in a highly conserved region is that there is a low probability that the region will evolve past the point of primer recognition, in which case, the amplification step would fail. Such a primer set is thus useful as a broad range survey-type primer. In another embodiment of the present disclosure, the intelligent primers produce bioagent identifying amplicons in a region which evolves more quickly than the stable region described above. The advantage of characterization bioagent identifying amplicon corresponding to an evolving genomic region is that it is useful for distinguishing emerging strain variants.

The present disclosure also has significant advantages as a platform for identification of diseases caused by emerging viruses. The present disclosure eliminates the need for prior knowledge of bioagent sequence to generate hybridization probes. Thus, in another embodiment, the present disclosure discloses a means of determining the etiology of a virus infection when the process of identification of viruses is carried out in a clinical setting and, even when the virus is a new species never observed before. This is possible because the methods are not confounded by naturally occurring evolutionary variations (a major concern for characterization of viruses which evolve rapidly) occurring in the sequence acting as the template for production of the bioagent identifying amplicon. Measurement of molecular mass and determination of base composition is accomplished in an unbiased manner without sequence prejudice.

Another embodiment of the present disclosure also provides a means of tracking the spread of any species or strain of virus when a plurality of samples obtained from different locations are analyzed by the methods described above in an epidemiological setting. In one embodiment, a plurality of samples from a plurality of different locations are analyzed with primers which produce bioagent identifying amplicons, a subset of which contain a specific virus. The corresponding locations of the members of the virus-containing subset indicate the spread of the specific virus to the corresponding locations.

The present invention also provides kits for carrying out the methods described herein. In some embodiments, the kit may comprise a sufficient quantity of one or more primer pairs to perform an amplification reaction on a target polynucleotide from a bioagent to form a bioagent identifying amplicon. In some embodiments, the kit may comprise from one to fifty primer pairs, from one to twenty primer pairs, from one to ten primer pairs, or from two to five primer pairs. In some embodiments, the kit may comprise one or more primer pairs recited in Table 2.

In some embodiments, the kit may comprise one or more broad range survey primer(s), division wide primer(s), or drill-down primer(s), or any combination thereof. A kit may be designed so as to comprise particular primer pairs for identification of a particular bioagent. A drill-down kit may be used, for example, to distinguish different sub-species types of adventitious contaminant viruses or genetically engineered adventitious contaminant viruses. In some embodiments, any of these kits may be combined to comprise a combination of broad range survey primers and division-wide primers so as to be able to identify the adventitious contaminant virus.

In some embodiments, the kit may contain standardized calibration polynucleotides for use as internal amplification calibrants.

In some embodiments, the kit may also comprise a sufficient quantity of reverse transcriptase (if an RNA virus is to be identified for example), a DNA polymerase, suitable nucleoside triphosphates (including any of those described above), a DNA ligase, and/or reaction buffer, or any combination thereof, for the amplification processes described above. A kit may further include instructions pertinent for the particular embodiment of the kit, such instructions describing the primer pairs and amplification conditions for operation of the method. A kit may also comprise amplification reaction containers such as microcentrifuge tubes and the like. A kit may also comprise reagents or other materials for isolating bioagent nucleic acid or bioagent identifying amplicons from amplification, including, for example, detergents, solvents, or ion exchange resins which may be linked to magnetic beads. A kit may also comprise a table of measured or calculated molecular masses and/or base compositions of bioagents using the primer pairs of the kit.

While the present invention has been described with specificity in accordance with certain of its embodiments, the following examples serve only to illustrate the invention and are not intended to limit the same. In order that the invention disclosed herein may be more efficiently understood, examples are provided below. It should be understood that these examples are for illustrative purposes only and are not to be construed as limiting the invention in any manner.

### EXAMPLES

### Example 1: Selection of Design and Validation of Primers that Define Bioagent Identifying Amplicons for Adventitious contaminant viruses

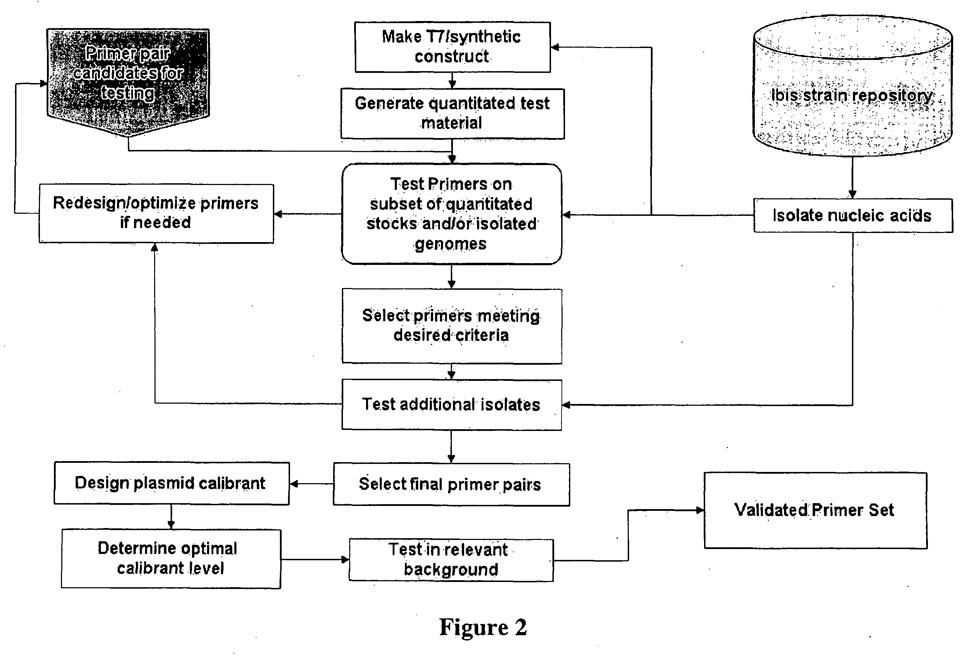

For design of primers that define adventitious contaminant virus identifying amplicons, a series of adventitious contaminant virus genome segment sequences were obtained, aligned and scanned for regions where pairs of PCR primers would amplify products of about 45 to about 150 nucleotides in length and distinguish species and/or individual strains from each other by their molecular masses or base compositions. A typical process shown in Figure 1 is employed for this type of analysis. Primer pair validation is carried out according to some or all of the steps shown in Figure 2.

A database of expected base compositions for each primer region was generated using an *in silico* PCR search algorithm, such as (ePCR). An existing RNA structure search algorithm (Macke et al., Nucl. Acids Res., 2001, 29, 4724-4735 has been modified to include PCR parameters such as hybridization conditions, mismatches, and thermodynamic calculations (SantaLucia, Proc. Natl. Acad. Sci. U.S.A., 1998, 95, 1460-1465. This also provides information on primer specificity of the selected primer pairs.

**Table 1: Primer Pairs for Identification of Adventitious Contaminant Viruses**

| **Primer Pair Number** | **Forward Primer Name** | **Forward Sequence** | **Forward SEQ ID NO:** | **Reverse Primer Name** | **Reverse Sequence** | **Reverse SEQ ID NO:** |
|---|---|---|---|---|---|---|
| 377 | RVL_X03614_22 56 2279 F | | 1 | RVL_X03614_23 02 2324 R | | 170 |
| 378 | RVL_X03614_22 39 2261 F | | 2 | RVL_X03614_23 02 2324 R | | 170 |
| 379 | PVL_U50363_31 76 3199 P | | 3 | PVL_U50363_32 72 3296 R | | 171 |
| 380 | **PVL_U50363_31** 53 3175 P | | 4 | PVL_U50363_32 65 3286 R | | 172 |
| 381 | MVL_AF266286_ 1625 1649 F | | 5 | MVL_AF266286_ 1720 1739 R | | 173 |
| 382 | MVL_AF266286_ 2033 2054 F | | 6 | MVL_AF266286_ 2122 2139 R | | 174 |
| 383 | MVL_AF266286_ 2002 2024 F | | 7 | MVL_AF266286_ 2035 2054 R | | 175 |
| 384 | MSVL_AF266286 3529 3548 P | | 8 | MSVL_AF266286 3587 3608 R | | 176 |
| 385 | PNVL_U50363_1 285 1301 P | | 9 | PNVL_U50363_1 318 1335 R | | 177 |
| 386 | PNVL_U50363_1 878 1898 F | | 10 | PNVL_U50363_1 927 1949 R | | 178 |
| 387 | PNVL_U50363_1 881 1898 F | | 11 | PNVL_U50363_1 927 1944 R | | 179 |
| 388 | PNVL_U50363_2 669 2687 F | | 12 | PNVL_U50363_2 743 2763 R | | 180 |
| 389 | MNVL_NC004148 24 47 F | | 13 | MNVL_NC004148 73 96 R | | 181 |
| 390 | MNVL_NC004148 30 47 F | | 14 | KNVL_NC004148 73 91 R | | 182 |
| 391 | MNVL_NC004148 1907 1931 F | | 15 | MNVL_NC004148 1984 2006 R | | 183 |
| 392 | MNVL_NC004148 1917 1931 P | | 16 | MNVL_NC004148 1984 1999 R | | 184 |
| 393 | MNVL_NC004148 2389 2414 F | | 17 | MNVL_NC004148 2468 2488 R | | 185 |
| 394 | MNVL_NC004148 2389 2408 F | | 18 | MNVL_NC004148 2479 2498 R | | 186 |
| 395 | MNVL_NC004148 2738 2756 **F** | | 19 | MNVL_NC004148 2794 2810 R | | 187 |
| 409 | CRVCP_AY21983 6 2 22 F | | 20 | CRVCP_AY21983 6 46 70 R | | 188 |
| 410 | CRVCP_AY21983 6 45 69 F | | 21 | CRVCP_AY21983 6 136 160 R | | 189 |
| 411 | CRVCP_AY21983 6 475 499 F | | 22 | CRVCP_AY21983 6 577 601 R | | 190 |
| 412 | CRVRP_AY21983 6 24 48 F | | 23 | CRVRP_AY21983 6 84 108 R | | 191 |
| 413 | CRVRP_AY21983 6 440 464 F | | 24 | CRVRP_AY21983 6 495 517 R | | 192 |
| 414 | CRVRP_AY21983 6 658 683 F | | 25 | CRVRP_AY21983 6 730 754 R | | 193 |
| 415 | CRVRP_AY21983 6 775 796 F | | 26 | CRVRP_AY21983 6 843 869 R | | 194 |
| 990 | HIV1_NC001802 7344 7364 F | | 27 | HIV1_NC001802 7413 7439 R | | 195 |
| 991 | HIV1_NC001802 7340 7361 F | | 28 | HIV1_NC001802 7413 7438 R | | 196 |
| 992 | HIV1_NC001802 4983 5014 F | | 29 | HIV1_NC001802 5104 5127_R | | 197 |
| 993 | HIV1_NC001802 1089 1117 F | | 30 | HIV1_NC001802 1178 1204 R | | 198 |
| 994 | HIV2_NC001722 8414 8439 F | | 31 | HIV2_NC001722 8476 8500 R | | 199 |
| 995 | HIV2_NC001722 8425 8450 F | | 32 | HIV2_NC001722 8476 8502 R | | 200 |
| 996 | HIV2_NC001722 5050_5075 F | | 33 | HIV2_NC001722 5169 5196 R | | 201 |
| 997 | HIV2_NC001722 5050 5075 F | | 33 | HIV2_NC001722 5156 5187 R | | 202 |
| 998 | HTLV1_NC00143 6 7221 7245 F | | 34 | HTLV1_NC00143 6 7330 7353 R | | 203 |
| 999 | HTLV1_NC00143 6 7094 7118 F | | 35 | HTLV1_NC00143 6 7153 7177 R | | 204 |
| 1000 | HTLV1_NC00143 6 7388 7410 F | | 36 | HTLV1_NC00143 6 7489 7516 R | | 205 |
| 1001 | HTLV1_NC00143 6 7818 7843 F | | 37 | HTLV1_NC00143 6 7925 7947 R | | 206 |
| 1002 | HTLV1_NC00143 6 7340 7361 F | | 38 | HTLV1_NC00143 6 7404 7428_R | | 207 |
| 1003 | HTLV1_NC00143 6_7131 7156 F | | 39 | HTLV1_NC00143 6 7211 7233_R | | 208 |
| 1004 | HTLV2_NC00148 8_8180 8200 F | | 40 | HTLV2_NC00148 8 8254 8279_R | | 209 |
| 1005 | HTLV2_NC00148 8 7757 7779 F | | 41 | HTLV2_NC00148 8 7840 7861_R | | 210 |
| 1006 | HTLV2_NC00148 8 2435 2456 F | | 42 | HTLV2_NC00148 8_2516 2540_R | | 211 |
| 1007 | HTLV2_NC00148 8 3592 3616 F | | 43 | HTLV2_NC00148 8 3680 3704 R | | 212 |
| 1008 | HTLV2_NC00148 8 2880 2904 F | | 44 | HTLV2_NC00148 8 2989 3013 R | | 213 |
| 1009 | HTLV2_NC00148 8 1896 1919 F | | 45 | HTLV2_NC00148 8 1993 2015 R | | 214 |
| 1010 | HTLV2_NC00148 8 1198 1222 F | | 46 | HTLV2_NC00148 8 1268 1291 R | | 215 |
| 1011 | HTLV2_NC00148 8 5735 5758 F | | 47 | HTLV2_NC00148 8 5847 5870 R | | 216 |
| 1012 | HTLV2_NC00148 8 5241 5265 F | | 48 | HTLV2_NC00148 8 5332 5356 R | | 217 |
| 1013 | HCV_NC001433_ 66 91 F | | 49 | HCV_NC001433_ 121 145 R | | 218 |
| 1014 | HCV_NC001433_ 66 91 F | | 49 | HCV_NC001433_ 146 167 R | | 219 |
| 1015 | HCV_NC001433_ 66 91 F | | 49 | HCV_NC001433_ 128 153_R | | 220 |
| 1016 | HCV_NC001433_ 51 74 F | | 50 | HCV_NC001433_ 121 145 2 R | | 221 |
| 1017 | HCV_NC001433_ 51 73 F | | 51 | HCV_NC001433_ 146 167 R | | 219 |
| 1018 | HCV_NC001433_ 51 73 F | | 51 | HCV_NC001433_ 128 153 R | | 220 |
| 1019 | HCV_NC001433_ 62 85 F | | 52 | HCV_NC001433_ 128 153 R | | 220 |
| 1020 | HCV_NC001433_ 227 248 F | | 53 | HCV_NC001433_ 277 298 R | | 222 |
| 1021 | HCV_NC001433_ 671 698_F | | 54 | HCV_NC001433_ 720 742 R | | 223 |
| 1022 | HCV_NC001433_ 8598 8623 F | | 55 | HCV_NC001933_ 8674 8700 R | | 224 |
| 1023 | WN_NC001563_8 365 8390 F | | 56 | WN_NC001563_8 434 8463 R | | 225 |
| 1024 | WN_NC001563_8 654 8678 F | | 57 | WN_NC001563_8 749 8771 R | | 226 |
| 1025 | WN_NC001563_9 026 9055 F | | 58 | WN_NC001563_9 101 9121 R | | 227 |
| 1026 | WN_NC001563_1 0135 10164 F | | 59 | WN_NC001563_1 0216 10237 R | | 228 |
| 1027 | WN_NC001563_9 016 9043 F | | 60 | WN_NC001563_9 088 9112 R | | 229 |
| 1028 | WN_NC001563_5 696 5721 F | | 61 | WN_NC001563_5 758 5783 R | | 230 |
| 1029 | WN_NC001563_5 687 5714 F | | 62 | WN_NC001563_5 796 5826 R | | 231 |
| 1030 | WN_NC001563_1 28 152 F | | 63 | WN_NC001563_1 87 212 R | | 232 |
| 1031 | WN_NC001563_5 994 6017_F | | 64 | WN_NC001563_6 079 6104 R | | 233 |
| 1032 | WN_NC001563_3 527 3552 F | | 65 | WN_NC001563_3 591 3613_R | | 234 |
| 1245 | HBV_X51970_32 0 342 F | | 66 | HBV_X51970_37 9 402 R | | 235 |
| 1246 | HBV_X51970_31 7 341 F | | 67 | HBV_X51970_37 9 406 R | | 236 |
| 1247 | HBV_X51970_31 1 335 F | | 68 | HBV_X51970_38 2 410 R | | 237 |
| 1248 | HBV_X51970_13 75 1399 F | | 69 | HBV_X51970_14 12 1436 R | | 238 |
| 1249 | HBV_X51970_17 77 1798 F | | 70 | HBV_X51970_18 68 1886 R | | 239 |
| 1250 | HBV_X51970_18 3 203 F | | 71 | HBV_X51970_22 8 254 R | | 240 |
| 1251 | HBV_X51970_18 0 202 F | | 72 | HBV_X51970_26 2 289 R | | 241 |
| 1252 | HBV_X51970_37 4 392 F | | 73 | HBV_X51970_42 3 450 R | | 242 |
| 1253 | HBV_X51970_36 8 388 F | | 74 | HBV_X51970_42 4 453 R | | 243 |
| 1254 | HBV_X51970_31 2 335_F | | 75 | HBV_X51970_37 6 398 R | | 244 |
| 2293 | HTLV_TAX_GENE _NC_001436_71 07 7130_F | | 76 | HTLV_TAX_GENE _NC_001436_71 69 7192 R | | 245 |
| 2294 | HTLV_TAX_GENE _NC_001436_70 94 7118 F | | 77 | HTLV_TAX_GENE _NC_001436_72 03 7226 R | | 246 |
| 2295 | HTLV_TAX_GENE _NC_001436_69 89 7017 F | | 78 | HTLV_TAX_GENE _NC_001436_71 09 7129 R | | 247 |
| 2408 | ARENAS_NC0042 96 474 494 F | | 79 | ARENAS_NC0042 96 520 540 R | | 248 |
| 2409 | ARENAS_NC0042 96_474_494_2_ F | | 80 | ARENAS_NC0042 96_520_540_2_ R | | 249 |
| 2410 | ARENAS_NC0092 96 931 953 F | | 81 | ARENAS NC0042 96 982 1002 R | | 250 |
| 2411 | ARENAS_NC0042 96_931_953_2_ F | | 82 | ARENAS_NC0042 96 982 1000 R | | 251 |
| 2412 | ARENAS_NC0042 93 459 479 F | | 83 | ARENAS_NC0042 93 505 527 R | | 252 |
| 2413 | ARENAS_NC0042 93 459 479 F | | 83 | ARENAS_NC0042 93 511 534 R | | 253 |
| 2414 | ARENAS_NC0042 93 459 479 F | | 83 | ARENAS_NC0042 93_511_534_2_ R | | 254 |
| 2415 | ARENAS_NC0042 93 459 479 F . | | 83 | ARENAS_NC0042 93 511 534P R | | 255 |
| 2416 | ARENAS_NC0042 93 915 937 F | | 84 | ARENAS_NC0042 93 975 994 R | | 256 |
| 2417 | ARENAS_NC0042 93 915 937 F | | 84 | ARENAS_NC0042 93 975 994P R | | 257 |
| 2418 | ARENAS_NC0042 93_915_937_2_ F | | 85 | ARENAS_NC0042 93 975 994 R | | 256 |
| 2419 | ARENAS_NC0042 93_915_937_2_ F | | 85 | ARENAS_NC0042 93 975 994P R | | 257 |
| 2420 | ARENAS_NC0042 93 915 937P F | | 86 | ARENAS_NC0042 93 975 994P R | | 257 |
| 2423 | POL_NC003461_ 1620 1649 F | | 87 | POL_NC003461_ 1747 1772 R | | 258 |
| 2424 | POL_NC003461_ 2107 2138 F | | 88 | POL_NC003461_ 2215 2244 R | | 259 |
| 2425 | POL_NC003461_ 2253 2279 F | | 89 | POL_NC003461_ 2302 2329 R | | 260 |
| 2426 | POL_NC003461_ 2253 2279 F | | 89 | POL_NC003461_ 2320 2349 R | | 261 |
| 2427 | POL_NC003461_ 2253 2279 F | | 89 | POL_NC003461_ 2320 2352 R | | 262 |
| 2428 | POL_NC003461_ 2256 2279 F | | 90 | POL_NC003461_ 2302 2324 R | | 170 |
| 2429 | POL_NC003461_ 2463 2488 F | | 91 | POL_NC003461_ 2497 2523 R | | 263 |
| 2430 | POL_NC003461_ 2935 2960 F | | 92 | POL_NC003461_ 2980 3004 R | | 264 |
| 2431 | POL_NC003461_ 2935 2960 2 F | | 93 | POL_NC003461_ 2980 3004 R | | 264 |
| 2432 | POL_NC003461_ 3644 3668 F | | 94 | POL_NC003461_ 3760 3783 R | | 265 |
| 2433 | POL_NC003461_ 3759 3782 F | | 95 | POL_NC003461_ 3880 3912 R | | 266 |
| 2434 | POL_NC003461_ 3759 3782 2 F | | 96 | POL_NC003461_ 3880 3912 2 R | | 267 |
| 2435 | RUBPOL_NC0034 43_1636_1661_ F | | 97 | RUBPOL_NC0034 43_1708_1734_ R | | 268 |
| 2436 | RUBPOL_NC0034 43_2067_2093_ F | | 98 | RUBPOL_NC0034 43_2143_2167_ R | | 269 |
| 2437 | RUBPOL_NC0034 43_2133_2156_ F | | 99 | RUBPOL_NC0034 43_2251_2275_ R | | 270 |
| 2438 | RUBPOL_NC0034 43_2237_2261P F | | 100 | RUBPOL_NC0034 43_2318_2341P R | | 271 |
| 2439 | RUBPOL_NC0034 43_2249_2276_ F | | 101 | RUBPOL_NC0034 43_2301_2324_ R | | 272 |
| 2440 | RUBPOL_NC0034 43_3689_3713_ F | | 102 | RUBPOL_NC0034 43_3754_3778_ R | | 273 |
| 2441 | PNVL_U50363_2 094 2120 F | | 103 | PNVL_U50363_2 161 2181 R | | 274 |
| 2442 | PNVL_U50363_2 110 2135 F | | 104 | PNVL_U50363_2 161 2181 R | | 274 |
| 2443 | PNVL_U50363_2 587 2612 F | | 105 | PNVL_U50363_2 677 2704 R | | 275 |
| 2444 | PNVL_U50363_2 581 2612 F | | 106 | PNVL_U50363_2 666 2686 R | | 276 |
| 2445 | PNVL_U50363_2 583 2609 F | | 107 | PNVL_U50363_2 677 2704 R | | 275 |
| 2446 | PNVL_U50363_2 660 2687 F | | 108 | PNVL_U50363_2 740 2769 R | | 277 |
| 2447 | PNVL_U50363_2 660 2684 F | | 109 | PNVL_U50363_2 740 2769 R | | 277 |
| 2448 | PNVL_U50363_2 660 2684 F | | 109 | PNVL_U50363_2 743 2776 R | | 278 |
| 2449 | PNVL_U50363_2 663 2687 F | | 110 | PNVL_U50363_2 740 2769 2 R | | 279 |
| 2450 | PNVL_U50363_1 868 1894 F | | 111 | PNVL_U50363_1 918 1946 R | | 280 |
| 2451 | PNVL_U50363_1 874 1898 F | | 112 | PNVL_U50363_1 918 1946 R | | 280 |
| 2452 | PNVL_U50363_1 872 1894 F | | 113 | PNVL_U50363_1 918 1946 2 R | | 281 |
| 2453 | MVL_AF266286_ 2002 2027 F | | 114 | MVL_AF266286_ 2035 2060 R | | 282 |
| 2454 | MVL_AF266286_ 2002 2027 2 F | | 115 | MVL_AF266286_ 2125 2144 R | | 283 |
| 2467 | PNVL_U50363_2 660 2684 1F | | 109 | PNVL_U50363_2 743 2776 1R | | 284 |
| 2533 | PAV_IMP_NC001 526_6222_6253 F | | 116 | PAV_IMP_NC001 526_6321_6355 R | | 285 |
| 2534 | PAV_IMP_NC001 526_6222_6253 F | | 116 | PAV_IMP_NC001 526_6324_6355 R | | 286 |
| 2535 | PAV_IMP_NC001 526_6222_6253 2 F | | 117 | PAV_IMP_NC001 526_6324_6355 2 R | | 287 |
| 2536 | PAV_IMP_NC001 526_6212_6238 F | | 118 | PAV_IMP_NC001 526_6324_6355 R | | 286 |
| 2537 | PAV_A9_NC0015 26_5632_5655_ F | | 119 | PAV_A9_NC0015 26_5691_5720_ R | | 288 |
| 2538 | PAV_A9_NC0015 26_5632_5655_ F | | 119 | PAV_A9_NC0015 26_5691_5720_ R | | 288 |
| 2539 | PAV_A9_NC0015 26_2688_2715_ F | | 120 | PAV_A9_NC0015 26_2773_2802_ R | | 289 |
| 2540 | PAV_A9_NC0015 26_1972_2003_ F | | 121 | PAV_A9_NC0015 26_2085_2112_ R | | 290 |
| 2541 | PAV_A7_NC0013 57_2011_2036_ F | | 122 | PAV_A7_NC0013 57_2096_2121_ R | | 291 |
| 2542 | PAV_A7_NC0013 57_2011_2036_ F | | 122 | PAV_A7_NC0013 57_2108_2135_ R | | 292 |
| 2543 | PAV_A7_NC0013 57_4507_4530_ F | | 123 | PAV_A7_NC0013 57_4607_4629_ R | | 293 |
| 2544 | PAV_A7_NC0013 57 748 767 F | | 124 | PAV_A7_NC0013 57 875 895 R | | 294 |
| 2545 | PAV_A7_NC0013 57 947 966 F | | 125 | PAV_A7_NC0013 57_1034_1057_ R | | 295 |
| 2546 | PAV_A10_NC000 904 875 903 F | | 126 | PAV_A10_NC000 904_997_1027_ R | | 296 |
| 2547 | PAV_A10_NC000 904_1000_1027 F | | 127 | PAV_A10_NC000 904_1055_1079 R | | 297 |
| 2548 | PAV_A10_NC000 904_1222_1246 F | | 128 | PAV_A10_NC000 904_1275_1296 R | | 298 |
| 2549 | POLYOMA_NC001 669_2685_2713 F | | 129 | POLYOMA_NC001 669_2774_2796 R | | 299 |
| 2550 | POLYOMA_NC001 669_2691_2719 F | | 130 | POLYOMA_NC001 669_2774_2796 R | | 299 |
| 2551 | POLYOMA_NC001 669_2698_2719 F | | 131 | POLYOMA_NC001 669_2774_2796 R | | 299 |
| 2552 | POLYOMA_NC001 669_2726_2749 F | | 132 | POLYOMA_NC001 669_2774_2796 R | | 299 |
| 2553 | POLYOMA_NC001 669_2726_2749 2 F | | 133 | POLYOMA_NC001 669_2774_2796 R | | 299 |
| 2554 | POLYOMA_NC001 669_2767_2788 F | | 134 | POLYOMA_NC001 669_2899_2917 R | | 300 |
| 2555 | POLYOMA_NC001 669_4496_4520 F | | 135 | POLYOMA_NC001 669_4583_4606 R | | 301 |
| 2556 | POLYOMA_NC001 669_4533_4558 F | | 136 | POLYOMA_NC001 669_4633_4660 R | | 302 |
| 2557 | POLYOMA_NC001 669_4537_4558 F | | 137 | POLYOMA_NC001 669_4621_4641 R | | 303 |
| 2558 | POLYOMA_NC001 669_2978_3000 F | | 138 | POLYOMA_NC001 669_3084_3110 R | | 304 |
| 2559 | POLYOMA_NC001 669_2967_2986 F | | 139 | POLYOMA_NC001 669_3087_3115 R | | 305 |
| 2560 | POLYOMA_NC001 669_3392_3412 F | | 140 | POLYOMA_NC001 669_3424_3446 R | | 306 |
| 2561 | POLYOMA_NC001 669_3780_3803 F | | 141 | POLYOMA_NC001 669_3816_3840 R | | 307 |
| 2643 | HTLV1_NC00143 6 7387 7410 F | | 142 | HTLV1_NC00143 6 7489 7516 R | | 205 |
| 2670 | PAV_IMP_MOD_N C001526_6222_ 6253 F | | 116 | PAV_IMP_MOD_N C001526_6321_ 6355 R | | 308 |
| 2671 | PAV_IMP_MOD_N C001526_6222_ 6253 F | | 116 | PAV_IMP_MOD_N C001526_6321_ 6355 2 R | | 309 |
| 2672 | PAV_IMP_MOD_N C001526_6222_ 6253 F | | 116 | PAV_IMP_MOD_N C001526_6321_ 6355 3 R | | 310 |
| 2673 | PAV_IMP_MOD_N C001526_6222_ 6253 F | | 116 | PAV_IMP_MOD_N C001526_6324_ 6355P R | | 311 |
| 2674 | PAV_IMP_MOD_N C001526_6222_ 6253 F | | 116 | PAV_IMP_MOD_N C001526_6324_ 6355 R | | 312 |
| 2675 | PAV_IMP_MOD_N C001526_6222_ 6253 F | | 116 | PAV_IMP_MOD_N C001526_6324_ 6355 2 R | | 313 |
| 2676 | PAV_IMP_MOD_N C001526_6222_ 6253 F | | 116 | PAV_IMP_MOD_N C001526_6324_ 6355 3 R | | 314 |
| 2677 | PAV_IMP_MOD_N C001526_6222_ 6253 F | | 116 | PAV_IMP_MOD_N C001526_6324_ 6355 4 R | | 315 |
| 2678 | PAV_IMP_MOD_N C001526_6222_ 6253 2 F | | 143 | PAV_IMP_MOD_N C001526_6324_ 6355 2 R | | 313 |
| 2679 | PAV_IMP_MOD_N C001526_6222_ 6253 3 F | | 144 | PAV_IMP_MOD_N C001526_6324_ 6355 3 R | | 314 |
| 2680 | PAV_IMP_MOD_N C001526_6222_ 6253 4 F | | 145 | PAV_IMP_MOD_N C001526_6324_ 6355 4 R | | 315 |
| 2681 | PAV_IMP_MOD_N C001526_6212_ 6238 F | | 118 | PAV_IMP_MOD_N C001526_6324_ 6355 2 R | | 313 |
| 2682 | PAV_IMP_MOD_N C001526_6212_ 6238 F | | 118 | PAV_IMP_MOD_N C001526_6324_ 6355 3 R | | 314 |
| 2683 | PAV_IMP_MOD_N C001526_6212_ 6238 F | | 118 | PAV_IMP_MOD_N C001526_6324_ 6355 4 R | | 315 |
| 2684 | PAV_IMP_MOD_N C001526_6212_ 6238 2 F | | 146 | PAV_IMP_MOD_N C001526_6324_ 6355 2 R | | 313 |
| 2685 | PAV_IMP_MOD_N C001526_6212_ 6238 3 F | | 147 | PAV_IMP_MOD_N C001526_6324_ 6355 3 R | | 314 |
| 2686 | PAV_IMP_MOD_N C001526_6212_ 6238 4 F | | 148 | PAV_IMP_MOD_N C001526_6324_ 6355 4 R | | 315 |
| 2687 | PAV_A9_MOD_NC 001526_5632_5 655 F | | 149 | PAV_A9_MOD_NC 001526_5691_5 720 R | | 288 |
| 2688 | PAV_A9_MOD_NC 001526_5632_5 655 2 F | | 150 | PAV_A9_MOD_NC 001526_5691_5 720 R | | 288 |
| 2689 | PAV_A9_MOD_NC 001526_2688_2 715 F | | 120 | PAV_A9_MOD_NC 001526_2773_2 802 R | | 316 |
| 2690 | PAV_A9_MOD_NC 001526_2688_2 715 F | | 120 | PAV_A9_MOD_NC 001526_2773_2 802 2 R | | 317 |
| 2691 | PAV_A9_MOD_NC 001526_2688_2 715 F | | 120 | PAV_A9_MOD_NC 001526_2773_2 802 3 R | | 318 |
| 2692 | PAV_A9_MOD_NC 001526_1972_2 003 F | | 151 | PAV_A9_MOD_NC 001526_2085_2 112 R | | 288 |
| 2693 | PAV_A7_A10_NC 000904_1912_1 937 F | | 122 | PAV_A7_A10_NC 000904_1997_2 022 R | | 319 |
| 2694 | PAV_A7_A10_NC 000904_1912_1 937 F | | 122 | PAV_A7_A10_NC 000904_1997_2 022 2 R | | 320 |
| 2695 | PAV_A7_A10_NC 000904_1912_1 937 2 F | | 152 | PAV_A7_A10_NC 000904_1997_2 022 R | | 319 |
| 2696 | PAV_A7_A10_NC 000904_1912_1 937 F | | 122 | PAV_A7_A10_NC 000904_2009_2 036 R | | 321 |
| 2807 | PYV_NO_MAMMAL _NC001663_313 2 3159 F | | 153 | PYV_NO_MAMMAL _NC001663_326 1 3281 R | | 322 |
| 2808 | PYV_NO_MAMMAL _NC001663_326 7 3295 F | | 154 | PYV_NO_MAMMAL _NC001663_335 0 3386 R | | 323 |
| 2809 | PYV_NO_MAMMAL _NC001663_357 8 3598 F | | 155 | PYV_NO_MAMMAL _NC001663_368 9 3716 R | | 324 |
| 2810 | PYV_NO_MAMMAL _NC001663_356 1 3586 F | | 156 | PYV_NO_MAMMAL _NC001663_368 6 3716 R | | 325 |
| 2811 | PYV_NO_MAMMAL _NC001663_326 7 3294 F | | 157 | PYV_NO_MAMMAL _NC001663_335 1 3386 R | | 326 |
| 2812 | PYV_NO_MAMMAL _NC001663_326 7 3294 2 F | | 158 | PYV_NO_MAMMAL _NC001663_335 1 3386 R | | 326 |
| 2813 | PYV_NO_MAMMAL _NC001663_326 7 3294 F | | 157 | PYV_NO_MAMMAL _NC001663_337 2 3404 R | | 327 |
| 2814 | PYV_NO_MAMMAL _NC001663_326 7 3294 2 F | | 158 | PYV_NO_MAMMAL _NC001663_337 2 3404 R | | 327 |
| 2864 | AAV_NS1_NC002 077_1005_1028 F | | 159 | AAV_NS1_NC002 077_1126_1149 R | | 328 |
| 2865 | AAV_NS1_NC002 077_1066_1094 F | | 160 | AAV_NS1_NC002 077_1126_1149 2 R | | 329 |
| 2866 | AAV_NS1_NC002 077_1020_1046 F | | 161 | AAV_NS1_NC002 077_1126_1149 2 R | | 329 |
| 2867 | AAV_NS1_NC002 077_1020_1046 F | | 161 | AAV_NS1_NC002 077_1072_1099 R | | 330 |
| 2868 | AAV_VP1_NC002 077 739 764 F | | 162 | AAV_VP1_NC002 077 832 859 R | | 331 |
| 2869 | AAV_VP1_NC002 077 743 764 F | | 163 | AAV_VP1_NC002 077 847 872 R | | 332 |
| 2870 | AAV_VP1_NC002 077 648 665 F | | 164 | AAV_VP1_NC002 077 680 703 R | | 333 |
| 2871 | AAV_VP1_NC002 077_648_665_2 F | | 165 | AAV_VP1_NC002 077_680_703_2 R | | 334 |
| 2872 | AAV_VP1_NC002 077 831 851 F | | 166 | AAV_VP1_NC002 077 886 910 R | | 335 |
| 2873 | AAV_VP1_NC002 077 838 867 F | | 167 | AAV_VP1_NC002 077 886 910 R | | 335 |
| 2874 | AAV_VP1_NC002 077_838_867_2 F | | 168 | AAV_VP1_NC002. 077_886_910_2 R | | 336 |
| 2875 | AAV_VP1_NC002 077 670 693 F | | 169 | AAV_VP1_NC002 077 748 772 R | | 337 |

A total of 205 primer pairs were designed and are shown in Table1, which represents a collection of primers (sorted by primer pair number) designed to identify adventitious contaminant viruses using the methods described herein. "I" represents inosine. Tp represents propynylated T and Cp represents propynylated C. Propyne modifications are at the 5-position of the base. Each primer pair number is an in-house database index number. Eace forward or reverse primer name shown in Table 1 indicates the gene region of the viral genome to which the primer hybridizes relative to a reference sequence. For example, the forward primer name RVL_X03614_2256_2279_F indicates that the forward primer (_F) hybridizes to residues 2256-2279 of a respirovirus (*Paramyxoviridae*) (RLV) sequence (GenBank Accession Number X03614). In this example, RLV is the primer pair name virus identifier. Table 2 indicates the primer pair name virus identifier for the primer pairs disclosed herein.

**Table 2: Primer Pair Name Identifiers for Selected Viruses**

| **Virus** | **Primer Pair Name Virus Identifier** |
|---|---|
| Respirovirus (Paramyxoviridae) | RVL |
| Pneumovirus (Paramyxoviridae) | PVL |
| Morbillivirus (Paramyxoviridae) | MVL |
| Measles virus (Morbillivirus;Paramyxoviridae) | MSVL |
| Pneumovirus (Paramyxoviridae) | PVNL |
| Metapneumovirus (Paramyxoviridae) | MNVL |
| Porcine circovirus (Circoviridae) | CRVCP |
| Human Immunodeficiency virus 1 | HIV1 |
| Human Immunodeficiency virus 2 | HIV-2 |
| HTLV-1 | HTLV1 |
| Hepatitis C virus | HCV |
| West Nile virus | WN |
| Hepatitis B virus (Hepadnaviridae) | HBV |
| Human T-cell Lymphotropic Virus | HTLV TAX GENE |
| Old world Arena Virus | ARENAS |
| Respirovirus | POL |
| Rubulavirus | RUBPOL |
| Pneumovirinae | PNVL |
| Papillomavirus | PAV IMP |
| Papillomavirus A9 | PAV A9 |
| Papillomavirus A7 | PAV A7 |
| Papillomavirus A10 | PAV A10 |
| Polyomavirus | POLYOMA |
| Polyomavirus (non-mammalian) | PYV NO MAMMAL |
| Dependovirus,(Parvovirinae)NS1 gene | AAV NS1 |
| Dependovirus,(Parvovirinae)VP1 gene | AAV VP1 |

### Design of Primers for Identification of Retroviruses

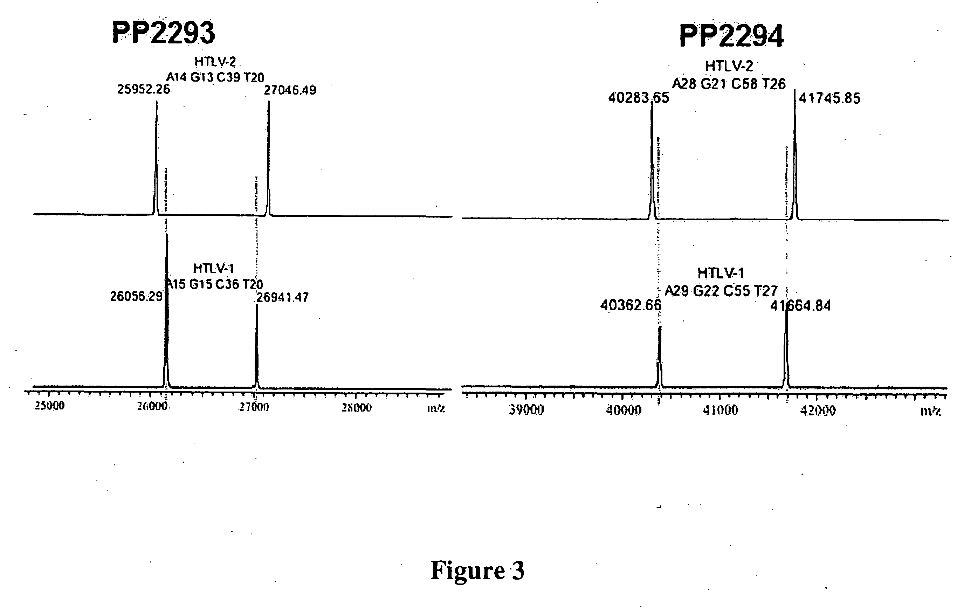

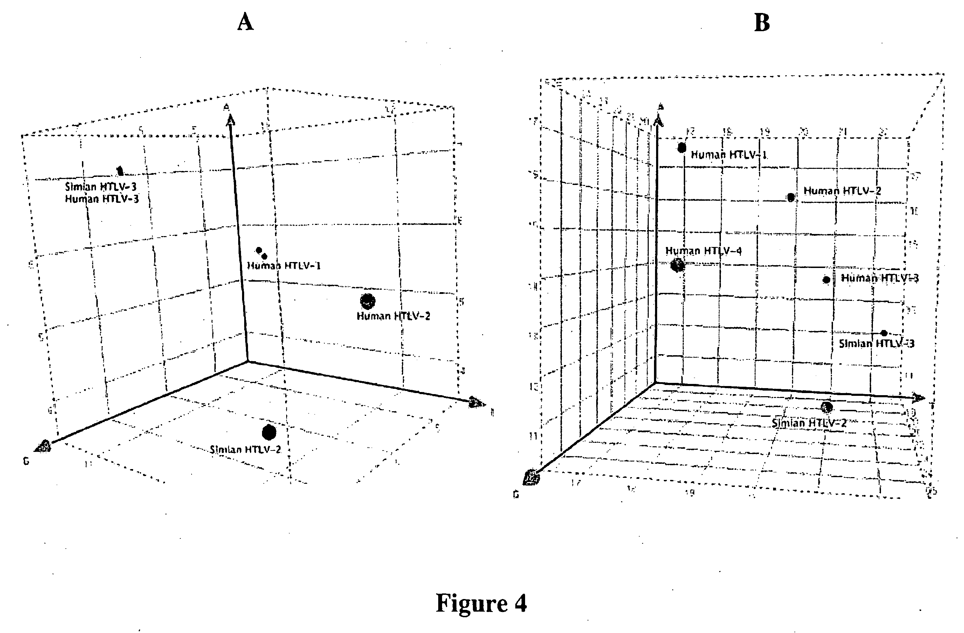

The objective of primer design for this viral family was to obtain primer pairs that would prime and produce retrovirus identifying amplicons for all known members of each of the genus groups and as yet unknown variants. The T-lymphotropic viruses, members of the deltaretrovirus genus, infect primates and cause leukemia and neurologic diseases. These 9 kilobase single stranded RNA viruses are highly transmissible. Primer pairs targeting the transcription activator (tax) gene were designed to broadly prime and resolve all known primate T-lymphotropic viruses including human T-lymphotropic viruses (HTLV-1 and -2 and the newly discovered HTLV-3 and -4), and simian T-lymphotropic viruses (STLV-1, -2 and -3). Figure 3 indicates that primer pair numbers 2293 (SEQ ID NOs: 76:245) and 2294 (SEQ ID NOs: 77:246) both amplify nucleic acid segments of the tax gene of HTLV-1 and HTLV-2 to give rise to amplification products which differ sufficiently in molecular mass to distinguish these two viruses. Shown in Figures 4A and 4B are 3D plots of the base compositions of retrovirus identifying amplicons of simian and human T-lymphotropic virus species indicating that the primer pairs can yield amplification products which are distinguishable on the basis of their base compositions.

### Design of Primers for Identification of Polyomaviruses

Approximately 200 complete Polyomavirus genome sequences were obtained from GenBank. These genome sequences (approximately 5.3 kilobases long) were aligned to each other using bioinformatics tools built in-house, and scanned for conserved target regions. Initial survey of the genome alignments revealed high degree of homology between the primate (SV40) and the human viruses (BK and JC), whereas the rest of the species were highly divergent and didn't share much sequence homology with these above species. For primer design purposes, SV40, BK and JC viral species were classified as a group. Nine different primer pairs (primer pairs 2549-2557) were designed to this cluster (See Table 1) and are expected to provide redundant detection and resolution of the three important Polyomavirus species. Most of these primers were targeted to the large T antigen gene of Polyomavirus. Three additional primer pairs (VIR 2559-2561) were designed to include Lymphotropic papovavirus (LPV, the African green monkey papovavirus) to the above cluster. While these new primers were less well conserved across any one species, they would nonetheless provide broader coverage of viral detection within this family. Additional primer pairs (RS10-14) targeting the rest of the viral species (murine, avian, bovine, etc.) were also designed. Taken together, these primers would provide complete coverage of all known Polyomaviruses.

All of the primer pairs were tested against multiple target species for performance and sensitivity. To test the performance of these primers, we obtained plasmid clones containing full length SV40 (ATCC: VRMC-4) and JC virus (ATCC: VRMC-1) DNA from ATCC. Plasmid concentrations were determined by OD measurements and used as an approximate estimate of the amount of input viral DNA template. Serial 10-fold dilutions of the plasmid were used for estimating limits of detection. These were tested against the entire panel of 12 primer pairs (VIR2549-61). The primers were initially tested at 10⁻⁷ and 10⁻⁸ fold dilutions of each of the plasmids and showed reliable detections, with the exception of primer 2555. Additional testing of a subset of these primers showed that while several primers were able to detect additional, lower dilutions, some of the primers dropped out below the 10⁻⁹ dilution. Based on this initial testing, a panel of six primers (VIR2550, 2551, 2553, 2554, 2557 and 2559) was chosen for use in cell-line characterization. These primers will be tested against known cell lines containing SV-40 and other Polyomaviruses.

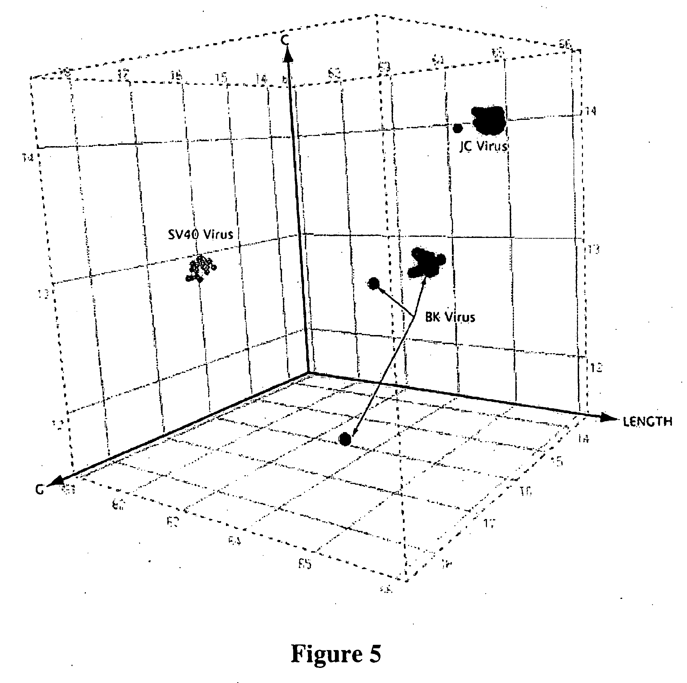

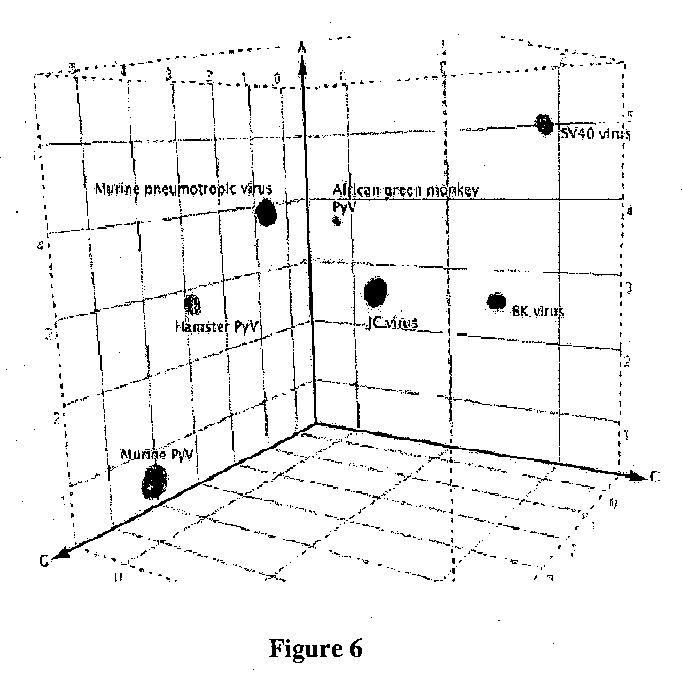

For routine screening of cell lines, it is anticipated that as few as two of the primer pairs described above along with the four primers targeting the animal Polyomavirus can provide complete coverage of all known and potentially novel Polyomaviruses. A nucleic acid segment within the large tumor antigen gene provides opportunities for broad priming across human and simian species due to a codon deletion at position 32 of the simian virus 40, which is exemplified by primer pair number 2555 (SEQ ID NO: 135:301). Species differentiation of SV40 virus, BK virus and JC virus is indicated in Figure 5 wherein base compositions of hypothetical amplification products of polyomaviruses are shown on a 3D diagram. Shown in Figure 6 is a 3D diagram of base compositions of hypothetical amplification products produced with primer pair number 2560 (SEQ ID NOs: 140:306).Murine pneumonotropic virus, African green monkey PyV virus, SV40 virus, BK virus, JC virus, hamster PyV and murine PyV virus can be distinguished from each other on the basis of base compositions of amplification products produced with primer pair number 2560.

### Design of Primers for Identification of Papillomaviruses

Broad primer pairs covering a set of important human Papillomaviruses (HPV 16, 18, 31, 45, 11, 6, 2) were designed (primer pair numbers 2533-2536). These belong to different groups, but have all been reported in literature to be "high risk" Covering all of these species broadly combined with group-specific primer pairs described above would be of great value. Additionally, several primer pairs were designed to cover broadly within a single group or across multiple groups of Papillomaviruses to increase robustness of detection.

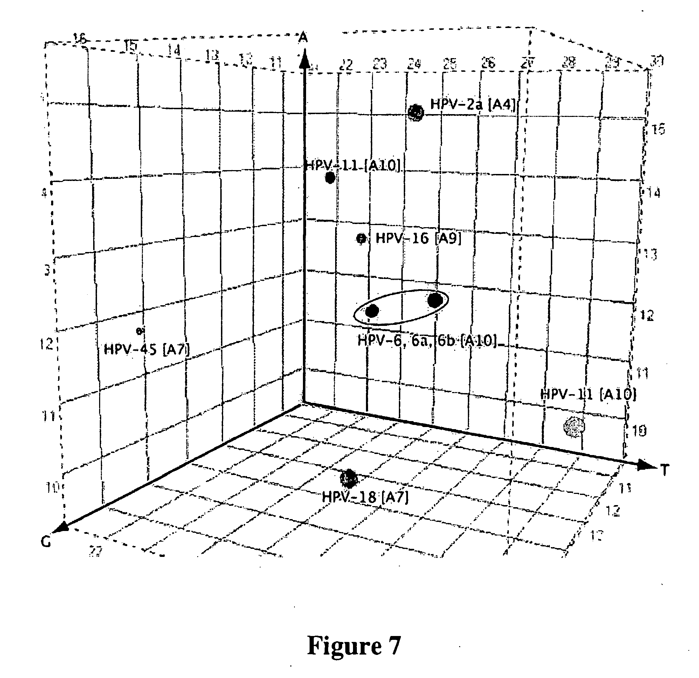

All of the primer pairs were tested against a panel of Papillomaviruses obtained from ATCC. The following viruses were obtained as full-length plasmid clones: ATCC 45150D (HPV-6b); ATCC 45151D (HPV-11); ATCC 45152D (HPV-18); and ATCC 45113D (HPV-16). Two of the broad primer pairs (numbers 2534 and 2536) amplified all four viruses tested at two different dilutions of the plasmids. Primer pair number 2535 amplified only two of the test isolates, while prime pair 2533 did not amplify any virus tested. Based on these initial results, Primer pair numbers 2534 and 2536 were selected for further optimization. A series of primer modifications, including use of inosines to overcome potential sequence mismatches were introduced in the forward and reverse primer pairs. Most of the modified primers tested showed improved performance across the test isolates. In addition to the primers broadly targeting the major species, a series of primers targeting Papillomavirus groups, A7, A9 and A10 that account for over 30 different Papillomaviruses were also tested. Shown in Figure 7 is a 3D diagram of base compositions of hypothetical amplification products of human Papillomaviruses produced with primer pair number 2534 (SEQ ID NOs: 116:286). Table 3 provides the primer pairs used for Papillomavirus identification and indicates isolates tested, target virus groups and major species covered.

**Table 3: Primer Pairs Targeting Human Papillomaviruses**

| **Primer Pair Number** | **Isolates Tested** | **Target Virus Group** | **Major Species Covered** |
|---|---|---|---|
| 2537 | HPV-16 | Group A9 | HPV-16, HPV-31, HPV-33, HPV-35, HPV-52, HPV-58, HPV-67, and RhPV |
| 2539 | | | |
| 2540 | | | |
| 2543 | HPV-18 | Group A7 | HPV-18, HPV-39, HPV-45, HPV-59, HPV-68, and HPV-70 |
| 2544 | | | |
| 2545 | | | |
| 2546 | HPV-6, HPV-11 | Group A10 | HPV-6, HPV-11, HPV-13, HPV-44, HPV-55, and PCPV |
| 2547 | | | |
| 2548 | | | |
| 2541 | HPV-6, HPV-11, HPV-18 | Groups A1, A7, A8, A10 and A11 | >30 different Papilloma viruses |
| 2542 | | | |

### Validation of Primer Pairs Designed for Identification of Papillomavirueses

For additional testing and validation, two different HeLa cell lines infected with HPV-18 were obtained from ATCC (CCL-2 and CCL-2.2). These were tested at limiting dilutions using a subset of the primers tested above. Results are shown below. The primer pairs used for this test included the major human PaV primer pairs, 2534, 2536 and 2685, the multi-group primer 2542, the Group A7 targeted primers 2544-5 and the Group A10 primer 2546.

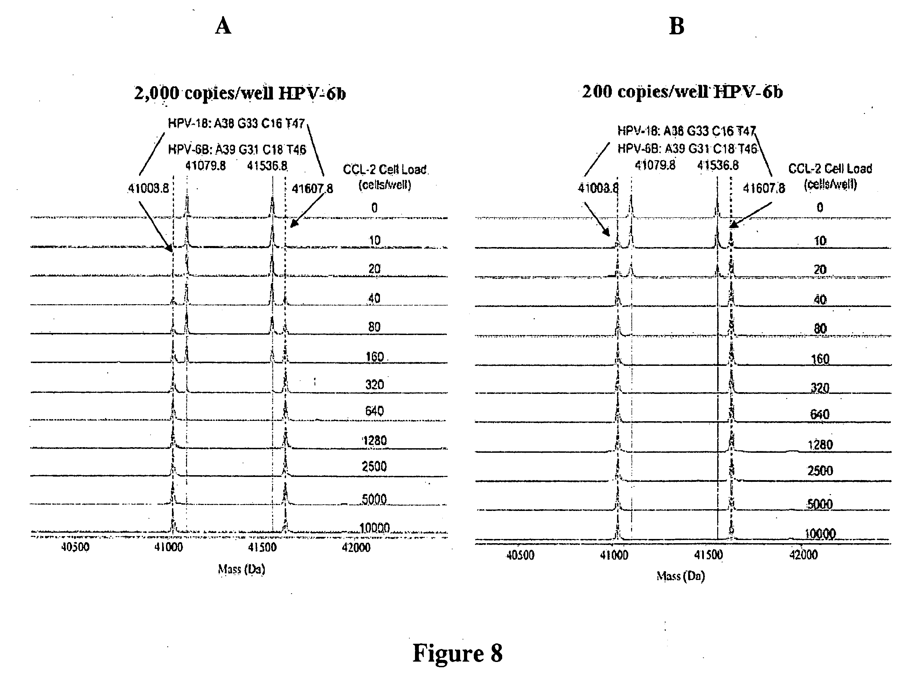

In addition to testing the performance of the primers on the cell lines, plasmid DNA containing HPV-6b was spiked into the CCL-2 cell line to determine the dynamic range of detection of the two viruses, cell line derived HPV-18 and the plasmid-derived HPV-6b, simultaneously, In all the tests done, the broad primers as well as the Group A7 primers showed detection of HPV-18 in both cell lines at input levels between 1-10 cells per well. At an estimated copy number of approximately 20 HPV-18 genomes per cell, this corresponds to detection sensitivities between 20-200 genomes from cell lines containing papillomavirus sequences. In experiments done with a co-spike of HPV-6b plasmid into these cell lines, the detection ranges were comparable. HPV-6b was spiked in at two different, fixed concentrations of 200 copies and 2000 copies per well. Figure 8A shows the performance of the broad primer pair, 2534 in detecting the two viruses. Figure 8A shows simultaneous detection of HPV-6b and HPV-18 when the plasmid DNA was spiked in at 2000 copies into a range of CCL-2 cell concentration from 1000 to 0 per well. HPV-18 was detected in all wells with the exception of the lowest input level (10 cells/well), in the presence of 2000 copies of HPV-6b. HPV-6b (2000 copies) was detected in the presence of HeLa cell loads up to 600 cells/well, with an effective HPV-18 concentration of ~12000 genomes/well. Figure 8B shows a similar curve using a plasmid spike of ~200 copies per well. In this case, HPV-18 was detected at all test concentrations, including the lowest cell concentration of 10 cells/well. Figure 6 c and d show the percent of wells where HPV-18 and/or HPV-6 were detected in quadruplicate repeats of the above experiment, The dynamic range for detection of the two viruses simultaneously is between 5-10 fold at the lower and higher ends, giving an overall dynamic range of ~25 fold for the detection of competing templates in the presence of each other. The above experiments further highlight the strength of TIGER HPV assays, where 2 or more viruses can be simultaneously detected using the same assay.

### Design of Primers for Identification of Parvoviridae

In order to detect the presence of Parvoviridae in cell lines, primers were designed that broadly target specific genera of the Parvovirinae sub-family family, including *Dependovirus, Erythrovirus* and *Parvovirus.* Parvoviridae of the most medical concern are: B19, AAV and murine minute virus. Approximately 500 complete Parvoviridae genome sequences were obtained from GenBank. These genome sequences (each approximately 5 kilobases long) were aligned and scanned for conserved target regions. Initial survey of the genome alignments revealed very little homology across the three major genera (*Parvovirus, Dependovirus, Erythrovirus*). However, conserved sequence regions were identified with significant homologies within each genus that were the targets for primer design. In all three genera, the conserved sequence regions were within two major nodes, one in the *rep* gene, encoding NS 1 protein, and the other in the capsid, *cap* gene, encoding glycoprotein VP1 protein. It is contemplated that this ability to prime across all known instances of species within each of these genera will enable surveillance for known parvoviridae members and detection of previously unknown parvoviridae species/strains in cells and cell-derived material.

### Validation of Primers Designed for Identification of Parvoviridae

The primer pairs, disclosed in Table 1, that were designed to target nucleic acids encoding Dependovirus NS1 or VP1 proteins (2864-2875), were tested for their ability to detect AAV-2, by effecting amplification of an AAV-2 full length clone, resulting in identification of AAV-2 after determining molecular mass of the amplification product by mass spectrometry and calculating base composition using methods described hereinabove. The AAV-2 plasmid was provided by Dr. John Chiorini, National Institutes of Health, Bethesda MD (Katano et al., BioTechniques, 2004, 36(4), 676-680). Initial testing of the primer pairs was done in limiting dilutions of the plasmid clone. Based on an OD estimation of starting material, 5,000 copies/genomes per well of AAV-2 plasmid were serially diluted and tested for primer performance. The results are shown in Table 4. An "X" indicates that the target was detected with the indicated primer pair and the indicated copy number.

**Table 4: Validation of Parvovirus Primer Pairs**

| | **AAV-2 Load (Genomes Per Well)** | | | | | | | | | | | |
|---|---|---|---|---|---|---|---|---|---|---|---|---|
| **Primer Pair Number** | **0** | **5** | **10** | **20** | **39** | **78** | **156** | **313** | **625** | **1250** | **2500** | **5000** |
| 2864 | | | | X | X | X | X | X | X | X | X | X |
| 2865 | | X | X | X | X | X | X | X | X | X | X | X |
| 2866 | | X | X | X | X | X | X | X | X | X | X | X |
| 2867 | | X | X | X | X | X | X | X | X | X | X | X |
| 2868 | | | | | | X | X | X | X | X | X | X |
| 2869 | | X | X | X | X | X | X | X | X | X | X | X |
| 2870 | | X | X | X | X | X | X | X | X | X | X | X |
| 2871 | | X | X | X | X | X | X | X | X | X | X | X |
| 2872 | | | | | | | | | | | | |
| 2873 | | | X | X | X | X | X | X | X | X | X | X |
| 2874 | | X | X | X | X | X | X | X | X | X | X | X |
| 2875 | | | X | X | X | X | X | X | X | X | X | X |

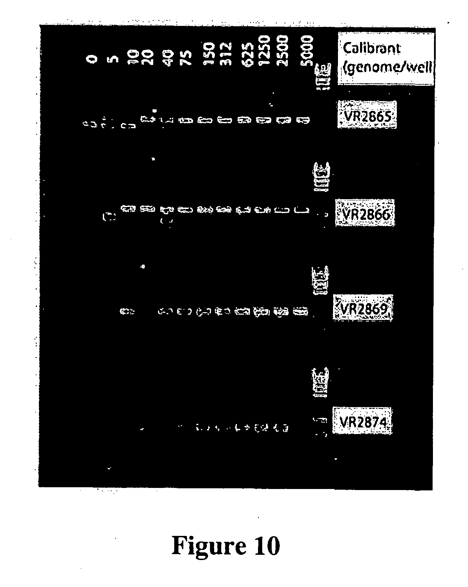

As shown in Table 4, the functioning primer pairs were able to detect the AAV-2 plasmid, at a concentration of at least 78 genomes per well, while several could detect more dilute concentrations. Based on the results shown in Table 4, seven most sensitive primer pairs (2865, 2866 and 2867 [targeted to NS1], and 2869, 2870, 2871 and 2874 [targeted to VP1]) were selected for additional testing and validation. Testing of primers using a synthetic DNA calibrant comprising the region encompassing these primers was generated and used in validation experiments with four of the seven primer pairs (2865, 2866, 2869 and 2874). The results are shown in figure 10. With these primer pairs, detection of the calibrant was similar to results shown in Table 3.

Mass spectrometry performed on amplification product generated using Primer Pair 2866 in the assay described above revealed mass and base composition that gave a unique match to the base composition of a previously known AAV-2 isolate indexed in the database. The results did not match base compositions of related sequences.

It is contemplated that, using the methods described herein, the primers targeted to Parvoviridae genera will specifically detect species and strains of this family in human samples, established cell cultures, Master Cell Banks, end-of-production cells, neoplastic-immortalized cell lines, cell-substrate derived biologicals, including primary and bulk harvest fluids, antibodies and vaccines, including viral vaccines, and will be useful in testing for contaminants in and determining the safety of cell-derived material, including vaccines.

### Design of Primers for Identification of Hepadnaviridae (including HBV)

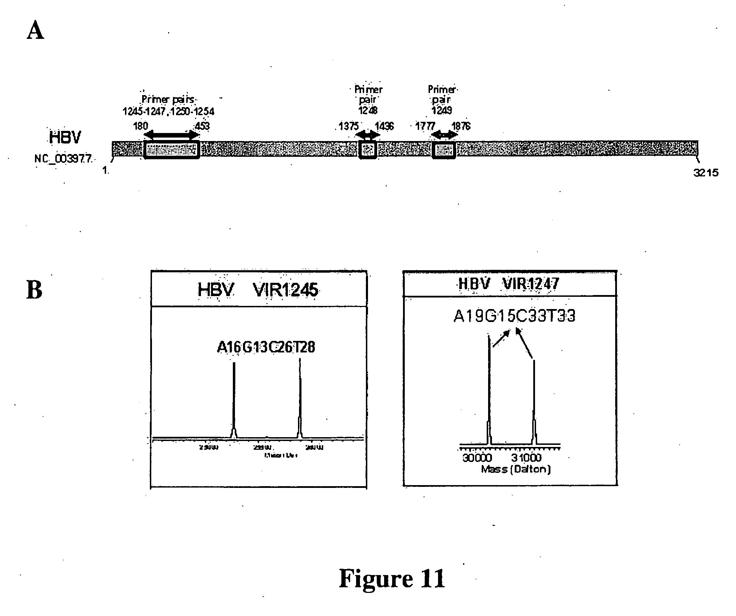

Primers targeting HBV were designed using methods described hereinabove. Primers were designed to the most highly conserved regions of multiple sequence alignments constructed for HBV. Primer sequences were analyzed for melting temperature and compatibility with respect to potential dimerization or secondary structure. All primer pair sequences were screened for specificity against sequences in the GenBank database as well as all human chromosomal sequences using in house methods described hereinabove. Primers were designed to hybridize within one of three conserved regions of GenBank accession number NC_003977 (incorporated herein as SEQ ID NO: 338). As shown in Figure 11a, the first of the three conserved regions comprises nucleotides 180-453 of SEQ ID NO:338 (including nucleotides that encode the glycoprotein HBVgp2 and the polymerase gp1), the second region comprises nucleotides 1375-1436 of SEQ ID NO:338 (including nucleotides that encode gp3 and gp1), and the third conserved region comprises nucleotides 1777-1876 of SEQ ID NO:338 (including includes nucleotides encoding gp3 and gp4 or core protein). Primer pairs designed to identify HBV are listed in Table 1 and include Primer Pair numbers 1245-1254. Specifically, Primer Pair numbers 1246 (SEQ ID NOs: 67:236) and 1247 (SEQ ID NOs: 68:237) were chosen for testing in validation studies and were tested for detection specificity using the methods described above, using isolated (prepared using the Qiagen Virus spin kit, Qiagen, Valencia, CA) viral genomic material from 6 different viral strains (Zeptometrix, Buffalo, NY). Use of both primer pairs resulted in generation of unique, appropriate amplicons in the presence of HBV from patient plasma. The base composition calculated from molecular mass was unique to HBV (figure 11b). The 5 additional viruses, not belonging to the Hepadnaviridae family were not detected by the HBV-targeted primers (figure 11b). HBV primers were able to detect (meaning effect amplification of an HBV amplification product, the molecular mass and base composition of which could be determined and calculated according to the methods described herein) as few as 100 genome copies. Primer pair 1247 was also validated using another source of HBV (Woodchuck), and produced the expected HBV amplicon with the correct basecount.

Additionally, internal calibrated controls were generated for each primer pair that contained primer binding sites, but were distinct from target virus elements by length and base composition. These controls were constructed in a plasmid vector and verified by sequencing. RNA runoff transcripts from the vectors were quantitated and will be used in quantification assays.

It is contemplated that, using the methods described herein, the primers targeted to Hepadnaviridae family members, for example, HBV, will specifically detect species and strains of this family in human samples, established cell cultures, Master Cell Banks, end-of-production cells, neoplastic-immortalized cell lines, cell-substrate derived biologicals, including primary and bulk harvest fluids, antibodies and vaccines, including viral vaccines, and will be useful in testing for contaminants in and determining the safety of cell-derived material, including vaccines.

### Design of Primers for Identification of Paramyxoviridae

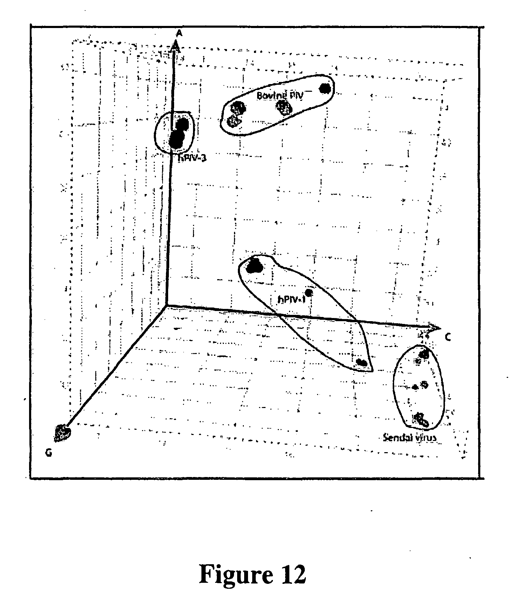

In order to detect the presence of Paramyxoviridae, primers were designed to broadly target various genera and species described hereinabove. More than 100 complete and partial genome sequences were obtained from GenBank (each about 15 KB in length), and aligned them to one another using bioinformatics tools built in-house, and scanned them for conserved target regions. Initial survey of genome alignments revealed little homology across the major genera (Respirovirus, Pneumovirus, Rubulavirus and Avulavirus). However, regions within each genus exhibited significant homologies and were used for primer design. In all major genera, the conserved regions were primarily within the RNA dependent RNA polymerase (RdRp) gene. Primers were designed and will be tested using material obtained from ATCC (shown in Table 5). Primers were designed to target RdRp of all Respirovirus species, including human and bovine PIV1 and PIV3 and Sendai virus. As shown in figure 12, these primers will amplify all known variants of these species and differentiate them using methods described herein. The primers are listed in Table 1. For initial testing, two different constructs were designed. The first uses a T7 RNA run-off product from ATCC VR-1380 (PAV-1) as the template. The resultant RNA will be used for validation. A second synthetic construct, comprising just regions chosen for primer design has also been generated and will provide a quantitative test material for sensitivity and detection methods as described herein.

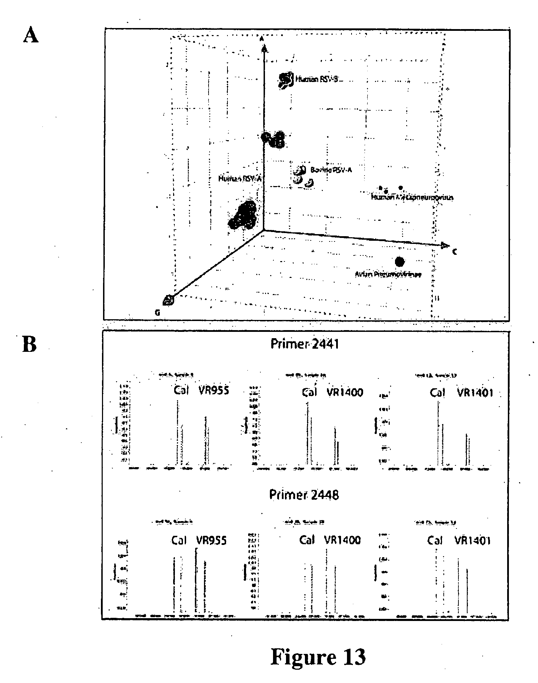

Primers were designed and targeted to the Pneumovirinae sub-family. Examples of such primers are listed in Table 1. As shown in figure 13, these primers will amplify all known pneumovirinae species, including human and bovine respiratory syncital virus (RSV), pneumonia virus of mice, and human metapneumovirus (HMPV), and can be used to uniquely identify individual species. Pneumovirinae-targeted primer pairs were initially tested using a T7 RNA run-off product from ATCC (VR-1400, RSV-B) as the template using the methods disclosed herein. Using this assay, all primer pairs tested (including Primer Pair numbers: 2442, 2452, 2441 and 2448) produced amplified products with determined base compositions that match RSV-B base compositions in the database uniquely and were clearly differentiated from all other species in the group. In further quantification testing experiments, four primer pairs were tested using the methods described herein and two different synthetic control constructs: an internal calibration control and an internal positive control, which were similar, but differed in length and composition. Each control was obtained as a purified plasmid preparation from Blue Heron Technologies (Bothell, WA) and accurately quantitated using a Nanodrop spectrophotometer. The assay results showed reliable detection of as few as 5 copies of the internal positive control, with robust detections at 40 copies or higher. The assay showed 1:1 quantification of the two standards when included at identical amounts (300 copies).

The two primer pairs that performed best in this quantification testing (Primer Pair number 2441- SEQ ID NOs: 103:274, and Primer Pair number 2448- SEQ ID NOs: 109:278) were chosen for further testing using three RSV-B isolates (VR-955, VR-1400 and VR-1401), each spiked at 1000x dilutions of the ATCC stock. Using the methods described herein, both primers produced robust amplicons from all three viral isolates (Figure 13b). Calculated base compositions from these amplicons matched RSV-B entries indexed in the database uniquely and were clearly differentiated from all other species of the sub-family including RSV-A.

Primer pairs were designed to target the Avulavirus genus, containing Newcastle disease and other avian paramyxovirusee or the Rubalavirus, which contains parainfluenza virus PIV-2, simian parainfluenza (SIV-5), porcine parainfluenza and mumps virus. Each of these primers was targeted to the RdRp gene region. Examples are listed in Table 1 and include Primer Pair numbers 2435, 2436, 2437, 2438, 2439 and 2440. These primer pairs will amplify all known members of the respective genus and can be used for species identification. These primers will be tested and validated using methods described herein.

It is contemplated that, using the methods described herein, the primers targeted to paramyxovirus sub-families and genera will specifically detect species and strains of this family in established cell cultures, Master Cell Banks, end-of-production cells, neoplastic-immortalized cell lines, cell-substrate derived biologicals, including primary and bulk harvest fluids, antibodies and vaccines, including viral vaccines, and will be useful in testing for contaminants in and determining the safety of cell-derived material, including vaccines.

**Table 5: Paramyxoviruses Used for Primer Testing and Validation**

| **Virus** | **Strain** | **ATCC Number** |
|---|---|---|
| RSV | 9320 | ATCC:VR-955 |
| Parainfluenza type I | | ATCC:VR-1380 |
| Parainfluenza 5 | DA | ATCC:VR-263 |
| Parainfluenza 3 | C243 | ATCC:VR-93 |
| Caciid parainfluenza 3 | TS-9 | ATCC:VR-1547 |
| RSVB | RSVBWash18537/'67[CH18537] | ATCC:VR-1401 |
| RSV B wildtype | B WV/14617/'85[B-1 wild Type] | ATCC:VR-1400 |
| Parainfluenza 4B | CH 19503 | ATCC:VR-1377 |
| Parainfluenza 4A | M-25 | ATCC:VR-1378 |
| Mumps (4) | Jones, Enders, Enders | ATCC:VR1438, 1379, 106 |
| Measles | Edmonston | ATCC:VR-24 |

### Example 2: Sample Preparation and PCR

Samples were processed to obtain viral genomic material using a Qiagen QIAamp Virus BioRobot MDx Kit. Resulting genomic material was amplified using an MJ Thermocycler Dyad unit and the amplicons were characterized on a Bruker Daltonics MicroTOF instrument. The resulting data was analyzed using GenX software (SAIC, San Diego, CA and Ibis, Carlsbad, CA).

All PCR reactions were assembled in 50 µL reaction volumes in a 96-well microtiter plate format using a Packard MPII liquid handling robotic platform and M.J. Dyad thermocyclers (MJ research, Waltham, MA). The PCR reaction mixture consisted of 4 units of Amplitaq Gold, 1x buffer II (Applied Biosystems, Foster City, CA), 1.5 mM MgCl₂, 0.4 M betaine, 800 µM dNTP mixture and 250 nM of each primer. The following typical PCR conditions were used: 95°C for 10 min followed by 8 cycles of 95°C for 30 seconds, 48°C for 30 seconds, and 72°C 30 seconds with the 48°C annealing temperature increasing 0.9°C with each of the eight cycles. The PCR was then continued for 37 additional cycles of 95°C for 15 seconds, 56°C for 20 seconds, and 72°C 20 seconds.

### Example 3: Solution Capture Purification of PCR Products for Mass Spectrometry with Ion Exchange Resin-Magnetic Beads

For solution capture of nucleic acids with ion exchange resin linked to magnetic beads, 25 µl of a 2.5 mg/mL suspension of BioClone amine terminated supraparamagnetic beads were added to 25 to 50 µl of a PCR (or RT-PCR) reaction containing approximately 10 pM of a typical PCR amplification product. The above suspension was mixed for approximately 5 minutes by vortexing or pipetting, after which the liquid was removed after using a magnetic separator. The beads containing bound PCR amplification product were then washed three times with 50mM ammonium bicarbonate/50% MeOH or 100mM ammonium bicarbonate/50% MeOH, followed by three more washes with 50% MeOH. The bound PCR amplicon was eluted with a solution of 25mM piperidine, 25mM imidazole, 35% MeOH which included peptide calibration standards.

### Example 4: Mass Spectrometry and Base Composition Analysis

The ESI-FTICR mass spectrometer is based on a Bruker Daltonics (Billerica, MA) Apex II 70e electrospray ionization Fourier transform ion cyclotron resonance mass spectrometer that employs an actively shielded 7 Tesla superconducting magnet. The active shielding constrains the majority of the fringing magnetic field from the superconducting magnet to a relatively small volume. Thus, components that might be adversely affected by stray magnetic fields, such as CRT monitors, robotic components, and other electronics, can operate in close proximity to the FTICR spectrometer. All aspects of pulse sequence control and data acquisition were performed on a 600 MHz Pentium II data station running Bruker's Xmass software under Windows NT 4.0 operating system. Sample aliquots, typically 15 µl, were extracted directly from 96-well microtiter plates using a CTC HTS PAL autosampler (LEAP Technologies, Carrboro, NC) triggered by the FTICR data station. Samples were injected directly into a 10 µl sample loop integrated with a fluidics handling system that supplies the 100 µl /hr flow rate to the ESI source. Ions were formed via electrospray ionization in a modified Analytica (Branford, CT) source employing an off axis, grounded electrospray probe positioned approximately 1.5 cm from the metalized terminus of a glass desolvation capillary. The atmospheric pressure end of the glass capillary was biased at 6000 V relative to the ESI needle during data acquisition. A counter-current flow of dry N₂ was employed to assist in the desolvation process. Ions were accumulated in an external ion reservoir comprised of an rf-only hexapole, a skimmer cone, and an auxiliary gate electrode, prior to injection into the trapped ion cell where they were mass analyzed. Ionization duty cycles > 99% were achieved by simultaneously accumulating ions in the external ion reservoir during ion detection. Each detection event consisted of 1M data points digitized over 2.3 s. To improve the signal-to-noise ratio (S/N), 32 scans were co-added for a total data acquisition time of 74 s.

The ESI-TOF mass spectrometer is based on a Bruker Daltonics MicroTOF™. Ions from the ESI source undergo orthogonal ion extraction and are focused in a reflectron prior to detection. The TOF and FTICR are equipped with the same automated sample handling and fluidics described above. Ions are formed in the standard MicroTOF™ ESI source that is equipped with the same off-axis sprayer and glass capillary as the FTICR ESI source. Consequently, source conditions were the same as those described above. External ion accumulation was also employed to improve ionization duty cycle during data acquisition. Each detection event on the TOF was comprised of 75,000 data points digitized over 75 µs.

The sample delivery scheme allows sample aliquots to be rapidly injected into the electrospray source at high flow rate and subsequently be electrosprayed at a much lower flow rate for improved ESI sensitivity. Prior to injecting a sample, a bolus of buffer was injected at a high flow rate to rinse the transfer line and spray needle to avoid sample contamination/carryover. Following the rinse step, the autosampler injected the next sample and the flow rate was switched to low flow. Following a brief equilibration delay, data acquisition commenced. As spectra were co-added, the autosampler continued rinsing the syringe and picking up buffer to rinse the injector and sample transfer line. In general, two syringe rinses and one injector rinse were required to minimize sample carryover. During a routine screening protocol a new sample mixture was injected every 106 seconds. More recently a fast wash station for the syringe needle has been implemented which, when combined with shorter acquisition times, facilitates the acquisition of mass spectra at a rate of just under one spectrum/minute.

Raw mass spectra were post-calibrated with an internal mass standard and deconvoluted to monoisotopic molecular masses. Unambiguous base compositions were derived from the exact mass measurements of the complementary single-stranded oligonucleotides. Quantitative results are obtained by comparing the peak heights with an internal PCR calibration standard present in every PCR well at 500 molecules per well. Calibration methods are commonly owned and disclosed in U.S. Provisional Patent Application Serial No. 60/545,425

### Example 5: De Novo Determination of Base Composition of Amplification Products using Molecular Mass Modified Deoxynucleotide Triphosphates

Because the molecular masses of the four natural nucleobases have a relatively narrow molecular mass range (A = 313.058, G = 329.052, C = 289.046, T = 304.046 - See Table 6), a persistent source of ambiguity in assignment of base composition can occur as follows: two nucleic acid strands having different base composition may have a difference of about 1 Da when the base composition difference between the two strands is G ↔ A (-15.994) combined with C ↔ T (+15.000). For example, one 99-mer nucleic acid strand having a base composition of A₂₇G₃₀C₂₁T₂₁ has a theoretical molecular mass of 30779.058 while another 99-mer nucleic acid strand having a base composition of A₂₆G₃₁C₂₂T₂₀ has a theoretical molecular mass of 30780.052. A 1 Da difference in molecular mass may be within the experimental error of a molecular mass measurement and thus, the relatively narrow molecular mass range of the four natural nucleobases imposes an uncertainty factor.

The present disclosure provides for a means for removing this theoretical 1 Da uncertainty factor through amplification of a nucleic acid with one mass-tagged nucleobase and three natural nucleobases. The term "nucleobase" as used herein is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

Addition of significant mass to one of the 4 nucleobases (dNTPs) in an amplification reaction, or in the primers themselves, will result in a significant difference in mass of the resulting amplification product (significantly greater than 1 Da) arising from ambiguities arising from the G ↔ A combined with C ↔ T event (Table 6). Thus, the same the G ↔ A (-15.994) event combined with 5-Iodo-C ↔ T (-110.900) event would result in a molecular mass difference of 126.894. If the molecular mass of the base composition A₂₇G₃₀ **5-Iodo-C₂₁ T₂₁** (33422.958) is compared with A₂₆G₃₁**5-Iodo**-C₂₂T₂₀, (33549.852) the theoretical molecular mass difference is +126.894. The experimental error of a molecular mass measurement is not significant with regard to this molecular mass difference. Furthermore, the only base composition consistent with a measured molecular mass of the 99-mer nucleic acid is A₂₇G₃₀**5-Iodo**-**C**₂₁T₂₁. In contrast, the analogous amplification without the mass tag has 18 possible base compositions.

**Table 6: Molecular Masses of Natural Nucleobases and the Mass-Modified Nucleobase 5-Iodo-C and Molecular Mass Differences Resulting from Transitions**

| **Nucleobase** | **Molecular Mass** | **Transition** | **A Molecular Mass** |
|---|---|---|---|
| A | 313.058 | A-->T | -9.012 |
| A | 313.058 | A-->C | -24.012 |
| A | 313.058 | A-->5-Iodo-C | 101.888 |
| A | 313.058 | A-->G | 15.994 |
| T | 304.046 | T-->A | 9.012 |
| T | 304.046 | T-->C | -15.000 |
| T | 304.046 | T-->5-Iodo-C | 110.900 |
| T | 304.046 | T-->G | 25.006 |
| C | 289.046 | C-->A | 24.012 |
| c | 289.046 | C-->T | 15.000 |
| c | 289.046 | C-->G | 40.006 |
| 5-Iodo-C | 414.946 | 5-Iodo-C-->A | -101.888 |
| 5-Iodo-C | 414.946 | 5-Iodo-C-->T | -110.900 |
| 5-Iodo-C | 414.946 | 5-Iodo-C-->G | -85.894 |
| G | 329.052 | G-->A | -15.994 |
| G | 329.052 | G-->T | -25.006 |
| G | 329.052 | G-->C | -40.006 |
| G | 329.052 | G-->5-Iodo-C | 85.894 |

Mass spectra of bioagent-identifying amplicons were analyzed independently using a maximum-likelihood processor, such as is widely used in radar signal processing. This processor, referred to as GenX, first makes maximum likelihood estimates of the input to the mass spectrometer for each primer by running matched filters for each base composition aggregate on the input data. This includes the GenX response to a calibrant for each primer.

The algorithm emphasizes performance predictions culminating in probability-of-detection versus probability-of false-alarm plots for conditions involving complex backgrounds of naturally occurring organisms and environmental contaminants. Matched filters consist of a *priori* expectations of signal values given the set of primers used for each of the bioagents. A genomic sequence database is used to define the mass base count matched filters. The database contains the sequences of known bacterial bioagents and includes threat organisms as well as benign background organisms. The latter is used to estimate and subtract the spectral signature produced by the background organisms. A maximum likelihood detection of known background organisms is implemented using matched filters and a running-sum estimate of the noise covariance. Background signal strengths are estimated and used along with the matched filters to form signatures which are then subtracted. The maximum likelihood process is applied to this "cleaned up" data in a similar manner employing matched filters for the organisms and a running-sum estimate of the noise-covariance for the cleaned up data.

The amplitudes of all base compositions of bioagent-identifying amplicons for each primer are calibrated and a final maximum likelihood amplitude estimate per organism is made based upon the multiple single primer estimates. Models of all system noise are factored into this two-stage maximum likelihood calculation. The processor reports the number of molecules of each base composition contained in the spectra. The quantity of amplification product corresponding to the appropriate primer set is reported as well as the quantities of primers remaining upon completion of the amplification reaction.

Base count blurring can be carried out as follows. "Electronic PCR" can be conducted on nucleotide sequences of the desired bioagents to obtain the different expected base counts that could be obtained for each primer pair. See for example, ncbi.nlm.nih.gov/sutils/e-pcr/; Schuler, Genome Res. 7:541-50, 1997. In one illustrative embodiment, one or more spreadsheets, such as Microsoft Excel workbooks contain a plurality of worksheets. First in this example, there is a worksheet with a name similar to the workbook name; this worksheet contains the raw electronic PCR data. Second, there is a worksheet named "filtered bioagents base count" that contains bioagent name and base count; there is a separate record for each strain after removing sequences that are not identified with a genus and species and removing all sequences for bioagents with less than 10 strains. Third, there is a worksheet, "Sheet 1" that contains the frequency of substitutions, insertions, or deletions for this primer pair. This data is generated by first creating a pivot table from the data in the "filtered bioagents base count" worksheet and then executing an Excel VBA macro. The macro creates a table of differences in base counts for bioagents of the same species, but different strains. One of ordinary skill in the art may understand additional pathways for obtaining similar table differences without undo experimentation.

Application of an exemplary script, involves the user defining a threshold that specifies the fraction of the strains that are represented by the reference set of base counts for each bioagent. The reference set of base counts for each bioagent may contain as many different base counts as are needed to meet or exceed the threshold. The set of reference base counts is defined by taking the most abundant strain's base type composition and adding it to the reference set and then the next most abundant strain's base type composition is added until the threshold is met or exceeded. The current set of data was obtained using a threshold of 55%, which was obtained empirically.

For each base count not included in the reference base count set for that bioagent, the script then proceeds to determine the manner in which the current base count differs from each of the base counts in the reference set. This difference may be represented as a combination of substitutions, Si=Xi, and insertions, Ii=Yi, or deletions, Di=Zi. If there is more than one reference base count, then the reported difference is chosen using rules that aim to minimize the number of changes and, in instances with the same number of changes, minimize the number of insertions or deletions. Therefore, the primary rule is to identify the difference with the minimum sum (Xi+Yi) or (Xi+Zi), *e.g.,* one insertion rather than two substitutions. If there are two or more differences with the minimum sum, then the one that will be reported is the one that contains the most substitutions.

Differences between a base count and a reference composition are categorized as one, two, or more substitutions, one, two, or more insertions, one, two, or more deletions, and combinations of substitutions and insertions or deletions. The different classes of nucleobase changes and their probabilities of occurrence have been delineated in U.S. Patent Application Publication No. 2004209260 (U.S. Application Serial No. 10/418,514)

Various modifications of the invention, in addition to those described herein, will be apparent to those skilled in the art from the foregoing description. Such modifications are also intended to fall within the scope of the appended claims.

## Claims

1. A method for identifying or determining the presence or absence of an adventitious contaminant virus in a sample, wherein said adventitious virus is a member of the *Dependovirus* genus, said method comprising:
contacting nucleic acids from the sample with at least one primer pair,
wherein each of the two primers of each of said at least one primer pair hybridizes to a conserved sequence region of a *Dependovirus* nucleic acid,
wherein the two conserved sequence regions flank a variable region of *Dependovirus,*
wherein each of said conserved sequence regions comprises at least a portion of a gene,
wherein each of the at least one primer pair comprises at least 100% sequence identity with any primer pair selected from the group represented by SEQ ID NOs: 160:329, 161:329, 161:330, 163:332, 164:333, 165:334, and 168:336;
amplifying the two conserved sequence regions and the variable region to produce an amplification product,
determining the molecular mass of said amplification product by mass spectrometry; and
comparing said determined molecular mass to a database comprising indexed molecular masses;
wherein a match between said determined molecular mass and a molecular mass in the database indicates the presence of said *Dependovirus* in said sample.

2. A method for identifying or determining the presence or absence of an adventitious contaminant virus in a sample, wherein said adventitious virus is a member of the *Dependovirus* genus, said method comprising:
contacting nucleic acids from the sample with at least one primer pair,
wherein each of the two primers of each of said at least one primer pair hybridizes to a conserved sequence region of a *Dependovirus* nucleic acid,
wherein the two conserved sequence regions flank a variable region of a *Dependovirus*, and
wherein each of said conserved sequence regions comprises at least a portion of a gene,
wherein each of the at least one primer pair comprises at least 100% sequence identity with any primer pair selected from the group represented by SEQ ID NOs: 160:329, 161:329, 161:330, 163:332, 164:333, 165:334, and 168:336;
amplifying the two conserved sequence regions and the variable region to produce an amplification product,
determining the molecular mass of said amplification product by mass spectrometry;
calculating the base composition of said amplification product from said molecular mass; and
comparing said calculated base composition to a database comprising indexed base compositions;
wherein a match between said calculated base composition and a base composition in the database indicates the presence of said *Dependovirus* in said sample.

3. The method of claim 1 or 2 wherein the amplification product is between about 45 and about 200 nucleic acids in length.

4. The method of claim 1 or 2 wherein each of the conserved sequence regions is between about 80% and about 100% identical among all species in a *Dependovirus* genus.

5. The method of claim 1 or 2 wherein the at least one primer pair comprises at least two primer pairs, wherein at least one of said at least two primer pairs hybridizes to a nucleic acid encoding a VP1 protein, and wherein at least one of said at least two primer pairs hybridizes to a nucleic acid encoding an NS 1 protein.

6. The method of any of claims 1 or 2, wherein the at least one primer pair comprises at least two primer pairs or at least four primer pairs.

7. An oligonucleotide primer pair for identifying a contaminating *Dependovirus* comprising a forward and a reverse primer, wherein each primer hybridize to a nucleic acid encoding an NS1 protein or a nucleic acid encoding a VP1 protein, said oligonucleotide primer pair comprising at least 100% sequence identity with any primer pair selected from the group represented by SEQ ID NOs: 160:329, 161:329, 161:330, 163:332, 164:333, 165:334, and 168:336.

8. A composition comprising the oligonucleotide primer pair of claim 7.

9. A kit for identification or detection of a contaminating *Dependovirus* comprising the composition of claim 8.

10. The kit of claim 9 further comprising at least one additional primer pair, wherein at least one of the primer pairs hybridizes to a nucleic acid encoding an NS1 protein and wherein at least one of the primer pairs hybridizes to a nucleic acid encoding a VP1 protein, and wherein use of the primer pairs will generate amplification products to identify the species, sub-species, serotype, genotype, or combination thereof, of one or more members of the *Dependovirus* genus.

11. The kit of claim 10 wherein each of the primer pairs will generate a unique amplification product in a multiplex reaction.

12. The composition of claim 8, wherein any non-templated T residue on the 5' end of one or both of said primers is removed.

13. A kit for identification or detection of one or more *Dependovirus* comprising:
a first primer pair having at least 100% sequence identity with the primer pair represented by SEQ ID NOs: 160:329,
a second primer pair having at least 100% sequence identity with the primer pair represented by SEQ ID NOs: 161:329,
a third primer pair having at least 100% sequence identity with the primer pair represented by SEQ ID NOs: 161:330,
a fourth primer pair having at least 100% sequence identity with the primer pair represented by SEQ ID NOs: 163:332,
a fifth primer pair having at least 100% sequence identity with the primer pair represented by SEQ ID NOs: 164:333,
a sixth primer pair having at least 100% sequence identity with the primer pair represented by SEQ ID NOs: 165:334, and
a seventh primer pair having at least 100% sequence identity with the primer pair represented by SEQ ID NOs: 168:336.

## Patentansprüche

1. Verfahren zur Identifizierung oder Bestimmung des Vorliegens oder Fehlens eines adventiven Kontaminationsvirus in einer Probe, wobei es sich bei dem adventiven Virus um ein Mitglied der Gattung *Dependovirus* handelt, wobei man in dem Verfahren
Nukleinsäuren aus der Probe mit wenigstens einem Primerpaar in Kontakt bringt,
wobei die beiden Primer eines jeden wenigstens einen Primerpaars jeweils an einen konservierten Sequenzbereich einer *Dependovirus*-Nukleinsäure hybridisieren, wobei die beiden konservierten Sequenzbereiche einen variablen Bereich von *Dependovirus* flankieren,
wobei die konservierten Sequenzbereiche jeweils wenigstens einen Teil eines Gens umfassen,
wobei jedes wenigstens eine Primerpaar wenigstens 100% Sequenzidentität mit einem beliebigen aus der durch SEQ ID NO: 160:329, 161:329, 161:330, 163:332, 164:333, 165:334 und 168:336 repräsentierten Gruppe ausgewählten Primerpaar umfasst,
die beiden konservierten Sequenzbereiche und den variablen Bereich unter Erhalt eines Amplifikationsprodukts amplifiziert,
die Molekülmasse des Amplifikationsprodukts massenspektrometrisch bestimmt und
die bestimmte Molekülmasse mit einer indexierte Molekülmassen umfassenden Datenbank vergleicht, wobei eine Übereinstimmung zwischen der bestimmten Molekülmasse und einer Molekülmasse in der Datenbank das Vorliegen des *Dependovirus* in der Probe anzeigt.

2. Verfahren zur Identifizierung oder Bestimmung des Vorliegens oder Fehlens eines adventiven Kontaminationsvirus in einer Probe, wobei es sich bei dem adventiven Virus um ein Mitglied der Gattung *Dependovirus* handelt, wobei man in dem Verfahren
Nukleinsäuren aus der Probe mit wenigstens einem Primerpaar in Kontakt bringt,
wobei die beiden Primer eines jeden wenigstens einen Primerpaars jeweils an einen konservierten Sequenzbereich einer *Dependovirus*-Nukleinsäure hybridisieren, wobei die beiden konservierten Sequenzbereiche einen variablen Bereich eines *Dependovirus* flankieren und
wobei die konservierten Sequenzbereiche jeweils wenigstens einen Teil eines Gens umfassen,
wobei jedes wenigstens eine Primerpaar wenigstens 100% Sequenzidentität mit einem beliebigen aus der durch SEQ ID NO: 160:329, 161:329, 161:330, 163:332, 164:333, 165:334 und 168:336 repräsentierten Gruppe ausgewählten Primerpaar umfasst,
die beiden konservierten Sequenzbereiche und den variablen Bereich unter Erhalt eines Amplifikationsprodukts amplifiziert,
die Molekülmasse des Amplifikationsprodukts massenspektrometrisch bestimmt,
die Basenzusammensetzung des Amplifikationsprodukts anhand der Molekülmasse berechnet und die berechnete Basenzusammensetzung mit einer indexierte Basenzusammensetzungen umfassenden Datenbank vergleicht,
wobei eine Übereinstimmung zwischen der berechneten Basenzusammensetzung und einer Basenzusammensetzung in der Datenbank das Vorliegen des *Dependovirus* in der Probe anzeigt.

3. Verfahren nach Anspruch 1 oder 2, wobei das Amplifikationsprodukt eine Länge zwischen etwa 45 und etwa 200 Nukleinsäuren aufweist.

4. Verfahren nach Anspruch 1 oder 2, wobei die konservierten Sequenzbereiche unter allen Spezies in einer *Dependovirus*-Gattung jeweils zwischen etwa 80% und etwa 100% identisch sind.

5. Verfahren nach Anspruch 1 oder 2, wobei das wenigstens eine Primerpaar wenigstens zwei Primerpaare umfasst, wobei wenigstens eines der wenigstens zwei Primerpaare an eine für ein VP1-Protein codierende Nukleinsäure hybridisiert und wobei wenigstens eines der wenigstens zwei Primerpaare an eine für ein NS1-Protein codierende Nukleinsäure hybridisiert.

6. Verfahren nach einem der Ansprüche 1 oder 2, wobei das wenigstens eine Primerpaar wenigstens zwei Primerpaare oder wenigstens vier Primerpaare umfasst.

7. Oligonukleotid-Primerpaar zur Identifizierung eines kontaminierenden *Dependovirus,* umfassend einen Vorwärts- und einen Rückwärtsprimer, wobei die Primer jeweils an eine für ein NS1-Protein codierende Nukleinsäure oder eine für ein VP1-Protein codierende Nukleinsäure hybridisieren, wobei das Oligonukleotid-Primerpaar wenigstens 100% Sequenzidentität mit einem beliebigen aus der durch SEQ ID NO: 160:329, 161:329, 161:330, 163:332, 164:333, 165:334 und 168:336 repräsentierten Gruppe ausgewählten Primerpaar umfasst.

8. Zusammensetzung, umfassend das Oligonukleotid-Primerpaar nach Anspruch 7.

9. Kit zur Identifizierung oder zum Nachweis eines kontaminierenden *Dependovirus,* umfassend die Zusammensetzung nach Anspruch 8.

10. Kit nach Anspruch 9, ferner umfassend wenigstens ein zusätzliches Primerpaar, wobei wenigstens eines der Primerpaare an eine für ein NS1-Protein codierende Nukleinsäure hybridisiert und wobei wenigstens eines der Primerpaare an eine für ein VP1-Protein codierende Nukleinsäure hybridisiert und wobei die Verwendung der Primerpaare Amplifikationsprodukte zur Identifizierung der Spezies, Subspezies, des Serotyps, Genotyps oder einer Kombination davon eines oder mehrerer Mitglieder der Gattung *Dependovirus* erzeugt.

11. Kit nach Anspruch 10, wobei die Primerpaare jeweils ein einmaliges Amplifikationsprodukt in einer Multiplex-Reaktion erzeugen.

12. Zusammensetzung nach Anspruch 8, wobei alle Nichtmatrizen-T-Reste am 5'-Ende eines der oder beider Primer entfernt wird.

13. Kit zur Identifizierung eines oder mehrerer *Dependovirus,* umfassend:
ein erstes Primerpaar mit wenigstens 100% Sequenzidentität mit dem durch SEQ ID NO: 160:329 repräsentierten Primerpaar,
ein zweites Primerpaar mit wenigstens 100% Sequenzidentität mit dem durch SEQ ID NO: 161:329 repräsentierten Primerpaar,
ein drittes Primerpaar mit wenigstens 100% Sequenzidentität mit dem durch SEQ ID NO: 161:330 repräsentierten Primerpaar,
ein viertes Primerpaar mit wenigstens 100% Sequenzidentität mit dem durch SEQ ID NO: 163:332 repräsentierten Primerpaar,
ein fünftes Primerpaar mit wenigstens 100% Sequenzidentität mit dem durch SEQ ID NO: 164:333 repräsentierten Primerpaar,
ein sechstes Primerpaar mit wenigstens 100% Sequenzidentität mit dem durch SEQ ID NO: 165:334 repräsentierten Primerpaar und
ein siebtes Primerpaar mit wenigstens 100% Sequenzidentität mit dem durch SEQ ID NO: 168:336 repräsentierten Primerpaar.

## Revendications

1. Procédé pour identifier ou déterminer la présence ou l'absence d'un virus contaminateur adventice dans un échantillon, dans lequel ledit virus adventice est un membre du genre *Dependovirus,* ledit procédé comprenant les étapes consistant à :
mettre en contact des acides nucléiques provenant de l'échantillon avec au moins une paire d'amorces,
dans lequel chacune des deux amorces de chacune desdites au moins une paire d'amorces s'hybride avec une région de séquence conservée d'un acide nucléique de *Dependovirus,*
dans lequel les deux régions de séquences conservées flanquent une région variable de *Dependovirus,*
dans lequel chacune desdites régions de séquences conservées comprend au moins une partie de gène,
dans lequel chacune de la paire au moins d'amorces comprend une identité de séquence d'au moins 100% avec une quelconque paire d'amorces choisie dans le groupe représenté par les SEQ ID n° : 160:329, 161:329, 161:330, 163:332, 164:333, 165:334 et 168:336 ;
amplifier les deux régions de séquences conservées et la région variable pour produire un produit d'amplification ;
déterminer la masse moléculaire dudit produit d'amplification, via une spectrométrie de masse ; et
comparer ladite masse moléculaire déterminée avec une base de données comprenant des masses moléculaires indexées ;
dans laquelle une correspondance entre ladite masse moléculaire déterminée et une masse moléculaire dans la base de données indique la présence dudit *Dependovirus* dans ledit échantillon.

2. Procédé pour identifier ou déterminer la présence ou l'absence d'un virus contaminateur adventice dans un échantillon, dans lequel ledit virus adventice est un membre du genre *Dependovirus,* ledit procédé comprenant les étapes consistant à :
mettre en contact des acides nucléiques provenant de l'échantillon avec au moins une paire d'amorces,
dans lequel chacune des deux amorces de chacune desdites au moins une paire d'amorces s'hybride avec une région de séquence conservée d'un acide nucléique de *Dependovirus,*
dans lequel les deux régions de séquences conservées flanquent une région variable de *Dependovirus,* et
dans lequel chacune desdites régions de séquences conservées comprend au moins une partie de gène,
dans lequel chacune de la paire au moins d'amorces comprend une identité de séquence d'au moins 100% avec une quelconque paire d'amorces choisie dans le groupe représenté par les SEQ ID n° : 160:329, 161:329, 161:330, 163:332, 164:333, 165:334 et 168:336 ;
amplifier les deux régions de séquences conservées et la région variable pour produire un produit d'amplification ;
déterminer la masse moléculaire dudit produit d'amplification, via une spectrométrie de masse ;
calculer la composition en bases dudit produit d'amplification à partir de ladite masse moléculaire ; et
comparer ladite composition en bases calculée avec une base de données comprenant des compositions en bases indexées ;
dans laquelle une correspondance entre ladite composition en bases calculée et une composition en bases dans la base de données indique la présence dudit *Dependovirus* dans ledit échantillon.

3. Procédé selon la revendication 1 ou la 2, dans lequel le produit de l'amplification a une longueur comprise entre 45 environ et 200 environ acides nucléiques.

4. Procédé selon la revendication 1 ou la 2, dans lequel chacune des régions de séquences conservées est identique au moins entre 80% environ et 100% environ, parmi toutes les espèces dans un genre *Dependovirus.*

5. Procédé selon la revendication 1 ou la 2, dans lequel la paire au moins d'amorces comprend au moins deux paires d'amorces, dans laquelle au moins l'une desdites au moins deux paires d'amorces s'hybride avec un acide nucléique codant pour une protéine VP1 et dans laquelle au moins l'une desdites au moins deux paires d'amorces s'hybride avec un acide nucléique codant pour une protéine NS1.

6. Procédé selon l'une quelconque des revendications 1 ou 2, dans lequel la paire au moins d'amorces comprend au moins deux paires d'amorces ou au moins quatre paires d'amorces.

7. Paire d'amorces d'oligonucléotides destinée à identifier un *Dependovirus* contaminateur, comprenant une amorce avant et une arrière, dans laquelle chaque amorce s'hybride avec un acide nucléique codant pour une protéine NS1 ou un acide nucléique codant pour une protéine VP1, ladite paire d'amorces d'oligonucléotides comprenant une identité de séquence d'au moins 100% avec une quelconque paire d'amorces choisie dans le groupe représenté par les SEQ ID n° : 160:329, 161:329, 161:330, 163:332, 164:333, 165:334 et 168:336.

8. Composition comprenant la paire d'amorces d'oligonucléotides selon la revendication 7.

9. Trousse pour l'identification ou la détection d'un *Dependovirus* contaminateur, comprenant la composition selon la revendication 8.

10. Trousse, selon la revendication 9, comprenant en outre au moins une paire d'amorces supplémentaire, dans laquelle au moins l'une des paires d'amorces s'hybride avec un acide nucléique codant pour une protéine NS1 et dans laquelle au moins l'une des paires d'amorces s'hybride avec un acide nucléique codant pour une protéine VP1 et dans laquelle une utilisation des paires d'amorces génèrera des produits d'amplification servant à identifier l'espèce, la sous-espèce, le sérotype, le génotype ou des combinaisons de ceux-ci d'un ou plusieurs membre(s) du genre *Dependovirus.*

11. Trousse selon la revendication 10, dans laquelle chacune des paires d'amorces génèrera un produit d'amplification unique dans une réaction multiplexée.

12. Composition selon la revendication 8, dans laquelle un quelconque résidu T non basé sur une matrice, à l'extrémité 5' de l'une desdites amorces ou des deux, est retiré.

13. Trousse pour l'identification ou la détection d'un ou plusieurs *Dependovirus,* comprenant :
une première paire d'amorces ayant une identité de séquence d'au moins 100% avec la paire d'amorces représentée par les SEQ ID n° : 160:329 ;
une deuxième paire d'amorces ayant une identité de séquence d'au moins 100% avec la paire d'amorces représentée par les SEQ ID n° : 161:329 ;
une troisième paire d'amorces ayant une identité de séquence d'au moins 100% avec la paire d'amorces représentée par les SEQ ID n° : 161:330 ;
une quatrième paire d'amorces ayant une identité de séquence d'au moins 100% avec la paire d'amorces représentée par les SEQ ID n° : 163:332 ;
une cinquième paire d'amorces ayant une identité de séquence d'au moins 100% avec la paire d'amorces représentée par les SEQ ID n° : 164:333 ;
une sixième paire d'amorces ayant une identité de séquence d'au moins 100% avec la paire d'amorces représentée par les SEQ ID n° : 165:334 ; et
une septième paire d'amorces ayant une identité de séquence d'au moins 100% avec la paire d'amorces représentée par les SEQ ID n° : 168:336.
